(19) Europäisches Patentamt

European Patent Office

Office européen des brevets

(11) **EP 0 552 142 B2**

(12) **NEW EUROPEAN PATENT SPECIFICATION**

(45) Date of publication and mention
of the opposition decision:
**17.12.2003 Bulletin 2003/51**

(45) Mention of the grant of the patent:
**07.01.1998 Bulletin 1998/02**

(21) Application number: **90911332.6**

(22) Date of filing: **19.07.1990**

(51) Int Cl.⁷: **C07K 7/04**, C07K 14/47,
C07K 14/725, C07K 16/28,
C07K 1/00, A61K 38/03,
A61K 38/17, C12P 21/04

(86) International application number:
**PCT/US90/04085**

(87) International publication number:
**WO 9100/1133 (07.02.1991 Gazette 1991/04)**

(54) **T CELL RECEPTOR PEPTIDES AS THERAPEUTICS FOR AUTOIMMUNE AND MALIGNANT DISEASE**

T-ZELL-REZEPTOR-PEPTIDE ALS HEILMITTEL FÜR AUTOIMMUNE UND BÖSARTIGE KRANKHEITEN

PEPTIDES RECEPTEURS DE CELLULES T CONSTITUANT UNE THERAPIE CONTRE DES MALADIES AUTOIMMUNES ET MALIGNES

(84) Designated Contracting States:
**AT BE CH DE DK ES FR GB IT LI LU NL SE**

(30) Priority: **19.07.1989 US 382804**
**19.01.1990 US 467577**

(43) Date of publication of application:
**28.07.1993 Bulletin 1993/30**

(73) Proprietor: **CONNETICS CORPORATION**
**Palo Alto, CA 94303 (US)**

(72) Inventor: **CONNETICS CORPORATION**
**Palo Alto, CA 94303 (US)**

(74) Representative: **Harrison Goddard Foote**
**Quality House**
**Quality Court**
**Chancery Lane**
**London WC2A 1HT (GB)**

(56) References cited:
**EP-A- 0 296 786**

• **EUROPEAN JOURNAL OF IMMUNOLOGY vol. 19, no. 2, February 1989, pp. 279-284, J. CHLUBA et al.**
• **PROCEEDINGS OF THE NATIONAL ACADEMY OF SCIENCES OF THE USA vol. 85, no. 22, 15 November 1988, pp. 8608-8612, K. SAKAI et al.**
• **NATURE vol. 341, no. 6242, 12 October 1989, pp. 541-544, A. VANDENBARK et al.**
• **FASEB JOURNAL vol. 4, no. 7, 26 April 1990, p. A2023, G. HASHIM et al.**
• **JOURNAL OF IMMUNOLOGY vol. 144, no. 12, June 1990, pp. 4621-4627, G.A. HASHIM et al.**
• **BIOLOGICAL ABSTRACTS 1990, D.N. BOURDETTE et al.,abstract no. 89059282, pp. 81-85**
• **BIOLOGICAL ABSTRACTS 1989, A.A. VANDENBARK et al., abstract no. 38033335, pp. 541-544**
• **BIOLOGICAL ABSTRACTS 1989, G.A. HASHIM et al., abstract no. 89016844, pp. 222-230**
• **JOURNAL OF IMMUNOLOGY vol. 141, no. 11, December 1988, pp. 3828-3832, H. OFFNER et al.**

**Description**

Cross-Reference to Related Application

This is a continuation-in-part of U.S. Patent Application Serial No. 07/467,577, filed January 19, 1990, which is a continuation-in-part of U.S. Patent Application Serial No. 07/382,804, filed July 19, 1989.

## BACKGROUND OF THE INVENTION

Field of the Invention

The invention in the field of immunology and immunotherapy is directed to peptides and their pharmaceutical compositions which are capable of preventing, suppressing or treating immune-related diseases such as autoimmune and malignant diseases.

Description of the Background Art

Autoimmune diseases are characterized by an unwanted and unwarranted attack by the immune system on the tissues of the host. While the mechanism for progress of these diseases is not well-understood, at least some of the details with respect to antigen presentation in this (and other) contexts is being elucidated. It is now thought that antigens, including autoantigens, are processed by antigen-presenting cells (APC), and the resulting fragments are then associated with one of the cell surface proteins encoded by the major histocompatibility complex (MHC). As a result, recognition of a peptide antigen is said to be MHC "restricted." When the MHC/antigen fragment complex binds to a complementary T cell receptor (TCR) on the surface of a T lymphocyte, it leads to activation and proliferation of the clone or subpopulation of T cells that bear that particular TCR. Once activated, T cells have the capacity to regulate other cells of the immune system which display the processed antigen and to destroy cells or tissues which carry epitopes of the recognized antigen.

A review of the role of TCRs in autoimmune diseases by Acha-Orbea, H., et al. (Ann. Rev. Immunol. 7:371-405 (1989)) discussed the tremendous variation in TCRs available in the immune system of an individual and the generation of this diversity by germ line gene organization and rearrangement of the DNA encoding TCR $\alpha$ and $\beta$ chains. The $\alpha$ chains are encoded by various combinations of variable (V), junction (J) and constant (C) region gene segments. TCR $\beta$ chains are additionally encoded by a diversity (D) region gene segment, and, thus comprise a rearranged VDJC sequence. Due to allelic exclusion, a clone of T cells expresses only one type of TCR $\alpha$-$\beta$ heterodimer.

A growing number of human diseases have been classified as autoimmune in nature (see, Theofilopoulos, A., In: D.P. Stites, et al., eds., Basic and Clinical Immunology, Lange Medical Publications, Los Altos, CA, 1988) of which several examples are rheumatoid arthritis (RA), myasthenia gravis (MG), multiple sclerosis (MS), systemic lupus erythematosus (SLE), autoimmune thyroiditis (Hashimoto's thyroiditis), Graves' disease, inflammatory bowel disease, autoimmune uveoretinitis, polymyositis and certain types of diabetes. Animal models have been developed for a number of these human autoimmune diseases. Among the best studied model is experimental allergic encephalomyelitis (EAE, also called experimental autoimmune encephalomyelitis), a model for MS.

Because it is now known that these and other autoimmune diseases involve the action of T helper cells stimulated by the binding of their TCR to an MHC/autoantigen (or non-autoantigen) complex, prevention and/or treatment strategies have been proposed which are based on the disruption of interactions between the MHC/antigen complex and the TCR. Wraith, D.C., et al. (Cell 57:709-715 (1989)), proposed approaches based on this principle, including vaccination with whole T cells (as initially described by I.R. Cohen's laboratory, discussed below), passive blockade using antibodies which bind to the TCR, passive blockade using antibodies that bind to the MHC portion of the complex, administration of antibodies reactive with the T helper cell marker, CD4, and the use of peptides which mimic the antigen of interest and compete for binding to the MHC or the TCR molecule.

Myelin basic protein, MBP, is the major autoantigen involved in EAE and is the leading candidate as an encephalitogen involved in MS.

Heber-Katz's group (Heber-Katz, E., et al., Ann. N.Y. Acad. Sci. 540:576-577 (1988); Owhashi, M., et al., J. Exp. Med. 168:2153-2164 (Dec. 1988)) has analyzed the fine specificity of recognition of MBP epitopes by rat T cells. When T cells from rats immunized with MBP were hybridized to a mouse T lymphoma line and cloned, the pattern of fine specificity and Southern blot analysis of the TCR $V_\beta$ gene rearrangement indicated a polyclonal response, even though 75% of the clones reacted to the 68-88 encephalitogenic determinant. A monoclonal antibody (mAb), designated 10.18, directed at one encephalitogenic T cell hybridoma proved to be an anti-idiotype or anti-clonotype which reacted only with T cell clones specific for the MBP 68-88 epitope. The mAb could block or reverse EAE when injected with, or 5 days after, the encephalitogenic MBP peptide. Soluble mAb 10.18 blocked the specific T cell clones, and immobilized mAb 10.18 stimulated their proliferation. Following induction of EAE with MBP, the proportion of mAb 10.18-binding cells increased from initially very low frequencies. The authors concluded that the 10.18+ T cells probably represent the dominant pathogenic T cell repertoire of EAE in Lewis rats. However, it was not known whether mAb 10.18 recognized a V region or an idiotypic determinant.

T cells expressing the $TCR_{\alpha\beta}$ heterodimer can induce idiotypic and V gene family-specific antibodies that can regulate T cell function (Owhashi, M., et al., supra; Gascoigne, N.R.J., et al., Proc. Natl. Acad. Sci.. USA 84:2936 (1987); Kappler, J.W., et al., Nature 332:35 (1988); Kappler, J.W., et al., Cell 49:263 (1987); MacDonald, H.R., et al., Nature 332:40 (1988)). For example, antibodies that recognize the TCR $V_{\beta}8$ sequence have been effective in the prevention and treatment of autoimmunity in mice and rats (Owhashi, M., et al., supra; Acha-Orbea, H., et al., Cell 54: 263-273 (1988); Urban, J., et al., Cell 54:577-592 (1988)). Obtaining such antibodies selective for V region gene products has been dependent upon the availability of T cell clones that express TCR encoded by the relevant V gene family, and requires a laborious screening procedure using whole cells to establish specificity

While antibody therapies in which antibodies are directed to MHC molecules and CD4 molecules have been generally successful in several animal models of autoimmunity, these approaches may be too nonspecific and potentially overly suppressive, since 70% of T cells bear the CD4 marker, and since all T cell-mediated responses and most antibody responses require MHC-associated antigen presentation.

I. R. Cohen's laboratory has developed an approach to the immunospecific treatment of autoimmunity which utilizes whole live orattenuated T lymphocytes as vaccines to treat or prevent EAE, experimental autoimmune thyroiditis (EAT), and experimental arthritis. This approach is reviewed in Cohen, I.R., Immunol. Rev. 94:5-21 (1986), which discusses several animal models of autoimmune disease wherein vaccination with disease-specific T lymphocytes has been used to generate prophylactic or therapeutic effects. The fine specificity of vaccination was dictated by the fine specificity of the T cell recognition, possibly implicating the TCR. For example, two different anti-MBP T cell lines, each reactive to a different epitope of MBP, were found to vaccinate against EAE specifically induced by the particular epitope, indicating some form of anti-idiotypic immunity. However, when attempts were made to isolate clones of MBP-specific or thy-roglobulin-specific T cells (in a thyroiditis model) from the non-clonal cell lines, only clones producing disease, but not resistance, were obtained. This led to the finding that appropriate aggregation or rigidification of cell membranes, by either hydrostatic pressure or chemical cross-linking, yielded cells which could induce protection more consistently Similarly, low doses (sub-encephalitogenic) of MBP-specific cells could also induce resistance to lethal EAE. The protective state was termed "counter-autoimmunity." This state involves T cell clones which can specifically proliferate in response to the vaccinating T cells, can suppress effector clones in vitro (non-specifically, presumably through release of a suppressive lymphokine), and can adoptively transfer counter-autoimmunity in vivo. Such counter-autoimmunity is accompanied by suppressed delayed hypersensitivity (DH) responses to the specific epitope and prevention or remission of clinical disease.

A major difficulty with the foregoing approaches is that they require the use of complex biological preparations which do not comprise well-defined therapeutic agents. Such preparations suffer from complex production and maintenance requirements (e.g., the need for sterility and large quantities of medium for producing large number of "vaccine" T cells), and lack reproducibility from batch to batch. The T cell "vaccine" preparations, to be useful in humans, must be both autologous and individually specific, that is, uniquely tailored for each patient. Furthermore, the presence of additional antigens on the surface of such T cells may result in a broader, possibly detrimental, immune response not limited to the desired T cell clones (Offner, H. et al., J. Neuroimmunol. 21:13-22 (1989).

EP-A-0340109 proposes the use of antibodies, oligopeptides or antiidiotype antibodies related to variable regions of a T cell receptor associated with an autoimmune disease to inhibit the function of the T cell receptor.

WO90/11294 proposes the use of T cells, T cell receptors or fragments thereof as vaccines against T cell mediated pathologies.

There is a great need, therefore, for agents and pharmaceutical compositions which have the properties of specificity for the targeted autoimmune response, predictability in their selection, convenience and reproducibility of preparation, and sufficient definition to permit precise control of dosage.

## SUMMARY OF THE INVENTION

This invention was made in response to a clear need for therapeutic agents and compositions capable of preventing, suppressing or treating immune-related diseases in a clone-specific manner, without causing generalized suppression of immunity, as is the case with most current immunotherapeutic and immunopharmacologic approaches. The invention was developed from the knowledge that lines or clones of T cells specific for autoantigens, which actually mediated autoimmune disease, could be converted into therapeutics by complex attenuation protocols, and injected directly into animals to prevent or treat the disease.

The inventor's attempts to achieve such cellular immunotherapy resulted in less than optimal results. When using attenuation methods disclosed in the prior art, the inventor achieved varying, unpredictable levels of protection, and the resultant immunity was not clonally limited, presumably because whole cell "vaccines" introduce a variety of antigens.

In an attempt to simplify and standardize this general approach and achieve highly specific immunity wherein only those clones of T cells that recognized the disease-associated antigen were affected, the inventor conceived of the present invention. It was recognized for the first time by the present inventor that an immunogenic peptide can be synthesized which mimics a portion of a disease-associated immunological "marker," such as the TCR of T cells involved in the disease process. Unexpectedly, immunization of a subject with the peptide directs the host immune response against the "marker" and thereby prevents or suppresses the development of the disease or treats the ongoing disease.

One hallmark of the invention is a method for selecting which peptide to use for preventing, suppressing or treating an immune-related disease, based on identifying the amino acid sequence of a marker TCR associated with the disease, predicting which segment of the TCR sequence is immunogenic based on several known algorithms, and determining which site or sites in the TCR structure is an appropriate target for an immune response which will result in protection from the disease.

One embodiment of the invention is a peptide having about 15-30 amino acids comprising an amino acid sequence of a marker T cell receptor characteristic of an immune-related disease, said peptide being capable of inducing protection from said disease and comprising at least part of the second complementarity determining region of said T cell receptor, or a functional derivative of said peptide, provided that the peptide does not have the sequence

```
Asp-Met-Gly-His-Gly-Leu-Arg-Leu-Ile-His-Tyr-Ser-Tyr-Asp-Val-Asn-Ser-
    Thr-Glu-Lys.
```

The invention also encompasses the peptide conjugated to a carrier, such as an additional heterologous amino acid sequence, in order to enhance the peptide's immunogenicity.

The invention is also directed to a pharmaceutical composition comprising the peptide or its functional derivative, in admixture with a pharmaceutically acceptable excipient.

Thus, the invention provides chemically defined peptides and therapeutics which can be specifically applied to designated immune-related diseases to disrupt the specific immunological responses responsible for induction or promotion of the disease process.

The diseases for which the invention is particularly useful include autoimmune diseases, such as rheumatoid arthritis, adjuvant arthritis, myasthenia gravis, encephalomyelitis, multiple sclerosis, thyroiditis, diabetes, inflammatory bowel disease and systemic Lupus erythematosus. The invention is also directed to malignant disease, such as T cell leukemias and lymphomas wherein the TCR serves as a tumor marker.

One embodiment of the invention is a method for selecting a peptide having an amino acid sequence of a T cell receptor wherein said T cell receptor is a marker T cell receptor characteristic of an immune-related disease, said peptide being capable of inducing protection from said disease, comprising the steps of:

(a) removing T cells from a subject susceptible to the disease;
(b) expanding the T cells of step (a) in culture in the presence of an autoantigen preparation;
(c) identifying the T cell receptor expressed by the expanded T cells of step (b); and
(d) selecting the peptide from the amino acid sequence comprising at least part of the second complementarity determining region of said T cell receptor.

The TCR may be identified through the use of TCR-specific antibodies or by determining the TCR amino acid sequence.

The invention further provides a method for preparing a peptide having an amino acid sequence of a TCR, wherein said T cell receptor is a marker T cell receptor characteristic of an immune-related disease, said peptide being capable of inducing protection from said disease, comprising the steps of:

(a) selecting a peptide, as described above: and
(b) synthesi ing the peptide or a functional derivative thereof.

Other embodiments of the invention are directed to polyclonal, monoclonal, or chimeric antibodies specific for the TCR peptide which are capable of protecting a subject from an immune-related disease, and to methods for preparing such antibodies. Also encompassed by this invention are the antibodies conjugated to cytotoxic agents, including ribosomal inhibiting proteins such as the ricin A chain.

The invention also includes the use of the above antibodies in the manufacture of a medicament for preventing, supressing or treating an autoimuune disease.

## BRIEF DESCRIPTION OF THE DRAWINGS

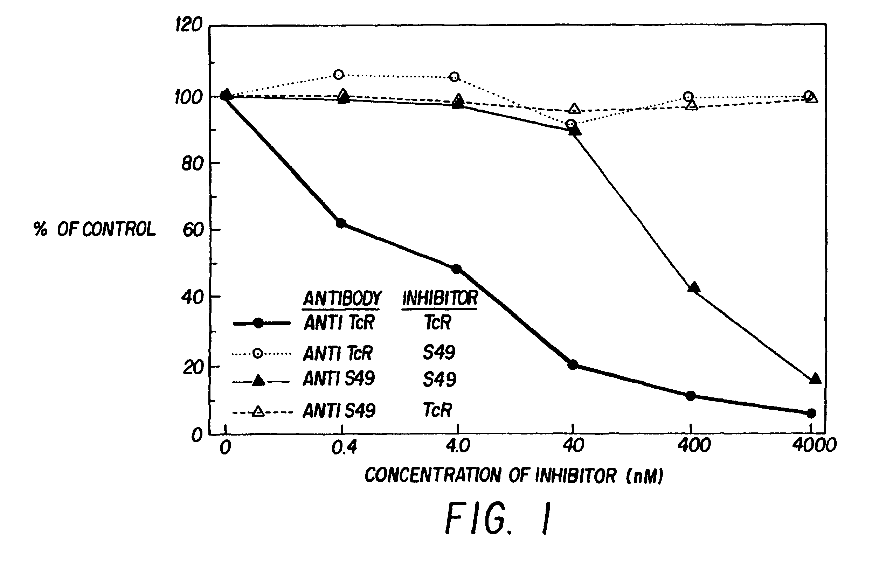

**Figure 1.** Peptide-specific inhibition of antibody reactivity. Antisera from 4 rats immuni ed with either the TCR Vβ8 (39-59) peptide alone or a combination of the TCR peptide and the GP-S49S MBP-derived autoantigen were pooled and diluted 40-360 fold. The antisera were tested for reactivity in direct ELISA with 25 ng peptide bound to microplate wells. This amount of peptide is equivalent to 10 pM TCR Vβ8(39-59) (MW = 2390 daltons) or 15 pM GP-549S (MW = 1630 daltons). Varying concentrations of inhibitor peptides were added in a range of 0.005 - 50 μg/well. The Absorbance measurements were determined in triplicate wells, and the reactivity calculated as the * of uninhibited control wells.

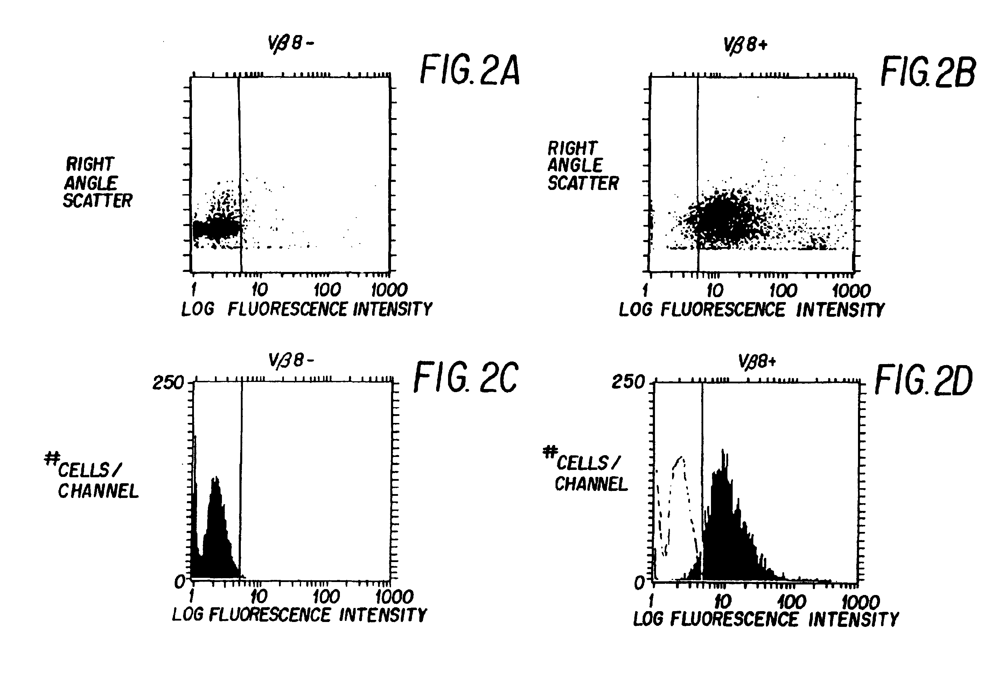

**Figure 2.** Antibodies to the TCR Vβ8(39-59) peptide stain Vβ8+ encephalitogenic T cells. Normal thymocytes (A and C) or GP-S49S-specific T line cells (B and D) were incubated with rabbit antibodies to TCR Vs8(39-59), followed by a mouse anti-rabbit IgG facilitating antibody and fluorescein-labeled goat anti-mouse IgG antibody. Flow cytometric analysis of staining was performed using a Coulter Epics C Cytofluorograph. A and C represent dot-plots of 10,000 cells showing cell size versus fluorescence intensity; B and D represent the corresponding histograms. The fluorescence intensity of T line cells stained with anti-TCR $V_\beta 8$(39-59) antibody (>90% stained) is increased compared to the 5% of normal thymocytes which stained with this antibody. Both thymocytes and T line cells incubated with normal rabbit IgG as a control for anti-TCR $V_\beta 8$(39-59) IgG showed background levels of staining (dotted line in box D).

**Figure 3.** Prevention, suppression and treatment of EAE with 50 μg TCR $V_\beta 8$-39-59 peptide/CFA. Rats were injected s.q. with the TCR peptide 40 days prior to, at the same time, or at disease onset 12 days after the induction of EAE with 50 μg GP-MBP/CFA.

**Figure 4.** Suppression of EAE with 50 μg TCR $V_\beta 8$-39-59 peptide given i.d. in the ear at the same time, or on days 7 or 11 after induction of EAE with 50 μg GP-MBP/CFA.

**Figure 5.** Treatment of EAE with 10 or 50 μg TCR VB8-39-59 peptide given i.d. in the ear at onset of clinical signs (day 12) after induction of EAE with 50 μg GP-MBP/CFA.

**Figure 6.** Peptide specificity of human MPB-specific T cell lines from MS pateints and normals.

**Figure 7.** Percentage of total proliferation response of T cell lines directed at each peptide compared to percentage of total clones responding to each peptide.

**Figure 8.** Cellular responses to TCR $V_\beta 8$- and $V_\beta 14$ peptides from EAE-recovered and TCR peptide-immunized rats. DTH is given in mm/100 and proliferation in CPM/1000, both background subtracted.

**Figure 9.** Treatment of relapsing EAE with TCR $V_\beta 17$ peptide.

## DESCRIPTION OF THE PREFERRED EMBODIMENTS

The compositions, methods, and products of this invention are applicable to human and veterinary uses.

The peptides of the present invention comprise sequences of about 13-30 amino acids which are immunogenic, that is, capable of inducing an immune response when injected into a subject.

By "functional derivative" is meant a "fragment," "variant," "analog," or "chemical derivative" of the peptide, which terms are defined below.

It is understood that the amino acid sequence comprising the peptide of this invention can be used alone or bound to, or contained within the sequence of, a longer peptide. The longer peptide may include sequences of an unrelated peptide, such as a carrier protein used to enhance the immunogenicity of the TCR oligopeptide. Such carriers are well known in the art and include heterologous proteins such as, for example, keyhole limpet hemocyanin (KLH), bovine serum albumin, tetanus toxoid and the like. Also included within the scope of this invention is the peptide conjugated to an antibody, and the peptide conjugated to a toxin. The toxins of this invention include the ribosomal inhibitory protein, such as, for example, the ricin A chain or Pseudomonas toxin.

As used herein, "marker TCR" refers to a TCR which is characteristic of a specified immune-related disease, such as autoimmune disease or malignant disease (i.e. cancer).

The term "immune-related disease" as used herein refers to a disease in which the immune system is involved in the pathogenesis of the disease, or in which appropriate stimulation of the immune system can result in protection from the disease. A preferred example of an immune-related disease to which this invention is directed is an autoimmune disease. Non-limiting examples of the autoimmune diseases contemplated by this invention are rheumatoid arthritis (RA), myasthenia gravis (MG), multiple sclerosis (MS), systemic lupus erythematosus (SLE), autoimmune thyroiditis (Hashimoto's thyroiditis), Graves' disease, inflammatory bowel disease, autoimmune uveoretinitis, polymyositis and certain types of diabetes.

Thus, a marker TCR for MS is a TCR which is capable of binding the complex between self MHC and the MBP fragment (or the MBP fragment alone), the MBP comprising the major autoantigen characteristic of this disease. In other autoimmune diseases, other TCRs serve as markers, as they are specific for the complex between MHC molecules and the autoantigens involved in these diseases. For example, in myasthenia gravis (MG), the autoantigen is thought to be the nicotinic acetylcholine receptor (AChR). Therefore, an identifiable TCR which binds AChR in the context of self MHC (or directly) and is expressed by AChR-reactive T cells which mediated the disease is a "marker TCR" for MG. Those of skill will recognize that determination of a marker TCR and of immunogenic peptides may be accomplished with the exercise of routine skill, using screening methods as are well-known in the art, when the teachings of the present invention are fully appreciated.

Also intended as immune-related diseases as used herein are malignancies wherein the tumor cell carries a tumor marker, such as a tumor antigen, capable of being recognized and responded to by the immune system. The TCR can serve as a tumor marker on T cell leukemia or T cell lymphoma cells.

In a subjected afflicted with, or susceptible to, an immune-related disease, introduction of the peptide carrying the amino acid seqeuence of a portion of the marker TCR results in generation of an immune response directed to the TCR and protection from the immune-related disease.

By the term "protection" from the disease as used herein is intended "prevention," "suppression" or "treatment" of the disease. "Prevention" involves administration of the protective composition prior to the induction of the disease. Thus, for example, in the animal model, EAE, successful administration of a protective composition prior to injection of the encephalitogen that induces the disease results in "prevention" of the disease.

"Suppression" involves administration of the composition after the inductive event but prior to the clinical appearance of the disease. Again, using the EAE example, successful administration of a protective composition after injection of the encephalitogen, but prior to the appearance of neurological symptoms comprises "suppression" of the disease.

"Treatment" involves administration of the protective composition after the appearance of the disease. In the EAE example, successful administration of a protective composition after injection of the encephalitogen and after clinical signs have developed comprises "treatment" of the disease.

It will be understood that in human medicine, it is not always possible to distinguish between "preventing" and "suppressing" since the ultimate inductive event or events may be unknown, latent, or the patient is not ascertained until well after the occurrence of the event or events. Therefore, it is common to use the term "prophylaxis" as distinct from "treatment" to encompass both "preventing" and "suppressing" as defined herein. The term "protection," as used herein, is meant to include "prophylaxis."

For the embodiment of the invention directed to autoimmune disease, the subject's immune response is directed to the particular TCRs which mark those T cells mediating the autoimmune process, and the peptides according to the invention thus are able to interfere with the binding of the MHC/antigen complex (or the antigen alone) needed for initiation or propagation of the autoimmune response.

In general, the peptide sequence represents a portion of the TCR itself and must be immunogenic, as defined below, corresponding to at least a part of the second complementarity determining region (CDR2) of the TCR $\alpha\beta$ heterodimer. Also intended within the scope of this invention are peptides corresponding to at least part of the second complementary determining region of the TCR $\gamma$ and TCR $\delta$ chains or their homologs in the $\gamma\delta$ heterodimer (see Strominger, J.L., Cell 57:895-898 (1989); and Clevers, H. et al., Ann Rev Immunol. 6:629-662 (1988)).

The CDRs of the TCR are defined by analogy to the structure of the immunoglobulin molecule wherein the CDRs comprised the amino acid sequences of the heavy or light chain variable regions which contacted antigen and constituted crucial portions of the antigen-binding site. All three TCR CDRs are believed to participate in binding to antigen and MHC (Davis, M.M., et al., Nature 334:395-402 (1988); Claverie, J.M., et al., Immun. Today 10:10-14 (1989)). By directing the immune response of the subject or the protective antibodies of this invention against the second complementary determining region of the 'marker TCR,' the likelihood of disrupting necessary binding or recognition events between the autoimmunity-associated T cell and the autoantigen and/or MHC is increased.

A "fragment" of the peptide of the present invention, refers to any subset of the molecule, that is, a shorter peptide.

A "variant" of the peptide refers to a molecule substantially similar to either the entire peptide or a fragment thereof. Variant peptides may be conveniently prepared by direct chemical synthesis of the variant peptide, using methods well-known in the art.

Alternatively, amino acid sequence variants of the peptide can be prepared by mutations in the DNA which encodes the synthesi ed peptide. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure (see European Patent Publication No. EP 75,444).

At the genetic level, these variants ordinarily are prepared by site-directed mutagenesis of nucleotides in the DNA encoding the peptide molecule, thereby producing DNA encoding the variant, and thereafter expressing the DNA in recombinant cell culture. The variants typically exhibit the same qualitative biological activity as the nonvariant peptide.

Preparation of a peptide variant in accordance herewith is preferably achieved by site-specific mutagenesis of DNA that encodes an earlier prepared variant or a nonvariant version of the TCR protein or peptide. Site-specific mutagenesis allows the production of peptide variants through the use of specific oligonucleotide sequences that encode the DNA sequence of the desired mutation, as well as a sufficient number of adjacent nucleotides, to provide a primer sequence of sufficient size and sequence complexity to form a stable duplex on both sides of the deletion junction being traversed. The technique of site-specific mutagenesis is well known in the art, as exemplified by Adelman et al., DNA 2:183 (1983). Typical vectors useful in site-directed mutagenesis include vectors such as the M13 phage, for example, as disclosed by Messing et al., Third Cleveland Symposium on Macromolecules and Recombinant DNA, Walton, A., ed., Elsevier, Amsterdam (1981). These phage are readily commercially available and their use is generally well known to those skilled in the art. Alternatively, plasmid vectors that contain a single-stranded phage origin of replication (Veira et al., Meth. Enzymol. 153:3 (1987)) may be employed to obtain single-stranded DNA.

In general, site-directed mutagenesis in accordance herewith is performed by first obtaining a single-stranded vector that includes within its sequence a DNA sequence that encodes the relevant peptide. An oligonucleotide primer bearing the desired mutated sequence is prepared, generally synthetically, for example, by the method of Crea et al., Proc. Natl. Acad. Sci. (USA) 75:5765 (1978). This primer is then annealed with the single-stranded protein-sequence-containing vector, and subjected to DNA-polymerizing enzymes such as E. coli polymerase I Klenow fragment, to complete the synthesis of the mutation-bearing strand. Thus, a mutated sequence and the second strand bears the desired mutation. This heteroduplex vector is then used to transform appropriate cells and clones are selected that include recombinant vectors bearing the mutated sequence arrangement. The mutated protein region may be removed and placed in an appropriate vector for protein production, generally an expression vector of the type that may be employed for transformation of an appropriate host.

An example of a terminal insertion includes a fusion of a signal sequence, whether heterologous or homologous to the host cell, to the N-terminus of the peptide molecule to facilitate the secretion of mature peptide molecule from recombinant hosts.

Another group of variants are those in which at least one aminoacid residue in the peptide molecule, and preferably, only one, has been removed and a different residue inserted in its place. Such substitutions preferably are made in accordance with the following list when it is desired to modulate finely the characteristics of a peptide molecule.

| Original Residue | Exemplary Substitutions | Original Residue | Exemplary Substitutions |
|---|---|---|---|
| Ala | gly; ser | Leu | ile; val |
| Arg | lys | Lys | arg; gln; glu |
| Asn | gln; his | Met | leu; tyr; ile |
| Asp | glu | Phe | met; leu; tyr |
| Cys | ser | Ser | thr |
| Gln | asn | Thr | ser |
| Glu | asp | Trp | tyr |
| Gly | ala; pro | Tyr | trp; phe |
| His | asn; gln | Val | ile; leu |
| lie | leu; val | | |

Substantial changes in functional or immunological properties are made by selecting substitutions that are less conservative than those in the above list, that is, by selecting residues that differ more significantly in their effect on maintaining (a) the structure of the peptide backbone in the area of the substitution, for example, as a sheet or helical conformation, (b) the charge or hydrophobicity of the molecule at the target site, or (c) the bulk of the side chain. The substitutions that in general are expected to those in which (a) glycine and/or proline is substituted by another amino acid or is deleted or inserted; (b) a hydrophilic residue, e.g., seryl or threonyl, is substituted for (or by) a hydrophobic residue, e.g., leucyl, isoleucyl, phenylalanyl, valyl, or alanyl; (c) a cysteine residue is substituted for (or by) any other residue; (d) a residue having an electropositive side chain, e.g., lysyl, arginyl, or histidyl, is substituted for (or by) a residue having an electronegative charge, e.g., glutamyl or aspartyl; or (e) a residue having a bulky side chain, e.g., phenylalanine, is substituted for (or by) one not having such a side chain, e.g., glycine.

Most deletions and insertions, and substitutions in particular, are not expected to produce radical changes in the characteristics of the peptide molecule. However, when it is difficult to predict the exact effect of the substitution, deletion, or insertion in advance of doing so, one skilled in the art will appreciate that the effect will be evaluated by routine screening assays. For example, a variant typically is made by site-specific mutagenesis of the peptide molecule-en-coding nucleic acid, expression of the variant nucleic acid in recombinant cell culture, and, optionally, purification from the cell culture, for example, by immunoaffinity adsorption on an anti-peptide antibody column (to absorb the variant by binding it to at least one epitope).

The activity of the cell lysate or purified peptide variant is then screened in a suitable screening assay for the desired characteristic. For example, a change in the immunological character of the peptide nolecule, such as binding to a given antibody, is measured by a competitive type immunoassay. Changes in T cell recognition of the variant peptide is measured by a DH assay in vivo or a T cell proliferation assay in vitro. Modifications of such peptide properties as redox or thermal stability, hydrophobicity, susceptibility to proteolytic degradation or the tendency to aggregate with carriers or into multimers are assayed by methods well known to the ordinarily skilled artisan.

An "analog" of a peptide refers to a non-natural molecule substantially similar to either the entire molecule or a fragment thereof.

A "chemical derivative" of a peptide of this invention contains additional chemical moieties not normally a part of the peptide. Covalent modifications of the peptides are included within the scope of this invention. Such modifications may be introduced into the molecule by reacting targeted amino acid residues of the peptide with an organic derivatizing agent that is capable of reacting with selected side chains or terminal residues.

Cysteinyl residues most commonly are reacted with $\alpha$-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, $\alpha$-bromo-$\beta$-(5-imidozoyl)propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Parabromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing $\alpha$-amino-containing residues include imidoesters such as methyl picolinimidate; pyridoxal phosphate; pyridoxal; chloroborohydride; trinitrobenzenesulfonic acid; 0-methylissurea; 2,4 pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high $pK_a$ of the guanidine functional group. Furthermore, these reagents may react with the groups of lysine as well as the arginine epsilon-amino group.

The specific modification of tyrosyl residues per se has been studied extensively, with particular interest in introducing spectral labels into tyrosyl residues by reaction with aromatic diazonium compounds or tetranitromethane. Most commonly, N-acetylimidizol and tetranitromethane are used to form 0-acetyl tyrosyl species and 3-nitro derivatives, respectively. Tyrosyl residues are iodinated using $^{125}$I or $^{131}$I to prepare labeled proteins for use in radioimmunoassay, the chloramine T method being suitable.

Carboxyl side groups (aspartyl or glutamyl) are selectively modified by reaction with carbodiimides (R'-N-C-N-R') such as 1-cyclohexyl-3-(2-morpholinyl-(4-ethyl) carbodiimide or 1-ethyl-3(4 azonia 4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Derivatization with bifunctional agents is useful for cross-linking the peptide to a water-insoluble support matrix or to other macromolecular carriers. Commonly used cross-linking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), and bifunctional maleimides such as bis-N-maleimido-1,8-octane. Derivatizing agents such as methyl-3-[(p-azidophanyl)dithio]propioimidate yield photoactivatable intermediates that are capable of forming crosslinks in the presence of light. Alternatively, reactive water-insoluble matrices such as cyanogen bromide-activated carbohydrates and the reactive substrates described in U.S. Patent Nos. 3,969,287; 3,691,016; 4,195,128; 4,247,642; 4,229,537; and 4,330,440 are employed for protein immobilization.

Other modifications include hydroxylation of proline and lysine, phosphorylation of hydroxyl groups of seryl or theonyl residues, methylation of the «-amino groups of lysine, arginine, and histidine side chains (T.E. Creighton, Proteins: Structure and Molecule Properties, W.H. Freeman & Co., San Francisco, pp. 79-86 (1983)), acetylation of the N-terminal amine, and, in some instances, amidation of the C-terminal carboxyl groups.

Such derivatized moieties may improve the peptide's solubility, absorption, biological half life, and the like. The moieties may alternatively eliminate or attenuate any undesirable side effect of the peptide and the like. Moieties capable of mediating such effects are disclosed, for example, in Remington's Pharmaceutical Sciences, 16th ed., Mack Publishing Co., Easton, PA (1980)

## Malignant Disease

Also susceptible to methods of the invention are lymphomas and leukemias. Lymphomas and many leukemias are tumors made up of lymphocytes that undergo uncontrolled proliferation (e.g., are malignant). Since several classes of leukemia and lymphoma are composed of T cells derived from a single malignant T cell precursor, all of the tumor cells bear the same TCR, which thus serves as a "tumor marker" which can be the target of protective compositions of this invention. Similarly, surface immunoglobulins can serve as tumor markers for B cell leukemias or lymphomas.

One embodiment of the invention is directed to the enhancement of an anti-tumor response by targeting the TCR of those T cells reacting against the "tumor marker" rather than the tumor marker itself. Thus, an immune response directed to the TCR on a tumor-specific T cell can be used to upregulate the antitumor response for the benefit of the host.

In fact, it will be appreciated that any disease involving a cell having a surface molecule that distinguishes that cell from other cells of the same histological type and from cells of a different histological type, contains a characteristic "marker," and will be susceptible to treatment by compositions which induce an immune response to the "marker," thereby altering activity of the cell bearing the "marker."

According to the present invention, the marker molecule itself may be relatively nonimmunogenic; it requires, at minimum, a characteristic antigenic epitope. This epitope itself may be inherently immunogenic, or it can be rendered immunogenic by treatments well known in the art, such as conjugation to an immunogenic carrier molecule. Thus, an epitope of a marker protein, either as a free peptide or in a form rendering it immunogenic, is capable of eliciting an antibody response, a cell-mediated immune response, or both, as conceived in the invention. Therefore, a composition which incorporates not the entire marker protein, but rather, a specific peptide region which is immunogenic or antigenic, will comprise a useful preparation for treating an immune-related disease characteri ed by this marker.

## Identification of Marker TCR-Bearing T Cells

The present invention utili es a synthetic peptide that represents at least part of the second complementarity determining region of the TCR having biological importance in ligand/MHC binding, and that is characteristic of a TCR V gene family. The invention therefore provides a much simpler approach for obtaining TCR V region-specific antibodies or T cells. Using other sequences from the same β chain to induce a spectrum of TCR V region-specific antibodies or T cells, those of skill will be able to map exposed epitopes of the TCR, and to establish the importance of these regions in ligand/MHC binding, with the exercise of routine skill.

Marker TCRs associated with a given disease are identified using known techniques. A genetic approach using patients known to have MG or MS was described by Oksenberg, J.R., et al., Proc. Natl. Acad. Sci. USA 86: 988-992 (1989). Sequences of the appropriate TCR β chain have been obtained by genomic analysis using restriction fragment length polymorphisms found in families having a prevalence of the particular autoimmune disease, as described by Seboun, E., et al., Cell 87:1095-1100 (1989); Burns, F.R., et al., J. ExD. Med. 169:27-39 (1989)).

It thus will be appreciated that, for the purposes of the present invention, determination of the marker TCR associated with an autoimmune disease and identification of peptides comprising an immunogenic sequence do not require that the autoantigen be characterized. It is sufficient that (a) the autoimmune disease involves a T cell-mediated immune response as a necessary part of the pathogenetic process, and (b) the disease has an organ-, tissue- or cell-specific target. In fact, as is known in the art (see, for example, Theofilopoulos, A., supra), the autoimmune disease may not involve an autoantigen at all at the inductive stage, but rather, may represent a response to an exogenous antigen, such as a bacterial or viral antigen, which is cross-reactive with self antigens, or results in an immunopathologic response directed to the exogenous antigen present in the host.

T cells recognizing an autoantigen or autoimmune disease-associated antigen (such as certain viral or bacterial antigens) are cloned, and may be fused to an immortalizing cell, such as a long term T cell line, a T cell lymphoma line or a T cell hybridoma, and are grown in culture. The cultured cells serve as the source of cDNA encoding the appropriate TCR. Such cDNA is cloned and expressed by methods well known in the art. (See, for example, Maniatis, T, et al., Molecular Cloning: A Laboratory Manual, (1982)).

In addition to the foregoing approaches, it will be appreciated that advantage may be taken of animal models to identify the TCR variable region loci which are associated with the autoimmune disease. Animals which are susceptible to any of a number of autoimmune diseases, non-limiting examples of which include EAE, experimental MG, experimental autoimmune thyroiditis, adjuvant arthritis, collagen-induced arthritis, and the like, have a particular TCRvariable locus associated with the disease which can be identified in vitro.

By the term "susceptible to a disease" is intended a state in which the animal possesses a gene or genes known to be associated with the disease, thereby increasing the risk that the individual with that gene or genes will develop that disease compared to the general population. Genes known to be associated with autoimmune diseases, for example, include MHC genes (especially class II), immunoglobulin V genes, TCR V genes, and the like. The term "susceptible" is also intended to encompass those individuals who actually have the disease. While a complete correlation between an autoimmune disease and the usage of a particular TCR is neither expected nor necessary to successfully practice the present invention, high correlations of about 60-70% have been found for presence or expression of a particular variable region gene and susceptibility to autoimmune disease in animals.

In an alternate embodiment of this invention, T cells isolated from humans who are susceptible to an autoimmune disease, and in particular susceptible individuals who have the autoimmune disease, are expanded in culture. Techniques for T cell expansion are described by Zamvil et al., Nature 317:355-358 (1985), and Nature 324:258-260 (1986).

In one embodiment employing this method, patient peripheral blood lymphocytes are removed and stimulated with the autoantigen or a specific peptide derived therefrom or related thereto, which is capable of stimulation comparable to that of the autoantigen. The autoantigen (or related peptide) is added to the lymphocyte cultures for several days. In one embodiment, cells are simulated with autoantigen for 5-6 days. In other embodiments, cells are stimulated for longer periods of time. The time required for stimulation is a function of the proportion of reactive cells in the blood sample, the activation state of these cells, and the potency of the stimulating preparation, and is readily determinable by one of skill in the art. After culture under such selective conditions, about $5 \times 10^5$ viable cells are isolated and restimulated with about $3 \times 10^7$ autologous antigen-presenting cells (irradiated to prevent their proliferation, such as with about 2500-4500 rad) and about 20 µg/ml of the autoantigen (or related peptide). About 7 days later, viable cells are collected and cloned by limiting dilution in the presence of about $10^3$ - $10^6$ antigen presenting cells, for example about $5 \times 10^5$ antigen-presenting cells, and human IL-2 or crude or pure combinations of lymphocyte growth factors (such as, for example, IL-4). The cells of such a T cell line are expanded and grown in tissue culture flasks for about one to two weeks. Such lines can be multiply restimulated with antigen-presenting cells, autoantigen preparations, and IL-2. Restimulation can typically be carried out once a week. If desired, such T cells can be cloned by any of a number of methods known in the art, such as, for example, limiting dilution or by picking cells from colonies growing in soft agar, generally about 2 days after restimulation.

In another embodiment, lymphocytes from an organ or body fluid are first cultured in the presence of IL-2. Under these conditions, selection will occur for cells already activated and only such cells grow. Subsequently, such T cells are stimulated with antigen-presenting cells and an autoantigen preparation. Using this approach, MBP-specific T cells from the spinal cord of rats with EAE can be selectively expanded in vitro.

As used in the present invention, the term "autoantigen" is not intended to be limiting to a defined or known macromolecule. For example, in the case of type I diabetes, the particular antigen associated with pancreatic islet (or beta) cells that is the trigger or target antigen of the T cell-mediated autoimmune response is unknown. For the present invention, the autoantigen used to stimulate cells in vitro, as described above, can comprise whole pancreatic islet cells, crude membrane preparations derived from such cells, partially purified purified membrane components, or when identified, the diabetogenic autoantigen. The same is true for other autoimmune diseases for which a unique autoantigen has not yet been identified, including Hashimoto's thyroiditis, arthritis in which the autoantigen is not collagen, Sjogren's disease, polymyositis, arteriosclerosis, and the like. One of skill will appreciate that as long as the immunogenic moiety to which the T cells respond is present in the stimulatory preparation, the methods of the invention can be carried out as described.

The presence of autoantigen-specific reactive T cells in the cloned, expanded T cell population can be readily determined by testing the ability of the cells to be activated in the presence of the autoantigen. Many assays are available, and well known in the art, to measure early or late events in the T cell activation process. Example of such methods include, but are not limited to, T cell proliferation (which can be measured as the uptake of radiolabeled thymidine), the secretion of interleukin-2, intracellular calcium mobilization, translocation of particular membrane enzymes involved in inositol phosphate metabolism, and changes in expression of cell surface molecules (which can be determined by flow cytometry).

The TCR expressed by a T cell clone responding to a particular autoantigen can be identified using TCR-specific antibodies, either polyclonal, monoclonal or chimeric (see below) which are specific for a TCR variable segment. to detect surface expression, employing techniques of fluorescence microscopy, flow cytometry, immunocytochemistry, or other techniques known in the art. Such antibodies have been described for a number of TCR a $_\beta$ chain V regions (see, for example, Owhashi, M., et al., supra; Gascoigne, N.R.J., et al., supra; Kappler, J.W., et al., 1987, 1988 (supra); and MacDonald, H.R., supra).

Alternatively, the DNA or mRNA of the T cell clone can be probed directly, or after amplification by the polymerase chain reaction (Synha et al., Science 239:1026 (1988); Saiki et al., Nature 324:163 (1986), by specific hybridization with nucleic acid probes for the various TCR gene families, using hybridization methods well known in the art. The TCR sequence, or a part thereof, can then be obtained directly from the amplified, rearranged DNA or mRNA.

Expression of a particular TCR can also be identified by determining the nucleic acid sequence encoding at least part of the TCR, for example, after cloning the TCR V gene, or by determining the amino acid sequence of at least part of a TCR protein. It will be apparent that any of the abovementioned approaches, or additional approaches known to one of skill in the art, will result in the identifying of the TCR expressed on a T cell or clone or line of T cells. This information is needed for the selection of an amino acid sequence which comprises the peptide or pharmaceutical preparations of this invention.

Where no specific autoantigen has been identified, the oligoclonality of T cells in the anatomic region associated with the disease can be used as a basis for enrichment of reactive T cells. For instance, cells uniquely associated with rheumatoid arthritis are found in the synovial fluid of the joint; cells uniquely associated with MS are found in the cerebrospinal fluid (CSF); and disease-associated T cells infiltrate the thyroid tissue in Hashimoto's thyroiditis and in Graves' disease. In these instances, T cells are isolated from the relevant anatomical location, and the cells expanded in culture as described above. (See also, Londei, M. et al., Science 228:85-89 (1985); Londei, M. et al. Acta Endocrinol. 115(suppl. 281):86-89 (1987); Stamenkovic, I. et al. Proc. Natl. Acad. Sci. USA 85:1179-1183 (1988); Lipoldova, M. et al. J. Autoimmun. 2:1-13 (1989); Oksenberg, J.R., et al., supra). The DNA or mRNA of such cells is isolated, cDNA prepared, and the differences in sequences of cDNA encoding the variable TCR loci are established by comparison of afflicted with unafflicted subjects. As an alternative to expanding the cells in culture, cellular DNA or, preferably, cDNA made from mRNA, can be obtained directly from T cells isolated from the subject, and the nucleic acid expanded by the PCR reaction, as above.

The antigens associated with a number of human and animal model autoimmune disease are presently known. Type II collagen and Mycobacterium tuberculosis 65 kD heat shock protein are antigens associated with rheumatoid arthritis; AChR is associated with MG, and with experimental allergic myasthenia gravis (EAMG) which can be induced in mice. Thyroglobulin is known to be the antigen associated with experimental allergic thyroiditis (EAT) in mouse. A similar disease, Hashimoto's thyroiditis involves an immune response to an antigen on thyroid follicular cells. In Graves' disease, the immune response is directed to the thyrotropin receptor on thyroid cells. Myelin basic protein (MBP) and proteolipid protein (PLP) are known to be associated with experimental allergic encephalomyelitis (EAE) in mouse and rat. EAE is a recognized model for multiple sclerosis in humans.

Therefore, those of skill will appreciate that the present invention is directed in one aspect to identification of peptides useful for prevention or therapy of human and animal diseases, including but not limited to those mentioned above.

Selection of Antigenic Peptides

An important embodiment of this invention comprises the combined method of identifying a TCR associated with an autoimmune disease, determining which oligopeptide sequence including at least part of the second complementarity determining region of the TCR is both immunogenic and important for T cell action in the disease process, synthesizing that peptide, and using it as a therapeutic agent.

Regions of relevant TCR sequences are identified for synthesis on the basis of their predicted antigenic or immunogenic properties. By the term "immunogenic" is intended the capacity of a peptide to induce an immune response, either T cell-mediated, antibody, or both. By the term "antigenic" is intended the capability of a peptide to be recognized, in free form by antibodies and in the context of MHC molecules in the case of antigen-specific T cells. Regions of a protein or peptide that are likely to be immunogenic or antigenic for T cells are identified, for example, using the approaches and algorithms described by Margalit, H. et al. (J. Immunol. 138:2213-2229 (1987) and Rothbard, J.B. et al. EMBO J. 7:93-100 (1988)). The Margalit et al. approach is based on analysis of immunodominant helper T cell antigenic sites leading to development of an algorithm based on an amphipathic helix model, in which antigenic sites are postulated to be helices with one predominantly polar and one predominantly apolar face. The approach of Rothbard et al., recognizes motifs similar to epitopes recognized preferentially by T helper or T cytotoxic cell clones, which can predict accurately areas within protein sequences that are capable of being recognized by MHC class I and II molecules, such recognition being assumed as necessary for T cell immunogenicity and antigenicity.

The regions of the TCR which are of immunoregulatory importance for the purposes of this invention include at least a part of the second complementarity determining region.

The use of the above approach to select peptide sequences for use in treating EAE in rats exemplifies the success of this approach. For example, a peptide comprising 16 amino acids corresponding to CDR1 of the marker TCR for EAE in Lewis rats, Vβ8 (25-41) was predicted by the above algorithms not to be immunogenic for T cells. In fact, this peptide does not induce T cell immunity and does not protect Lewis rats from EAE. A peptide corresponding to the CDR1 of a different TCR β chain which is not associated with EAE, Vβ14(25-41), was predicted to be immunogenic for T cells, and indeed was found to induce T cell immunity in Lewis rats, but, as expected, did not protect from EAE. Similarly the CDR2 peptide, Vβ14(39-59), corresponding to an TCR not associated with EAE, was predicted to be immunogenic, and did induce immunity, but, again, did not protection from EAE. According to the invention, the CDR2-related peptide of the relevant TCR, Vβ8(39-59), was predicted to be both immunogenic and protective in EAE, and indeed, was shown to be so (see Examples, below).

The size of the peptide selected for use in this invention is largely determined by the requirement for immunogenicity, while maintaining the minimal epitope structure such that a T cell or antibody specific for the peptide will recognize and react with the TCR on an intact T cell. For example, peptides of this invention, in order to be sufficiently immunogenic and to have a high probability of including the relevant epitope of the TCR which can lead to modulation of T cell activity, are of the range of about 15-30 amino acids, although peptide fragments of differing length are also contemplated. The successful use of a 21 amino acid TCR peptide present on the TCR $_\beta$ chain associated with EAE in rats to treat EAE according to the methods of this invention is amply demonstrated in the Examples below.

Immunogenicity of peptides useful in the present invention can be screened by well-known methods, such as use of a DH response in an animal. In such a response, an animal is "sensitized" by injection of an appropriate dose of an antigen, typically subcutaneously (SC), frequently with an adjuvant, such as complete Freund's adjuvant (CFA). Generally about 5-15 days later, the response is "elicited" by challenging the animal, typically intradermally (ID), with an appropriate dose of the antigen, typically in saline or other buffer. The response is assessed 24-48 hours later. Non-limiting examples of assay methods which measure DH include size of erythema (redness) and induration (swelling) at the site of antigen injection, ear swelling, footpad swelling, tail swelling, accumulation of systemically injected $^{125}$I-labeled iododeoxyuridine in the challenge site, accumulation of intravenously (IV) injected radiolabeled serum protein, such as albumin, in the challenge site, and accumulation of IV-injected labeled inflammatory cells, such as lymphocytes or neutrophils, in the challenge, site. For example, an ear swelling response upon appropriate ID challenge in the ear pinna of about 0.15-0.25 mm, and preferably about 0.20 mm (in a Lewis rat) represents a positive DH response. One skilled in the art will understand that variations in peptide size, dose, route of sensitization or elicitation of DH, carriers used, adjuvants used, etc., will affect the timing and extent of the DH response.

For a peptide to be considered immunogenic, as intended here, a dose of about 10-200 µg per animal, and preferably about 25-100 µg of the peptide per animal should be able to sensitize an animal for a DH response. Furthermore, in a sensitized animal, a dose of about 1-100 µg, and preferably about 5-50 µg, of the peptide is able to elicit a DH response upon ID challenge.

Synthesis of Peptides and Assay

The desired peptides, with sequences determined as described above, are prepared using standard synthesis techniques including solution and solid phase sequential amino acid conjugation and recombinant production in prokaryotic or eukaryotic hosts. Verification that the peptide prepared is immunogenic can easily be determined using a DH reaction in an animal (e.g., mouse or rat), as described above. The peptide is administered subcutaneously, and the animal is challenged about 9-14 days later ID in the ear pinna. The ear swelling response, measured 24 or 48 hours after challenge, serves as a simple and reliable measure of the presence of T cell-mediated immunity to the peptide.

Verification of the ability of the immunogenic peptide to actually modulate autoimmunity may be attained using an appropriate animal model, taking into account the species differences in the TCR-related peptides. For example, although it is preferred to use a sequence representing the CDR2 of a human marker TCR in treating humans, the corresponding region of the marker TCR for the animal disease model is used in the animal disease.

Animal model systems which can be used to screen the effectiveness of the peptides in protecting against or treating the disease are available, as discussed above. Of course, the identical peptides may not be effective in humans since they may not correspond to an appropriate site of the disease-associated human TCR, or may not be sufficiently immunogenic in humans. It is to be understood that modifications of the peptide sequence delineated in a particular animal model may be required in order to treat subjects belonging to other species, including humans. Thus, verification that, for example, a particular CDR2-associated peptide sequence is effective in protecting against a particular disease can be obtained in these models, leading to predictions that the corresponding human sequence would be a preferred candidate as an effective peptide therapeutic for humans. Determination of the corresponding TCR sequence in the human (or in different non-human animal species), using approaches described above, thus permits modification of the peptide for use in the human (or other species).

The following is a non-exclusive list of animal disease models of human autoimmune diseases with which a TCR peptide can be assessed for its ability to modify disease, and to induce antibodies and T cells which are capable, upon transfer, of modifying disease. Systemic lupus erythematosus (SLE) is tested in susceptible mice as disclosed by Knight et al., J. Exp. Med. 147:1653 (1978) and Reinertsen et al., N. Eng. J. Med. 299:515 (1978). MG is tested in SJL/J female mice by inducing the disease with soluble AChR protein from another species as described in Lindstrom, J., et al., Adv. Immunol. 42:233-284 (1988). Arthritis is induced in a susceptible strain of mice by injection of Type II collagen as described by Stuart, J.M., et al., Ann. Rev. Immunol. 2:199-218 (1984). Adjuvant arthritis is induced in susceptible rats by injection of Mycobacterial heat shock protein as described by Van Eden, W., et al., Nature 331: 171-173 (1988). Thyroiditis is induced in mice by administration of thyroglobulin as described by Maron, R., et al., J. Exp. Med. 152: 1115-1120 (1980). Insulin-dependent diabetes mellitus (IDDM) occurs naturally or can be induced in certain strains of mice such as those described by Kanasawa et al., Diabetologia 27:113 (1984). Other mouse strains can be caused to exhibit this disease by transferring lymphocytes from this strain.

EAE in mouse and rat serves as a model for MS in humans. In this model, the demyelinating disease is induced by administration of myelin basic protein (MBP) or proteolipid protein (PLP), or Theiler's virus, as described by Paterson, P.Y., Textbook of Immunopathology (Mischer et al., eds.), Grune and Stratton, New York, pp. 179-213 (1986); McFarlin, D.E., et al., Science 179:478-480 (1973); and Satoh, J., et al., J. Immunol. 138:179-184 (1987).

For measuring preventative, suppressive, or therapeutic benefit of the compositions of this invention in humans, certain clinical outcome measures are used. In MS, for example, quantitative parameters include: (a) clinical disability, (b) on-study exacerbation rate, and (c) magnetic resonance imaging (MRI) brain plaque load (which is an important recent parameter used to evaluate MS patients). These measures involve separate blinded examination or unblinded examination by a treating physician. Neuropsychological measures of cognitive impairment are used as an independent determinant of disability. Clinical disability is typically measured by the McAlpine Scale, the Kurtzke Score, a modification Kurtzke Score termed the Expanded Disability Status Score (EDSS). An improvement of ½ unit on the EDSS (Range of 1-9) is considered significant. One clinical measure, the patients ability to walk, is rated by the Ambulation Index, wherein an improvement of 1 or more units is considered significant. These clinical measures are well known in the art and are described in detail in McAlpine, D., et al., Multiple Sclerosis, Livingston Press, Edinburgh (1955); Binken, P.J. et al., Handbook of Clinical Neurology, Volume 9, Amsterdam-North Holland Publishers, Amsterdam (1970); and Field, E.J. et al., Multiple Sclerosis: A Critical Review, M.M.T.P Press, Ltd., Lancaster, England, (1977).

Measurement of improvement in RA, for example, is based on a number of primary clinical endpoints, including resolution or reduction of swelling, reduction in duration of morning stiffness, decreased erythrocyte sedimentation rate and/or C-reactive protein, resolution of rheumatoid-associated conditions as rheumatoid nodules, and reduction in lymphocyte counts. Secondary endpoints include reduction in fatigue and improvement in overall condition as assessed by the patient and the physician. Clinical outcomes are divided into the following: (a) Complete Response - greater than 90% decrease in joint swelling, tenderness, and morning stiffness; (b) Marked Response - 50-90% decrease in joint swelling, tenderness, and morning stiffness; (c) Moderate Response - 30-50% decrease in joint swelling, tenderness, and morning stiffness; and (d) No Response - ≤ 30% decrease in joint swelling, tenderness, and morning stiffness.

Similar measurements which allow evaluation of the preventive, suppressive, or treatment effects of the peptides, antibodies, T cells and other compositions of the present invention in additional immune-related disease are known to those of skill in the art.

Passive Immunity

In addition to the use of a TCR peptide for active immunization, further embodiments of the present invention involve antibodies specific for the TCR peptide, for passive transfer of anti-TCR immunity. Passive antibody-mediated immunity may involve any of a number of effector mechanisms, such as, for example, antibody-dependent cellular cytotoxicity, or complement-dependent cytotoxicity. Alternatively, the antibody is used to deliver a toxic agent in a specific manner, such as ricin A chain, for example.

For passive vaccination, a subject animal is injected with the appropriate peptide, as described below, and the peripheral blood lymphocytes or lymphocytes from another organ, such as a draining lymph node, are harvested. B cells may be recovered from the initial cell population taken from the TCR peptide-immunized animal, and immortalized by fusion to cell line fusion partners using standard techniques to produce hybridomas for production of monoclonal antibodies specific for the TCR peptide. Hybridomas producing appropriate antibodies are screened by conventional immunoassays, such as direct ELISA assays, for reactivity with the TCR peptide antigen or with the relevant T cells.

Monoclonal antibodies (mAbs) to specific antigens, such as the TCR peptides of this invention, may be obtained by methods known to those skilled in the art. See, for example, Kohler and Milstein, Nature 256:495-497 (1975) and U.S. Patent No. 4,376,110. Such antibodies may be of any immunoglobulin class, including IgG, IgM, IgE, IgA, IgD, and any subclass thereof. Alternatively, antibodies can be prepared from polyclonal antisera taken from animals immunized with the TCR peptide, subjected to various purification schemes known in the art, and used directly for passive transfer of anti-TCR immunity.

Monoclonal antibodies of rodent origin are "humanized" by linking a cDNA molecule encoding the V region of the mAb to DNA encoding the human constant region, using any of several approaches described in Cabilly et al., U.S. Patent 4,816,567 (3/28/89) and Eur. Patent Pub. EP125023 (11/14/84); Taniguchi et al., Eur. Patent Pub. EP171496 (2/19/86); Morrison et al., Eur. Patent Pub. EP173494 (3/5/86); Neuberger et al., PCT Pub. WO8601533 (3/13/86); Kudo et al., Eur. Patent Pub. EP184187 (6/11/86); Robinson et al., PCT Pub. WO 8702671 (5/7/87); Cabilly et al., Proc. Natl. Acad. Sci. USA81:3273-3277 (1984); Morrison et al., Proc. Natl. Acad. Sci. USA81:6851-6855 (1984); Boulianne et al., Nature 312:643-646 (1984); Morrison, Science, 229:1202-1207 (1985); Neuberger et al., Nature 314: 268-270 (1985); Takeda et al., Nature 314:452-454 (1985); Tan et al., J. Immunol. 135:3564-3567 (1985); Jones et al., Nature 321:522-525 (1986); Oi et al., BioTechniques 4:214 (1986); Sahagan et al., J. Immunol. 137:1066-1074 (1986); Sun et al., Hybridoma 5 (Supp. 1):S17-S19 (1986); Sun et al., Proc. Natl. Acad. Sci. USA 84:214-218 (1987); Liu et al., Proc. Natl. Acad. Sci. USA 84:3439-3443 (1987); Liu et al., J. Immunol. 139:3521 3526 (1987); Better, M., et al., Science 240:1041-1043 (May 20, 1988); and Horwitz, A. H., et al., Proc. Natl. Acad. Sci. USA 85:8676-8682 (1988)). The preferred method of chimeric antibody production combines five elements: (1) Isolation of messenger RNA (mRNA) from a mouse B cell hybridoma line producing the monoclonal antibody, cloning and cDNA production therefrom; (2) Preparation of a full length cDNA library from purified mRNA, from which the appropriate variable (V) region gene segments of the light (L) and heavy (H) chain genes can be (i) identified with appropriate probes, (ii) sequenced, and (iii) made compatible with a constant (C) region gene segment; (3) Preparation of C region gene segment modules by cDNA preparation and cloning; (4) Construction of complete H or L chain coding sequences by linkage of the cloned specific immunoglobulin V region gene segments described in (2), above, to cloned human C region gene segment modules described in (3); and (5) Expression and production of chimeric L and H chains in selected hosts, including prokaryotic and eukaryotic cells.

Many vector systems are available for the expression of cloned H and L chain genes in mammalian cells (see Glover, D.M., ed., DNA Cloning. Vol. II, pp143-238, IRL Press, 1985). Different approaches can be followed to obtain complete $H_2L_2$ antibodies. It is possible to co-express H and L chains in the same cells to achieve intracellular association and linkage of H and L chains into complete tetrameric $H_2L_2$ antibodies. The co-expression can occur by using either the same or different plasmids in the same host. Genes for both H and L chains can be placed into the same plasmid, which is then transfected into cells, thereby selecting directly for cells that express both chains. Alternatively, cells may be transfected first with a plasmid encoding one chain, for example the L chain, followed by transfection of the resulting cell line with an H chain plasmid containing a second selectable marker. Cell lines producing $H_2L_2$ molecules via either route can be transfected with plasmids encoding additional copies of H, L, or H plus L chains, in conjunction with additional selectable markers, to generate cell lines with enhanced properties, such as higher production of assembled $H_2L_2$ antibody molecules or enhanced stability of the transfected cell lines.

The chimeric antibodies of this invention have both the TCR-recognizing specificity of the mouse mAb and the biological properties of human antibodies, which include resistance to clearance in the human and much less immunogenicity (allowing multiple treatments).

The anti-TCR peptide antibodies (polyclonal, monoclonal and chimeric) of this invention can be used therapeutically as immunoconjugates (see for review: Dillman, R.O., Ann. Int. Med. 111:592-603 (1989)). They can be coupled to cytotoxic proteins, including ribosomal inhibitory proteins such as Ricin-A, Pseudomonas toxin, and Diphtheria toxin, as well as other proteins such as tumor necrosis factor. Toxins conjugated to antibodies or other ligands, are known in the art (see, for example, Olsnes, S. et al., Immunol. Today 10:291-295 (1989)). An additional example of such a conjugated antibody is XomaZyme[R]-CD5 Plus, which is an anti-CD5 mAb conjugated to ricin A chain. This preparation is effective in prophylaxis and therapy of graft-versus-host disease, and of refractory rheumatoid arthritis in humans. This particular toxin-conjugated antibody is specific for most T lymphocytes and a subset of B lymphocytes. Cells having the CD5 marker drop rapidly in response to treatment. Since antibody to a TCR peptide will react with a much smaller proportion of total lymphocytes, higher doses of an anti-TCR antibody conjugated to ricin A will be tolerated by patients, or conversely, lower doses will be effective. Effective doses of a ricin A conjugated monoclonal antibody to a TCR peptide are in the range of about 0.005 to 0.5 mg/kg/day, with the preferred dose in the range of about 0.05 to 0.2 mg/kg/day.

The anti-TCR peptide antibodies of this invention can be conjugated to additional types of therapeutic moieties including, but not limited to, radionuclides and cytotoxic drugs, to treat individuals with autoimmunity or with malignant or lymphoproliferative disease. Non-limiting examples of radionuclides which can be coupled to antibodies and delivered in vivo to sites of antigen include $^{212}Bi$, $^{131}I$, $^{186}Re$, and $^{90}Y$. Such radionuclides exert their cytotoxic effect by locally irradiating the cells, leading to various intracellular lesions, as is well-known in the art of radiotherapy.

Cytotoxic drugs which can be conjugated to antibodies and subsequently used for in vivo therapy include, but are not limited to, daunorubicin, doxorubicin, methotrexate, and mitomycin C. Cytotoxic drugs interfere with critical cellular processes including DNA, RNA, and protein synthesis. For a fuller exposition of these classes of drugs which are known in the art, and their mechanisms of action, see Goodman, A.G., et al. , Goodman and Gilman's The Pharmacological Basis of Therapeutics, 7th Ed., Macmillan Publishing Co., (1985).

Treatment of an individual using the antibodies, fragments or derivatives of this invention comprises parenterally admininstering a single or multiple doses of the antibody, fragment or derivative thereof. The effective dose is a function of the individual antibody, the presence and nature of a conjugated therapeutic agent, the subject and his clinical status, and can vary from about 10 ng/kg body weight to about 100 mg/kg body weight. The route of administration may include IV, SC, intramuscular, intrapulmonary, intraperitoneal (IP), intranasal, intrathecal, intradermal, transdermal or other known routes.

Formulation of Peptides

The preclinical and clinical therapeutic use of the present invention in the treatment of disease or disorders will be best accomplished by those of skill, employing accepted principles of diagnosis and treatment. Such principles are known in the art, and are set forth, for example, in Braunwald, E. et al., eds., Harrison's Principles of Internal Medicine, 11th Ed., McGraw-Hill, publisher, New York, N.Y. (1987).

The peptides and compositions of the present invention, or their functional derivatives, are well suited for the preparation of pharmaceutical compositions. The pharmaceutical compositions of the invention may be administered to any animal which may experience the beneficial effects of the compositions of the invention. Foremost among such animals are humans, although the invention is not intended to be so limited.

The pharmaceutical compositions of the present invention may be administered by any means that achieve their intended purpose. For example, administration may be by parenteral, subcutaneous, intravenous, intradermal, intramuscular, intraperitoneal, transdermal, or buccal routes. Alternatively, or concurrently, administration may be by the oral route. The peptides and pharmaceutical compositions can be administered parenterally by bolus injection or by gradual perfusion over time.

The dosage administered will be dependent upon the age, sex, health, and weight of the recipient, kind of concurrent treatment, if any, frequency of treatment, and the nature of the effect desired.

The dose ranges for the administration of the compositions of the present invention are those large enough to produce the desired effect, whereby, for example, an immune response to the peptide, as measured by DH or antibody production, is achieved, and the immune-related disease is significantlyl prevented, suppressed, or treated. The doses should not be so large as to cause adverse side effects, such as unwanted cross reactions, generalized immunosuppression, anaphylactic reactions and the like.

Preferred doses for humans range between about 0.001 - 25 mg/kg body weight.

In addition to peptides of the invention which themselves are pharmacologically active, pharmaceutical compositions may contain suitable pharmaceutically acceptable carriers comprising excipients and auxiliaries which facilitate processing of the active compounds into preparations which can be used pharmaceutically. Preferred compositions include the inclusion of an adjuvant, such as alum, or other adjuvants known in the art. (See, for example, Warren, H. S. et al., Ann. Rev. Immunol. 4:369-388 (1986); Chedid, L., Feder. Proc. 45:2531-2560 (1986)).

To enhance delivery or bioactivity, the peptides can be incorporated into liposomes using methods and compounds known in the art.

Preparations which can be administered orally in the form of tablets and capsules, preparations which can be administered rectally, such as suppositories, and preparations in the form of solutions for injection or oral introduction, contain from about 0.001 to about 99 percent, preferably from about 0.01 to about 95 percent of active compound (s), together with the excipient.

Suitable excipients are, in particular, fillers such as saccharides, for example lactose or sucrose, mannitol or sorbitol, cellulose preparations and/or calcium phosphates, for example tricalcium phosphate or calcium hydrogen phosphate, as well as binders such as starch paste, using, for example, maize starch, wheat starch, rice starch, potato starch, gelatin, tragacanth, methyl cellulose, hydroxypropylmethylcellulose, sodium carboxymethylcellulose, and/or polyvinyl pyrrolidone.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

Possible pharmaceutical preparations which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatin rectal capsules which consist of a combination of the active compounds with a base. Possible base materials include, for example, liquid triglycerides, polyethylene glycols, or paraffin hydrocarbons.

Suitable formulations for parenteral administration include aqueous solutions of the peptides in water-soluble form, for example, water-soluble salts. In addition, suspensions of the peptides as appropriate oily injection suspensions may be administered. Suitable lipophilic solvents or vehicles include fatty oils, for example, sesame oil, or synthetic fatty acid esters, for example, ethyl oleate or triglycerides. Aqueous injection suspensions may contain substances which increase the viscosity of the suspension include, for example, sodium carboxymethyl cellulose, sorbitol, and/or dextran. Optionally, the suspension may also contain stabilizers.

The peptides are formulated using conventional pharmaceutically acceptable parenteral vehicles for administration by injection. These vehicles are nontoxic and therapeutic, and a number of formulations are set forth in Remington's Pharmaceutical Sciences, (supra). Nonlimiting examples of excipients are water, saline, Ringer's solution, dextrose solution and Hank's balanced salt solution. Formulations according to the invention may also contain minor amounts of additives such as substances that maintain isotonicity, physiological pH, and stability.

The peptides of the invention are preferably formulated in purified form substantially free of aggregates and other protein materials, preferably at concentrations of about 1.0 ng/ml to 100 mg/ml.

Effective doses of the peptides of this invention for use in preventing, suppressing, or treating an immune-related disease are in the range of about 1 ng to 100 mg/kg body weight. A preferred dose range is between about 10 ng and 10 mg/kg. A more preferred dose range is between about 100 ng and 1 mg/kg.

The immunogenicity of the peptide may be enhanced by including it in a longer peptide or chain or by conjugating it to "immunological" carriers, such as KLH, serum albumin, tetanus toxoid, and the like, using standard linking techniques. A variety of such methods is known in the art, e.g., use of condensing agents such as dicyclohexylcarbodiimide or use of linkers, such as those commercially available from Pierce Chemical Co., Rockford, IL.

Doses of TCR peptide-specific antibodies vary as a function of antibody species origin, isotype, affinity, nature (polyclonal, monoclonal, chimeric) and other characteristics which are known to one of skill. For example, a monoclonal antibody to a TCR peptide will be administered at a dose of between 0.01 - 50 mg/kg.

The following examples are intended to be illustrative, but not to limit, the invention.

## EXAMPLE 1

### COMPOSITIONS OF PEPTIDES USEFUL TO TREAT EAE, AS A MODEL OF MS

Introduction

EAE is a well-recognized rat model of the human autoimmune disease, multiple sclerosis. Accordingly, the utility of the present invention was demonstrated by showing that the administration of the peptide representing the appropriate CDR2 peptide of the TCR which, in rats, is a marker TCR for EAE, prevents EAE in these animals. In this model, the disease is induced by injecting the subject rat with an encephalitogenic form of myelin basic protein, such as, for example, guinea pig basic protein (GPBP) or a synthetic peptide that corresponds to residues 72-89 of GPBP (GPBP (72-89)). Injection of either of these peptides in complete Freund's adjuvant (CFA) induces encephalitogenic T cell clones that utilize preferentially the rat homologs of mouse TCR $V_\alpha 2$ and $V_\beta 8$ genes (Chou, Y.K., et al., J. Neurosci. Res. 22:181-187 (1989); Burns, F.R., et al., J. Exp. Med. 169:27-39 (1989)).

The inventors reported the complete nucleotide and deduced amino-acid sequence for the rearranged rat $TCR_\alpha$ and $_\beta$ chain genes (with sequence homology to the mouse $V_\alpha 2$ and $V_\beta 8$ families respectively) used in response to the major encephalitogenic epitope of basic protein, GPBP(72-89)( Burns, F.R., et al., supra). Within the TCR $V_\beta 8$ region, a 21-amino acid sequence was identified and synthesized that included the second complementarity determining region (CDR2) and was predicted to be immunogenic for T cells (based on the algorithms of Margalit et al. and Rothbard et al. (supra).

This peptide has the sequence: Asp-Met-Gly-His-Gly-Leu-Arg-Leu-Ile-His-Tyr-Ser-Tyr-Asp-Val-Asn-Ser-Thr-Glu-Lys-Gly and is termed "TCR $V_\beta 8$(39-59)."

A control peptide was synthesized from the corresponding region for a different TCR $V_\beta$ sequence that was homologous to the mouse $V_\beta 14$ family (Williams et al., supra).

Specific Immunity to TCR Peptide

Four rats were immunized by subcutaneous (SC) injection of 400 µg TCR peptide in CFA (100 µg Mycobacterium/rat) and peptide-specific immune responses were measured after 30 days.

To measure antibodies specific for TCR $V_\beta 8$(39-59) peptide, serum from the immunized rats was tested by direct ELISA. THe TCR peptide was coated onto plastic microplates (25 ng of TCR peptide/well). Serum dilutions were added and the plates incubated for 2 hours. The reaction was developed by addition of peroxidase-conjugated antibodies specific for Ig H and L chains. A chromogenic substrate for peroxidase was added and the colored reaction product was measured as the absorbance at 405 nm ($A_{405}$) using a colorimetric plate reader.

A 1:200 dilution of immune serum gave an absorbance of $0.63 \pm 0.12$ units. Control sera from rats immunized with the control peptide (derived from the corresponding CDR2 region of an unrelated TCR chain, $V_\beta14$) gave a reaction of only $0.02 \pm 0.01$ units. Thus, a specific antibody response was obtained to the TCR peptide.

In addition, the rats showed a specific T cell response in vivo, measured as a delayed hypersensitivity (DH) reaction to intradermal (ID) challenge with the $V_\beta8(39-59)$ TCR peptide, but not with the $V_\beta14$ peptide.

Table 1

Immunization with TCR $V_\beta8$ Peptide Prevents Induction of EAE

| Immunization Protocol[2] | EAE Induction[1] | | | |
|---|---|---|---|---|
| | Incidence | Day of Onset | Duration | Severity[3] |
| TCR $V_\beta8$ peptide | 0/10 | -- | -- | -- |
| TCR $V_\beta14$ peptide | 4/4 | 14±2 | 6±2 | 3.1±0.3 |
| Saline | 14/14 | 14±2 | 6±1 | 3.0±0.5 |

[1] EAE was induced by SC challenge with 50 μg GPBP + 400 μg Mycobacteria in complete Freund's adjuvant (CFA) 30 days after TCR peptide (or saline) immunization.

[2] Rats were injected SC with either: (1) 100 μg of the TCR peptide [DMGHGLRLIHYSDVNSTEKG (single-letter code)] representing residues 39-59 of the rat cDNA clone $V_\beta510$, homologous to the mouse $V_\beta8$ family (Burns et al., J. Exp. Med. 169:27-39 (1989)); (2) 100 μg of TCR peptide [APGGTLQQLFYSFNVGQSELF] representing residues 39-59 of the rat cDNA clone CRTB188 homologous to the mouse $V_\beta14$ family (Williams, C.B. et al., J. Immunol. 142:1037-1035 (1989)); or (3) saline. The peptides or saline were mixed with CFA containing 100 μg Mycobacteria prior to injection.

[3] Values represent the mean of the maximum severity of EAE. 0, no signs; 0.5, lethargy, weight loss; 1, limp tail; 2, hind-leg weakness; 3, hind-quarter paralysis, incontinence; 4, moribund.

TCR Peptide-Specific Immunity Protects Against Clinical EAE

In addition to showing specific immunity toward the TCR peptide, the peptide-immunized rats were found to be protected against clinical EAE.

Immunization of Lewis rats with the TCR $V_\beta8(39-59)$ peptide, but not with the TCR$V_\beta14$ peptide or saline, prevented completely the induction of EAE (Table 1). The $V_\beta8(39-59)$-immunized rats developed both specific antibodies to the $V_\beta8(39-59)$ peptide and a delayed hypersensitivity (DH) response of 0.17 mm ear swelling to 50 μg TCR $V_\beta8$ (39-59) peptide. The control $V_\beta14$ peptide also induced specific immunity to itself but did not confer protection against EAE.

TCR Peptide-Specific Immunity Generates Specific T Cells

In addition to demonstration of antibody production, DH, and protection against EAE, the TCR peptide elicited demonstrable antigen-specific (i.e., TCR peptide-specific) T cells.

Rats were immunized SC with 400 µg TCR $V_\beta 8(39\text{-}59)$ peptide in CFA (containing 1 mg M. tuberculosis) and were challenged SC with either 50 µg GPBP in CFA at the same time or with 100 µg of GPBP 30 days later. Twenty days after the simultaneous challenge, draining lymph nodes (LN) were removed and lymphocyte suspensions prepared.

A fraction of the cells were tested for proliferative response to antigens or mitogens in vitro ($5 \times 10^5$ cells/well).

The remainder of the cells were restimulated in bulk culture (in 6 cm. diameter petri dishes) with the appropriate TCR peptide (50 µg/ml) for 3 days followed by transfer to IL-2 rich medium for an additional 4 days. These cells were subsequently tested for proliferative responses to antigens or and mitogens by restimulation in the presence of irradiated thymic accessory cells (($2 \times 10^4$ cells/well). In some cases, stimulation by TCR peptide was carried out in the presence of 2 µg/well monoclonal antibodies. Results are shown in Table 2 (underlined values show statistically significant responses).

Lymph node (LN) cells isolated from the protected rats responded to the TCR $V_\beta 8(39\text{-}59)$ peptide as well as to GPBP and PPD (purified protein derivative of M. tuberculosis). This was further evidence for the concurrent presence of TCR-specific as well as autoantigen-specific T cell reactivity.

T-cell lines were selected from the LN of the protected rats that responded specifically to the $V_\beta 8(39\text{-}59)$ but not to the $V_\beta 14$ peptide (Table 2). The TCR $V_\beta 8(39\text{-}59)$-specific T cells were strongly positive by immunofluorescence for the CD4 marker and weakly positive for the CD8 marker. The proliferative response to the $V_\beta 8(39\text{-}59)$ peptide was restricted only by MHC class I molecules.

A GPBP-specific T-cell line was also selected from protected rats immunized with both the TCR $V_\beta 8(39\text{-}59)$ peptide and GPBP. This line had an uncharacteristically low response to the encephalitogenic 72-89 peptide in comparison to its responsiveness to GPBP. Once selected and activated, GPBP-specific T cell line cells from TCR peptide-protected rats were encephalitogenic (administration of $10^7$ cells caused hind-leg paralysis in 3 rats), indicating that TCR $V_\beta 8$ (39-59) peptide immunization did not result in the deletion of precursors of encephalitogenic T cells.

Mixing of the TCR $V_\beta 8(39\text{-}59)$-specific and BP-specific T cells did not impair the response to GPBP, even in the presence of the TCR peptide (Table 2). The presence of TCR

Table 2

| Specificity of T Cell Lines Derived from Rats Protected from EAE by the TCR $V_\beta 8$ Peptide | | | | |
|---|---|---|---|---|
| | | T Cell Proliferation (cpm x $10^{-3}$)[1] | | |
| Stimulant | LN | TCR $V_\beta 8$ peptide selected line | GPBP-selected | TCR $V_\beta 8$ line |
| | | | + GPBP line | |
| Medium | 11 | 2 | 1 | 6 |
| Con A | 99 | 96 | 80 | 123 |
| TCR $V_\beta 8(39\text{-}59)$ | 27 | 100 | 1 | 140 |
| +OX-6 (anti I-A) | -- | 99 | -- | -- |
| +OX-17 (anti I-E) | -- | 100 | -- | -- |
| +OX-18 (anti class I) | -- | 31 | -- | -- |
| +W3/25 (anti-CD4) | -- | 105 | -- | -- |
| +OX-8 (anti-CD8) | -- | 22 | -- | -- |
| TCR $V_\beta 14$ peptide | 10 | 2 | -- | -- |
| GPBP | 16 | 3 | 93 | 102 |
| 1-37 | 13 | -- | 1 | 13 |
| 44-89 | 20 | -- | 46 | 56 |
| 44-68 | 15 | 1 | 2 | 22 |
| 72-89 | 16 | 1 | 27 | 20 |

Table 2 (continued)

| Specificity of T Cell Lines Derived from Rats Protected from EAE by the TCR $V_\beta$ 8 Peptide | | | | |
|---|---|---|---|---|
| | | T Cell Proliferation (cpm x $10^{-3}$)[1] | | |
| Stimulant | LN | TCR $V_\beta$8 peptide selected line | GPBP-selected | TCR $V_\beta$8 line |
| | | | + GPBP line | |
| + OX-6 | -- | -- | 1 | -- |
| + OX-17 | -- | -- | 23 | -- |
| + OX-18 | -- | -- | 27 | -- |
| + W3/25 | -- | -- | 1 | -- |
| + OX-8 | -- | -- | 26 | -- |
| 87-99 | 12 | -- | 1 | -- |
| 90-170 | 13 | -- | 1 | 14 |
| GPBP + $V_\beta$8(39-59) | 30 | -- | 55 | 149 |
| PPD | 48 | 2 | 1 | -- |
| Rat BP | 11 | 1 | 9 | 9 |
| Underlined values indicate significant stimulation. --, not done. | | | | |

$V_\beta$8(39-59)-specific T cells, however, caused an increased response to all of the peptides of GPBP except the encephalitogenic 72-89 sequence. The TCR peptide-specific T cells therefore altered the peptide recognition pattern of GPBP-reactive T cells, which provides evidence of the existence of cell-cell interactions.

Direct Interactions Between TCR Peptide-Specific and BP-Specific T Cells

T cells from the LN of the immunized rats were tested for responsiveness in vitro to attenuated $V_\beta8^+$ or $V_\beta8^-$ T cells. The stimulator T cells were irradiated (2500R), and 2 x $10^4$ cells were cultured (in the absence of additional accessory cells) with 2 x $10^5$ isolated TCR-specific T cells for 3 days, pulsed for the last 18 hours with $^3$H-thymidine, and isotope uptake was measured by liquid scintillation spectroscopy.

In the absence of a stimulator T cell line, "background" responses were on the order of 7000 cpm (Table 3). When the stimulator line was specific for the GPBP S72-89 epitope, and therefore expressed the Vs8 TCR, the response was 31,000 cpm. However, the when the stimulator line was specific for the GPBP 55-74 peptide, and therefore did not express the $V_\beta$8 TCR, there was no significant response above background (8000 cpm). Thus, only $V_\beta8^+$ cells could be recognized by T cells specific for the TCR peptide, indicating the presence of direct recognition of the $V\beta$8 peptide on the target T cell by a regulatory $V_\beta$8-specific T cell. The results indicate the direct recognition of the TCR sequence on the surface of the stimulator T cell. The TCR peptide-specific T cells, however, were not cytotoxic for the BP-reactive target cells.

Passive Transfer of EAE Protection bv TCR Peptide-Specific T Cells

The protective ability of $V_\beta$8(39-59) peptide-specific T cells was established by adoptive transfer. Rats injected with as few as $10^7$ $V_\beta$8(39-59)-specific T cells did not develop EAE (Table 4). The transferred protection appeared to be T-cell mediated; $V_\beta$8(39-59)-specific antibodies were not detectable in the serum of protected rats. DT results (Table 4) indicated that the adoptively transferred T cells could prevent the induction of EAE without compromising T cell recognition of other antigens.

## Table 3

### Response of TCR $V_\beta 8$ Peptide-specific T Cell Line to Attenuated $V_\beta 8^+$ or $V_\beta 8^-$ T Cells

| Specificity of Stimulator Line | $V_\beta 8$ Expression of Stimulator Line | Proliferation of Responder T Cell Line (cpm x $10^{-3}$) |
|---|---|---|
| None added | | $7 \pm 2$ |
| GPBP (S72-89) | + | $31 \pm 3$ |
| GPBP (55-74) | - | $8 \pm 1$ |

T cell line cells were irradiated (2,500 R) and $2 \times 10^4$ cells (as stimulators) were mixed with $2 \times 10^5$ TCR-specific responder T cells for 3 days. Cultures were pulsed with $^3$H-thymidine for the final 18h, the cells were harvested, and proliferation assessed as $^3$H-thymidine uptake. Background proliferation of irradiated T cells specific for GPBP (S72-89) and GPBP (55-74) was 0.1 and 0.2 cpm (x $10^{-3}$), respectively.

EP 0 552 142 B2

Table 4

TCR Vβ8 Peptide-specific T-cells Protect against EAE

| Transfer dose | Induction of EAE (GPBP/CFA) | | | | DH (mm × 10⁻²) | |
|---|---|---|---|---|---|---|
| | Incidence | Day of Onset | Duration | Severity² | GPBP | PPD³ |
| None | 5/5 | 12 | 6.5 | 3.1 | 32 | 21 |
| 3 × 10⁷ | 0/5 | -- | -- | 0 | 24 | 21 |
| 1 × 10⁷ | 0/4 | -- | -- | 0 | ND | ND |

1 T cell line cells were stimulated with TCR Vβ8 peptide plus thymic accessory cells for 3 days prior to intraperitoneal transfer into naive recipient rats. The recipient rats were challenged on the same day with GPBP/CFA.
2 Values represent the mean of the maximum severity of EAE. See legend to Table 1.
3 Ear swelling in response to ID challenge with GPBP or PPD was measured 24 h. after challenge and represents a DH response to the antigen.

Specificity of T Cell Lines Derived From Protected Rats

The ability of $V_\beta 8$(39-59)-specific T cells to (a) alter the response patterns of GPBP-specific T cell lines in vitro, (b) protect naive rats from EAE, and (c) reduce DH reactions in vivo, indicated that the pattern of response to BP epitopes might be altered in rats protected by TCR peptide-specific T cells. As shown in Table 5, LN cells from the EAE-protected animals responded well to the TCR peptide of GPBP as compared to LN cells from the control group. In contrast, LN cells from the protected group showed a significant response to the 87-99 peptide of BP, whereas LN cells from the control group did not respond to this peptide. The selection of a TCR $V_\beta 8$(39-59)-specific T cell line from the LN of adoptively protected rats (Table 5) indicated that TCR peptide-specific T cells had migrated to and persisted in the LN that drained the site of GPBP injection.

DISCUSSION

These results demonstrate for the first time the use of a synthetic peptide from the CDR2 region of the TCR to induce $V_\beta 8$-specific regulatory T cells that prevent the induction of EAE. Computer modeling of ternary interactions among TCR chains, antigenic peptides, and MHC restriction molecules is consistent with CDR involvement in peptide/MHC binding when the TCR is folded in an energetically favorable conformation (Davis et al. and Claverie et al., supra). The regulatory effects of T cells specific for CDR2 support the notion that this region has biological importance. Although the inventors do not intend to be bound by any particular theory, it seems unlikely that the responder T cell interacts directly with the functional TCR Vs8 molecule on the target T cell surface. Indeed, it is conceivable that endogenous TCR peptide could be 'processed" and expressed preferentially on the T-cell surface in association with class I molecules (Long, E.O., Immunol. Today 10:232-234 (1989)). If a natural form of the TCR peptide is associated with the MHC molecule on the T cell surface, the interacting TCR-specific T cell could interfere with normal T cell activation by a BP epitope.

Table 5

| Antigen Specificity of T Cell Lines Derived from the Lymph Nodes of Rats Protected from EAE by the Transfer of TCR Vβ8 Peptide-specific T cells | | | | |
|---|---|---|---|---|
| Transfer Protoco/[1] | Stimulant | Proliferation (cpm x $10^{-3}$) | | |
| | | LN[2] | TCR Peptide Selected Line[3] | GPBP Selected Line |
| A. TCR $V_\beta$8 peptide-specific T Cells | Medium | 7 | 2 | 5 |
| | Con A | 125 | 46 | 39 |
| | $V_\beta$8 peptide | 90 | 52 | 6 |
| | GPBP | 80 | 0 | 16 |
| | 1-37 | 7 | NO | 6 |
| | 44-89 | 12 | NO | 17 |
| | 44-68 | 9 | 4 | 9 |
| | 72-89 | 12 | 3 | 11 |
| | 87-99 | 41 | 4 | 9 |
| | 90-170 | 12 | ND | 8 |
| B. Saline | Medium | 12 | Not | 6 |
| | Con A | 38 | selected | 88 |
| | $V_\beta$8 peptide | 8 | | 6 |
| | G P BP | 32 | | 65 |
| | 1-37 | 12 | | 6 |
| | 44-89 | 34 | | 91 |
| | 44-68 | 19 | | 24 |
| | 72-89 | 29 | | 64 |
| | 87-99 | 11 | | 6 |
| | 90-170 | 11 | | 7 |

[1] Lymph node (LN) cells were collected 20 days after simultaneous injection of (A) 3 x $10^7$ TCR $V_\beta$8 peptide-specific T line cells, or (B) saline, along with GPBP/CFA.

[2] LN cells were tested directly (LN column).

[3] T cell lines were selected from these LN cells by culture with TCR $V_\beta$8 peptide (second column) or G PBP (third column) followed by IL2 as described in Underlined values indicate significant stimulation.

Vaccination with attenuated T cells indicates that protective immunity is induced against target structures shared by different T cell clones specific for the same disease-inducing epitope, but do not implicate the TCR directly. The immunogenicity and immunoregulatory activity demonstrated here of a defined region of the TCR $V_\beta$8 chain expressed by encephalitogenic T cells is an important step forward in understanding anti-idiotypic regulation, and provides a clear explanation for the protective effects of the peptide immunization approach. The approach of the present invention, using a synthetic peptide to induce TCR peptide-specific antibodies, is of value in producing a variety of highly specific antibodies for assessing sequences important in TCR function. The potential regulatory properties of antibodies raised to the $V_\beta 8(39\text{-}59)$ peptide are illustrated in Example II.

TCR peptide vaccination has application in human autoimmune or malignant conditions that are characterized by common TCR V-gene usage.

## EXAMPLE II

ANTIBODIES AGAINST A SYNTHETIC TCR V REGION PEPTIDE SUPPRESS EAE

This Example provides an evaluation of the effects of immunization with the TCR $V_\beta 8(39\text{-}59)$ peptide on EAE induced with the encephalitogenic guinea pig basic protein (GPBP) peptide, S87-99. and on antibody responses to GPBP peptides S49S or 587-99. Antibody responses against the TCR $V_\beta 8(39\text{-}59)$ peptide are described and the ability of these antibodies to react with $V_\beta 8^+$ T cells and to suppress clinical signs of EAE are evaluated.

The results demonstrate that the TCR $V_\beta 8(39\text{-}59)$ peptide can induce both protection against EAE and elevated titers of antibody specific for either of the GPBP epitopes which are encephalitogenic in Lewis rats. Furthermore, anti-TCR $V_\beta 8(39\text{-}59)$ antibodies are shown to suppress EAE independent of regulatory T cells. Thus, both humoral and cellular regulatory mechanisms are generated after immunization of the Lewis rats with the TCR $V_\beta 8(39\text{-}59)$ peptide.

### A. MATERIALS AND METHODS

#### 1. Peptide synthesis and purification.

All peptides used in this study were synthesized by a minor modification (Hashim, G.A., et al., J. Neurosci. Res. 16:467 (1986)) of the solid phase method (Merrifield, R.B., J. Amer. Chem. Soc. 85:2149 (1963)) using Boc-amino acid-resin-ester (Peninsula Laboratories, San Carlos, CA). The peptides (Table 6) were synthesized with t-Boc-L-amino acid derivatives, starting with t-Boc-L-Glycine-0-resin ester (0.65 mmole/g: 0.78 mmole). Coupling and deblocking reactions were routinely monitored by the Kaiser test (Kaiser, E., et al., Anal. Biochem. 34:595 (1970)) for free amino groups. Single deblocking and occasional double coupling reaction steps were sufficient for the synthesis of all peptides used in this study. Peptide Gp-S49S defines region 69-84 of GPBP and has a C-terminal glycine. Residue numbers of GPBP peptides used in this study correspond to those reported for bovine myelin basic protein (Eylar, E.H., et al., J. Biol. Chem. 246:5770 (1971)).

Peptides containing tryptophan were first treated with 10% piperidine for 30 minutes to remove the formyl blocking groups and then, like all other peptides, were cleaved from the resin, together with other side chain deprotection, by treatment with HF at 0°C in the presence of anisole. After removal of the HF, the resin-peptide mixture was washed 4 times with ether and dried. The peptide was extracted with water, lyophilized and filtered through a Sephadex G10 column (2.5 X 100 cm) that was equilibrated with and eluted by 5% acetic acid. Acid insoluble peptides were extracted from the resin-peptide mixture with 0.03 M ammonium carbonate, filtered on a Sephadex G10 column that was equilibrated with and eluted by 0.03 M ammonium bicarbonate, and lyophilized. Further purification of the peptide was achieved using HPLC with a Bondapak C18 column equilibrated with 0.1% trifluoroacetic acid (TFA) in water and eluted with a linear gradient up to 40% acetonitrile containing 0.1% TFA over a period of 60 minutes. The purity of the peptides was documented by HPLC and by amino acid composition analysis.

#### 2. Test and control Peptides.

The TCR $V_\beta 8(39\text{-}59)$ peptide was synthesized according to the sequence identified by Burns, F.R., et al. (J. Exp. Med. 169:27 (1989)). As controls for TCR $V_\beta$ gene family specificity and for the CDR2 hypervariable region, additional peptides were synthesized, including TCR $V_\beta 14(39\text{-}59)$, which comprises the corresponding CDR2 of the $V_\beta 14$ gene family (Williams, C.B., et al., J. Immunol. 142:1027 (1989)), and TCR $V_\beta B(25\text{-}41)$, corresponding to a sequence in the CDR1 region adjacent to peptide TCR $V_\beta 8(39\text{-}59)$ (Burns, F.R., et al., op. cit.). Other control peptides included a series that defines specific regions of GPBP. Peptides Gp-S49S and Gp-S87-99 define respectively the major and minor encephalitogenic sequences for Lewis rats. Peptides Gp-S67 (residues 69-81 ) and Gp-S53 (residues 75-84) define respectively T cell and B cell epitopes within the major encephalitogenic epitope encompassed in peptide Gp-S49S (residues 69-84). Peptide Gp-S55-74 defines a non-encephalitogenic T cell determinant in Lewis rats (Offner, H., et al., J. Exp. Med. 170:355 (1989)), and Gp-NAc-1-16 encompasses the encephalitogenic sequence for the PL/J strain of mouse (Zamvil, S.S., et al., Nature 324:258 (1986)).

3. <u>Peptide coupling to KLH.</u>

Keyhole limpet hemocyanin (KLH) (Calbiochem Corp., La Jolla, CA) was dissolved in phosphate buffered saline (PBS), dialyzed against PBS at 4°C overnight and lyophilized. A known weight of KLH (8 mg or 1-2 μmoles) together with the peptide to be coupled (10 μmoles) were dissolved in 1 ml deionized water. After the pH was adjusted to 4.5 with 0.01 N HCl, 375 mg of 1-ethyl-3 (3-dimethylaminopropyl)-carbodiimide (Pierce Chemical Co., Rockford, IL) in 0.5 ml water were added and the reaction mixture was stirred for 1 hour at room temperature. The mixture was then placed in dialysis bags and dialyzed against 3 changes of PBS at 4°C and lyophilized. The amount of peptide coupled to KLH was calculated from the increase in mass of the non-dialyzable portion of the KLH.

### Table 6

#### Amino Acid Sequence of Peptides Derived from the TCR and Related Peptides from Myelin Basic Protein

---

TCR $V_\beta 8(39-59)$:

```
  39 40            45              50              55            59
  AspMetGlyHisGlyLeuArgLeuIleHisTyrSerTyrAspValAsnSerThrGluLysGly
```

TCR $V_\beta 8(25-41)$:

```
  25          30            35          40 41
  LysGlnAsnAsnAsnHisAsnAsnMetTyrTrpTyrArgGlnAspMetGly
```

TCR $V_\beta 14(39-59)$:

```
  39 40            45              50              55            59
  AlaProGlyGlyThrLeuGlnGlnLeuPheTyrSerPheAsnValGlyGlnSerGluLeuVal
```

TCR $V_\beta 14(24-41)$:

```
  24            30              35            40 41
  ThrValLysGlyThrSerAsnProAsnLeuTyrTrpTyrTrpGlyAlaProGly
```

```
                69 70 71 72 73 74 75 76      79 80 81 82 83 84
  Gp-S49S:      GlySerLeuProGlnLysSerGln-----ArgSerGlnAspGluAsn
  Gp-S53 :                        SerGln-----ArgSerGlnAspGluAsn
  Gp-S67 :      GlySerLeuProGlnLysSerGln-----ArgSerGln
```

```
                55           60      63   65                70          74
  Gp-(S55-74):  SerGlyLysAspSerHisHisAlaThrArgThrThrHisTyrGlySerLeuProGlnLys
```

```
                87      90            95          99
  Gp-(S87-99):  ValHisPhePheLysAsnIleValThrProArgThrPro
```

```
                1           5           10              16
  Gp-NAc(1-16): N-Ac-AlaSerGlnLysArgProSerGlnArgHisGlySerLysTyrLeuAla
```

---

All peptides were synthesized by the solid phase method, purified by gel filtration and high pressure liquid chromatography as described in the methods section. Peptides from the TCR are numbered according to Burns <u>et al.</u> (<u>supra</u>) and Williams et al. (<u>supra</u>) and guinea pig myelin basic protein (GPBP) peptides are numbered according to Eylar <u>et al.</u> (<u>J. Biol. Chem.</u> 246:5770 (1971)). Peptide Gp-(S55-74) has an unnatural threonine for alanine substitution at position 63.

4. <u>Preparation of anti-peptide antibodies.</u>

Male Lewis rats weighing 200-250 g were immunized with a single dose of 100 μg of the free peptide. The peptide was emulsified in complete Freund's adjuvant (CFA) and injected SC. Each rat received 100 μl of the emulsion containing 100 μg peptide and 100 μg <u>M. butyricum.</u> Likewise, Lewis rats were immunized with 100 μg of a particular

peptide and challenged either simultaneously or at a later date with another peptide. Immunized rats were pre-bled from the tail vein before and at intervals after immunization.

New Zealand white rabbits, weighing 6-7 lbs., were pre-bled and immunized with 0.5 ml CFA emulsion containing 4 mg peptide and 2 mg M. butyricum. The emulsion was injected SC in multiple sites in the dorsal area of the neck and the tail. Rabbits were immunized either with the free peptide or with peptide conjugated to KLH. Immunized rabbits were boosted on days 7, 14 and 21 with 1 mg peptide emulsified in incomplete Freund's adjuvant and injected SC on the flank. All rabbits were bled via the ear vein after they were placed in restraining cages and tranquilized with ace-promozine. To prevent hypovolemia, the amount of blood removed was replaced with sterile saline. Sera from individual rats or rabbits were prepared from clotted blood by centrifugation. All sera were decomplemented for 30 minutes at 56°C and frozen in small aliquots to which sodium azide was added.

### 5. Preparation of immunoglobulin.

IgG was prepared from serum by published methods (Steinbuch, M., et al., Arch. Biochem. Biophys. 134:279 (1969)) and purified by ion exchange chromatography with DEAE-sephadex. The serum was diluted with one volume of 0.06 M acetate buffer and the pH adjusted to 4.8 at room temperature. Caprylic acid, 6.8 g/100 ml serum, was added dropwise under vigorous stirring for 30 minutes. The mixture was then centrifuged, the supernatant was adjusted to pH 5.7, dialyzed against deionized water and lyophilized.

### 6. Antibody assays.

Antibody reactivity was determined by an adaptation for peptides (Hashim, G.A., et al., J. Neurosci. Res. 24: 222 (1989)) of the direct enzyme-linked immunosorbent assay (ELISA) and by the inhibition ELISA as described by Cleveland, W.L., et al. (Methods in Enzymol. 121:95 (1986)). Peroxidase-labeled rabbit anti-rat or goat anti-rabbit immunoglobulin (affinity-purified Hand L chains, Cooper Biomedical, Malvern, PA) was used together with 0-phenylenedi-amine for enzyme substrate, and the optical density was measured at 450-650nm in a colorimetric plate reader (Model Vmax, Molecular Devices, Mento, CA).

### 7. EAE Induction.

EAE was induced in male Lewis rats (225-250 g) as described (Hashim, G.A., et al., J. Neurosci. Res. 24: 222 (1989)). Each rat received a single SC injection of a CFA emulsion containing 100 μg peptide and 100 μg M. butyricum. Immunized rats were inspected daily for clinical signs of EAE and were terminated between days 25 and 30 following challenge. At this time, sera from individual rats were collected, and the brain and spinal cord tissues were processed for histology.

### 8. Prevention and suppression of EAE in Lewis rat.

Male Lewis rats were immunized with 100 μg TCR $V_\beta 8(39-59)$ peptide emulsified in CFA and injected SC. The immunized rats were bled from the tail for antibody determination and were challenged with 100 μg of an encephalitogenic peptide (Gp-S49S or Gp-S87-99). Groups of rats were challenged either on the same day or on day 40-41 after they were immunized, based on the observed time course of anti-TCR $V_\beta 8(39-59)$ antibody production.

To study EAE suppression by anti-TCR $V_\beta 8(39-59)$ antibodies, Lewis rats were challenged with the encephalitogenic peptide Gp-S49S and injected intraperitoneally with either saline (control) or either Lewis rat or rabbit anti-TCR $V_\beta 8(39-59)$ IgG, given every other day for 14 days. Each rat received a total of either 49 or 70 mg rat or rabbit IgG, respectively, and was terminated on day 24 after the challenge. When injected in sterile saline over a period of 14 days, rabbit IgG remained at high levels in the circulation of recipient rats on days 12 and 24 after transfer and did not interfere with the development of anti-Gp-S49S antibodies.

### 9. Antibody staining of $V_\beta 8^+$ and $V_\beta 8^-$ T cells.

Rat or rabbit IgG (10 μg) from normal or TCR $V_\beta 8(39-59)$ immunized animals was incubated at various concentrations for 30 minutes with $10^6$ normal rat thymocytes (known to be mostly $V_\beta 8^-$) or a Gp-S49S-reactive, GPBP-specific T cell line (known to be $V_\beta 8^+$). After several washes, the cells were incubated with 10 μg mouse anti-rat or anti-rabbit IgG for an additional 30 minutes as an amplification step. After further washing, the cells were stained with fluoresceinated goat anti-mouse IgG (H + L chain specific), washed, fixed in 2% formalin, and evaluated for fluorescence intensity at 488 nm using a Coulter Epics C Cytofluorograph. The cells were gated electronically on the basis of forward angle versus right angle scatter patterns to include the major lymphocyte populations, which were then evaluated for FITC fluorescence.

B. RESULTS

1. Prevention of EAE by TCR $V_\beta 8$(39-59) peptide immunization.

To evaluate prophylactic effects of anti-TCR $V_\beta 8$(39-59) immunity on EAE induced by various encephalitogenic epitopes, Lewis rats were first immunized with the TCR $V_\beta 8$(39-59) peptide, and 44 days later, EAE was induced with either Gp-S49S or Gp-S87-99. As is shown in Table 7, immunization with the TCR $V_\beta 8$(39-59) peptide reduced markedly the severity of Gp-S49S-induced EAE, and prevented Completely 587-99-induced EAE. Although histological scores in both protected groups were reduced, inflammation in the CNS was generally less affected by TCR $V_\beta 8$(39-59) peptide immunization than were clinical parameters.

2. Suppression of EAE with the TCR $V_\beta 8$(39-59) peptide.

To evaluate suppression of EAE, the TCR $V_\beta 8$(39-59) peptide was given simultaneously with the encephalitogenic dose of GPBP or Gp-S49S. As is shown in Table 7, the TCR $V_\beta 8$(39-59) peptide prevented GPBP-induced EAE in most rats, and markedly reduced the clinical severity in the remaining animals. A similar result was obtained in with Gp-S49S-induced EAE. In contrast, the TCR $V_\beta 14$(39-59) control peptide had no suppressive effects on EAE (Table 7). Again, histological signs of EAE were relatively less affected by TCR $V_\beta 8$(39-59) immunization than clinical signs.

3. Antibody responses against the TCR $V_\beta 8$(39-59) peptide.

TCR $V_\beta 8$-specific antibodies raised against intact T cell clones (Owhashi, M., et al., J. Exp. Med. 168:2153 (1988); Gascoigne, N.R.J., et al., Proc. Natl. Acad. Sci., USA 84:2936 (1987); Kappler, J.W., et al., Nature 332:35 (1988); Kappler, J.W., et al., Cell 49:263 (1987); MacDonald, H.R., et al., Nature 332:40 (1988)) have proven efficacious in

Table 7

| Prevention and Suppression of EAE by Immunization with the TCR $V_\beta$ 8(39-59) Peptide | | | | |
|---|---|---|---|---|
| Antigen | Treatment Day | N | Clinical Score | Histological Score |
| GP-BP | 0 (Imm) | 8 | 3.1 ± 0.6 | ND |
| TCR $V_\beta 8$(39-59) GPBP | 0 (Imm) 0 (Chall) | 8 | 0.7 ± 0.3 | ND |
| Gp-S49S | 0 (Imm) | 4 | 3.0 (2.5 - 4.0) | Br: 3.0 ± 0.8 Sc: 3.3 ± 0.5 |
| TCR $V_\beta 8$(39-59) Gp-S49S | 0 (Imn) 0 (Chall) | 4 | 0.5 (0.5 - 0.5) | |
| TCR $V_\beta 8$(39-59) Gp-S49S | 0 (Imm) 44(Chall) | 8 | 0.8 (0.0 - 1.0) | Br: 1.8 ± 1.7 Sc: 2.8 ± 1.0 |
| Gp-(S87-99) | 0 (Imm) | 6 | 2.3 (1.5 - 3.0) | Br: 2.2 ± 1.1 Sc: 1.0 ± 0.0 |
| TCR $V_\beta 8$(39-59) Gp-(S87-99) | 0 (Imm) 44(Chall) | 4 | 0.0 | Br: 0.5 ± 0.6 Sc: 0.8 ± 0.5 |
| TCR-$V_\beta 14$(39-59) Gp-S49S | 0 (Imn) 0 (Chall) | 4 | 2.9 (1.5 - 4.0) | |

Table 7   (continued)

| Prevention and Suppression of EAE by Immunization with the TCR $V_\beta$ 8(39-59) Peptide | | | | |
|---|---|---|---|---|
| Antigen | Treatment Day | N | Clinical Score | Histological Score |
| Groups of Lewis rats were immunized with the listed antigens on the indicated treatment days ("Imm"). Each rat received 2 SC injections in the base of the tail of 0.1 ml containing 100 µg free peptide emulsified in CFA. Immunized rats were challenged on indicated days ("Chall") with either the encephalitogenic GPBP (50 µg), Gp-S49S (100 µg) or Gp-(S87-99) (100 µg), injected in the foot pad as an emulsion (0.1 ml) in CFA. Rats were inspected daily for clinical signs of disease. Tissues were taken for histology 23 to 26 days after challenge. The clinical scores represent the mean of all rats per group and are scored as described in Table 1. Ranges of clinical scores appear in brackets. The histological scores of brain (Br) and spinal cord (Sc) of individual rats are based on the number of lesions: 1=1-2 lesions; 2=3-5; 3=6-8; 4=9 or more lesions in a hematoxylin-stained sagittal section of the brain or the entire length of the spinal cord. | | | | |

the prevention and treatment of EAE in both Lewis rats (Owhashi, M., et al., J. Exp. Med. 168:2153 (1988)) and PUJ mice (Acha-Orbea, H., et al., Cell 54:263 (1988); Urban, J., et al., Cell 54:577 (1988)). It thus was very important to determine whether antibodies could be raised against the synthetic TCR $V_\beta$8(39-59) peptide, and if so, whether they had clinical utility.

Such antibodies could indeed be raised and were found to be clinically effective. Antibodies against TCR $V_\beta$8 (39-59) were detected in the sera of Lewis rats as early as 7 days after a single injection of 100 µg of the free peptide in CFA (Table 8). Although a high degree of variability in the antibody response was observed, the antibody titers increased gradually over time. None of the TCR peptide-immunized rats developed any signs of EAE, and all remained healthy throughout the 41 day observation period.

Rabbits immunized with either the free or KLH-conjugated TCR $V_\beta$8(39-59) peptide produced much higher titers of antibodies than did rats (Table 8). Antibody titers remained high for over 6 months, showing detectable reactivity at dilutions of up to 1/320,000.

Table 8

| ANTIBODY RESPONSE AGAINST TCR PEPTIDE $V_\beta$8(39-59) IN LEWIS RATS AND RABBITS | | | | |
|---|---|---|---|---|
| Day After Challenge | Serum Dilution | N | Reactivity Against TCR $V_\beta$8(39-59) | Clinical Signs of EAE |
| Lewis Rats | | | | |
| 7 | 1:40 | 4 | 90 (51 - 161) | None |
| 14 | 1:40 | 4 | 214 (83 - 471) | None |
| 21 | 1:40 | 4 | 115 (52 - 288) | None |
| 33 | 1:40 | 4 | 311 (44 - 712) | None |
| 41 | 1:40 | 12 | 466 (103 - 1132) | None |
| Rabbits | | | | |
| TCR $V_\beta$8(39-59)-KLH conjugate: | | | | |
| 75 | 1:40, 000 | | 482 | None |
| 160 | 1:80,000 | | 399 | |
| TCR $V_\beta$8(39-59) free peptide: | | | | |
| 75 | 1:40,000 | | 133 | None |
| 160 | 1:80,000 | | 217 | |

Table 8   (continued)

| ANTIBODY RESPONSE AGAINST TCR PEPTIDE $V_\beta 8$(39-59) IN LEWIS RATS AND RABBITS | | | | |
|---|---|---|---|---|
| Day After Challenge | Serum Dilution | N | Reactivity Against TCR $V_\beta 8$(39-59) | Clinical Signs of EAE |
| TCR $V_\beta 8$(39-59) free peptide: | | | | |
| Male Lewis rats (225-250g) were challenged SC with 100 µg TCR Vβ8(39-59) + 100 µg M. butyricum in CFA. Each rat was bled from the tail vein on indicated days after challenge. Rabbits (6 lbs) received a course of imnunization that is detailed in the Materials and Methods. Antibody reactivity of individual sera against TCR $V_\beta 8$(39-59) was documented by direct ELISA and the average reactivity of individual sera per group is presented as Absorbance Units (x10$^3$). In brackets are shown the ranges of antibody reactivity of all antisera per group. All sera were heat inactivated and diluted before assay against 25 ng of plated peptide. The term "None" designates the complete absence of clinical signs of EAE. | | | | |

4. Antibody responses against the encephalitogenic peptide S49S.

Immunization of Lewis rats with GPBP or GP-S49S (residues 69-84) peptide induces antibodies that recognize several different epitopes, one of which is comprised of residues 82-84 (Asp-Glu-Asn) and is evidenced by antibody binding to Gp-S53 (residues 75-84) (Day, E.D., et al., J. Neurosci. Res. 18:214 (1987); Hashim, G.A., et al., J. Neurosci. Res. 17:375 (1987)). These antibody responses depend on T cell help, which can be provided by encephalitogenic T cells specific for the GP-S49S peptide. Although immunization with the TCR $V_\beta 8$(39-59) peptide prevents and suppresses EAE mediated by Gp-S49S-specific T cells of the helper phenotype, it is important to determine the effect of such immunization on anti-S49S antibody formation.

The antibody response to Gp-S49S was detected as early as 7 days after immunization with the Gp-S49S in CFA (Table 9). Periodic bleeding of the immunized rats showed marked increases in antibody titers to both Gp-S49S and Gp-S53 during the next 48 days, the anti-Gp-S53 response appearing only after the development and eventual recovery from EAE.

After preimmunization and protection against EAE with TCR $V_\beta 8$(39-59), the 26 day antibody responses to Gp-S49S and Gp-S53 were elevated two to four fold relative to the that in rats not treated with the TCR peptide (Table 9). Similarly, anti-587-99 responses were increased >4 fold in rats preimmunized and protected with the TCR $V_\beta$ 8 (39-59) peptide (Table 9). Thus, an immune response directed against $V_\beta$ 8$^+$ T cells actually enhanced antibody responses to several distinct B cell epitopes of GPBP.

Table 9

| ANTIBODY RESPONSE AGAINST ENCEPHALITOGENIC PEPTIDES IN RATS IMMUNIZED WITH TCR Vβ8 (39-59) PEPTIDE DURING THE COURSE OF EAE | | | | | | | |
|---|---|---|---|---|---|---|---|
| | | | | -----Antisera Reactivity Against------ | | | |
| Day after Challenge | Dilution | N | TCR $V_\beta 8$ (39-59) | Gp-S49S | Gp-S53 | Gp-(S87-99) | Clinical Signs |
| No TCR Immunization Gp-S49S Challenge | | | | | | | |
| 7 | 1:40 | 6 | | 92 | 0 | | None |
| 14 | 1:40 | 5 | | 353 | 15 | | 6/6 HLP |
| | | | | | | | 1 died |
| 21 | 1:40 | 4 | | 1360 | 104 | | 4 recov. |
| | | | | | | | 1 died |
| 29 | 1:40 | 4 | 12 | 935 | 222 | 19 | 4 recov. |
| 48 | 1:320 | 4 | 10 | 1333 | 335 | 5 | 4 recov. |
| | | | | (10664) | (2680) | | |

Table 9   (continued)

| ANTIBODY RESPONSE AGAINST ENCEPHALITOGENIC PEPTIDES IN RATS IMMUNIZED WITH TCR Vβ8 (39-59) PEPTIDE DURING THE COURSE OF EAE | | | | | | | |
|---|---|---|---|---|---|---|---|
| | | | -----Antisera Reactivity Against------ | | | | |
| Day after Challenge | Dilution | N | TCR V$_\beta$8 (39-59) | Gp-S49S | Gp-S53 | Gp-(S87-99) | Clinical Signs |
| TCR V$_\beta$8 (39-59) on d. 0 | | | | | | | |
| Gp-S49S on d. 48 | 1:80 | | 629 | 875 | 455 | 11 | None 0/4 rats |
| Bleed on d. 74 | | | (1258) | (1750) | (910) | | |
| No TCR Immunization Gp-S(87-99) on d. 21 | 1:40 | | 10 | 8 | 14 | 550 | 4/4 2/4 HLP |
| TCR V$_\beta$8-39-59 on day 0 | | | | | | | None |
| Gp-S(87-99) on d. 44 | | | | | | | |
| Bleed on d. 65 | 1:320 | | 621 (4968) | 38 | 46 | 348 (784) | 0/4 rats |
| Rats were either immunized with 100 µg TCR Vβ8(39-59) peptide or injected with saline and were challenged on indicated days with either Gp-S49S or Gp(S87-99), SC in emulsion containing 100 µg M. butyricum in CFA. Rats were bled on the indicated days. Groups immunized with TCR Vβ8(39-59) and challenged with either Gp-S49S or Gp-(S87-99) were terminated on days 26 and 21 after challenge, respectively. Antibody reactivity (binding to 25 ng/ well of peptide ) of individual sera was measured in direct ELISA. Results are presented as average Absorbance (x 10$^3$) at 450-650 nm (automatically corrected for background). Theoretical Absorbances (shown in parentheses) were normalized to a 1:40 dilution. Hind leg paralysis (HLP) was accompanied by incontinence. Because of the severity of the disease, 2 of the Gp-S49S-challenged rats died on days 14 and 18, respectively. Variation within groups of 2-3 samples was less than 5%. | | | | | | | |

5. Specificity of anti-peptide antibodies.

To evaluate the specificity of several antisera, binding to a panel of synthetic TCR and GPBP peptides was assessed. The results (Table 10), show that anti-Gp-S49 antiserum which recognized GP-S49S and its C-terminal fragment, Gp-S53, did not react with the N-terminal fragment of Gp-S49 (i.e., Gp-S67), with any other regions of GPBP, or with any of the TCR V region peptides. Similarly, rat or rabbit antisera to the TCR V$_\beta$8(39-59) peptide, recognized only the immunogen, and not other TCR sequences (including the 3 overlapping residues - AspMetGly - present on TCR V$_\beta$8(25-41), or GPBP peptides. Antisera from rats immunized simultaneously with TCR V$_\beta$ 8(39-59) peptide plus Gp-S49S or Gp-S87-99 demonstrated the same specificity for each of the immunogens as antisera from singly-immunized rats (Table 5). The specificity of antibody reactivity to TCR V$_\beta$8(39-59) and to Gp-S49S was confirmed by peptide-specific, dose dependent inhibition of binding in ELISA (Figure 1).

6. Antibodies to TCR V$_\beta$8(39-59) recoqnize V$_\beta$8$^+$ T cells.

To interpret potential regulatory effects of antibodies to TCR V$_\beta$8(39-59), it was crucial to establish whether or not the peptide-specific antibodies interacted directly with V$_\beta$8$^+$ T cells. To evaluate such reactivity, V$_\beta$8$^+$ encephalitogenic T cells or normal thymocytes which are predominantly V$_\beta$8$^-$ were incubated with rat or rabbit anti-TCR V$_\beta$ 8 (39-59) IgG antibody, followed by mouse anti-rat or anti-rabbit facilitating antibody, and fluorescein-labeled goat anti-mouse IgG antibody. As is shown in Figure 2, the rabbit IgG raised to the KLH-conjugated TCR V$_\beta$8(39-59) peptide caused an increased fluorescence intensity in the entire V$_\beta$8$^+$ encephalitogenic T cell population (>90% positive versus a control antiserum), as opposed to approximately 5% of the normal thymocyte population. Rat IgG and rabbit IgG raised against unconjugated TCR peptide also stained selectively the $V_\beta 8^+$ T cells, although with less intensity. These results indicate that antibody to the TCR $V_\beta 8(39\text{-}59)$ peptide can bind specifically to $V_\beta 8^+$ T cells. None of the antisera were cytotoxic

Table 10

| REACTIVITY OF ANTIBODIES AGAINST TCR $V_\beta$ 8(39-59) AND GPBP PEPTIDES WITH A PANEL OF SYNTHETIC MYELIN BASIC PROTEIN AND TCR β CHAIN PEPTIDES | | | | | | | | | |
|---|---|---|---|---|---|---|---|---|---|
| $V_\beta 8$ 39-59 | $V_\beta 8$ 25-41 | $V_\beta 14$ 39-59 | $V_\beta 14$ 24-41 | Gp S49S | Gp S53 | Gp S67 | Gp S55-74 | Gp S87-99 | Gp N-Ac 1-16 |
| Rat anti-Gp-S49S: (1:160 dilution) | | | | | | | | | |
| 14 | 20 | 21 | 18 | 2211 | 1535 | 16 | 17 | 7 | 14 |
| Rat anti-TCR $V_\beta 8(39\text{-}59)$: (1:160 dilution) | | | | | | | | | |
| 222 | 11 | 21 | 9 | 12 | 8 | 12 | 14 | 5 | 10 |
| Rat anti-TCR $V_\beta 8(39\text{-}59)$ and Gp-S49S: (1:640 dilution) | | | | | | | | | |
| 421 (1684) | 16 | 20 | 11 | 2190 (8760) | 1639 (6556) | 8 | 14 | 5 | 12 |
| Rat anti-TCR $V_\beta 8(39\text{-}59)$ and Gp-(S87-99): (1:320 dilun) | | | | | | | | | |
| 621 (1242) | 13 | - | 18 | 38 | 6 | 8 | 14 | 348 (696) | 12 |
| Rabbit anti-TCR $V_\beta 8(39\text{-}59)$-KLH: (1:40,000 dilution) | | | | | | | | | |
| 408 (102000) | 0 | 25 | 3 | 2 | 4 | 0 | 0 | 5 | 1 |
| Lewis rats were immunized with 100 μg of Gp-S49S, TcR $V_\beta 8(39\text{-}59)$ or both, SC in Freund's adjuvant (100 μg M. butyricum/rat). Antisera were prepared between days 54 and 62 after immunization and pools of high titer antisera were made from 2 to 4 immunized rats. Rabbit antisera was isolated on day 43 after immunization with the TCR peptide conjugated to KLH. All antisera were heat-inactivated for 30 min at 57°C. Antibody reactivity, at the indicated dilutions, against the various peptide antigens was measured by direct ELISA using rabbit anti-rat or goat anti-rabbit IgG (L+H)-peroxidase labeled. The values shown are the Absorbance measurements at 450-650 nm (x 10³). All figures were automatically corrected for background reactivity in the absence of plated peptides. Numbers in brackets represent theoretical Absorbance values calculated for a 1:160 dilution. | | | | | | | | | |

for $V_\beta 8^+$ T cells in the presence of complement, as measured by both chromium release or dye exclusion, suggesting that antibody binding altered T cell function without killing the cells.

7. <u>Suppression of EAE with anti-TCR $V_\beta 8(39\text{-}59)$ antibody.</u>

Lewis rats immunized with TCR $V_\beta 8(39\text{-}59)$ were not only protected against EAE, but developed circulating antibodies specific for the immunizing peptide. To evaluate the role of these antibodies in down-regulating EAE, Lewis rats were challenged with Gp-S49S in CFA and were then treated with TCR $V_\beta 8(39\text{-}59)$-specific IgG (rat or rabbit). Rats receiving Lewis rat IgG every other day for 12 days developed mild clinical signs of EAE with reduced histology in the brain, but more extensive lesions in the spinal cord compared to controls (Table 11). Rats receiving rabbit IgG developed minimal clinical signs with little change in histological scores (Table 6). Thus, passive administration of anti-TCR $V_\beta 8 (39\text{-}59)$ antibodies over a 12 day period suppressed clinical but not histological signs of EAE.

C. DISCUSSION

The results presented herein constitute the first demonstration of the ability of a synthetic TCR V-region peptide to induce specific antibodies that can suppress the induction of EAE. These TCR $V_\beta8(39-59)$ peptide-specific antibodies are able to bind T cells bearing the entire, intact TCR, and thereby alter the function of these cells without lysing them. Coupled with results presented in Example I, these results indicate that both antibody and cell-mediated immune responses can provide independent immunoregulatory actions on encephalitogenic T lymphocytes that utilize common V region genes in response to epitopes of MBP. Both regulatory mechanisms have potent preventative and suppressive effects on the induction of clinical signs of autoimmune disease.

Table 11

| SUPPRESSION OF EAE IN LEWIS RATS BY PASSIVELY TRANSFERRED ANTI-TCR $V_\beta$ 8(39-59) ANTIBODIES | | | | | |
|---|---|---|---|---|---|
| Treatment | Antibody Against | | | -----EAE Activity---- | |
| | TCR $V_\beta$8 (39-59) | Gp-S49S | N | Clinical Scores | Histological Scores |
| No anti-TCR Ab | | | | | |
| Bleed: day 21 | 15 | 1360 | 6 | 3.1 (1.5-4.0) | Br: 3.3 ± 0.5 Sc: 2.8 ± 1.0 |
| Rat anti-TCR | | | | | |
| Bleed: day 0 Bleed: day 12 Bleed: day 24 | 5 149 162 | 12 165 (3014) | 4 | 1.5 (1.5-2.0) | Br: 1.0 ± 1.0 Sc: 4.0 ± 0.0 |
| Rabbit anti-TCR | | | | | |
| Bleed: day 0 Bleed: day 12 Bleed: day 24 | 10 500 130 | 16 34 322 | 6 | 0.7 (0.5-1.0) | Br: 2.2 ± 1.0 Sc: 2.7 ± 1.5 |
| EAE was induced in Lewis rats by injection of 100 µl of emulsion containing 100 µg Gp-S49S and 100 µg M. butyricum in the footpad. Beginning on the day of induction and every other day for 12 days, experimental groups received either 7 mg Lewis rat IgG (which contained 17 Absorbance Units/mg IgG) or 10 mg rabbit IgG (which contained 6570 Absorbance Units/mg IgG) from animals that had been immunized with TcR $V_\beta$8(39-59). The IgG was dissolved in sterile saline and injected intraperitoneally. Treatment with non-immune rat or rabbit IgG did not influence the course of EAE. All animals were inspected daily for clinical signs of EAE, bled from the tail vein on indicated days. Sera were tested for antibody to TCR or GPBP peptide in direct ELISA (results are Absorbance x $10^3$). All animals were sacrificed on day 24 after EAE induction and brains (Br) and spinal cords taken for histological evaluation as described in the legend to Table 7. | | | | | |

The TCR $V_\beta$8(39-59) peptide, which was predicted to be a good T cell immunogen based on the algorithms of Margalit et al. and Rothbard et al. (supra), proved to be a potent B cell immunogen, especially in rabbits. THe antibodies were highly specific for the immunizing peptide (by both direct reaction and inhibition assays), stained only $V_\beta8^+$ T cells, and suppressed EAE mediated by $V_\beta$ 8+ T cells.

In conclusion, the synthetic peptide TCR $V_\beta$8(39-59) induced both T cell immunity and antibody production in Lewis rats. Both T cells and antibodies, alone or together, are capable of regulating the immune response to an encephalitogenic challenge. The ability of this TCR peptide to activate regulatory T cells and protective antibodies demonstrates the utility of this approach for the control of human autoimmune diseases.

**EXAMPLE III**

CLINICAL EAE IN RATS TREATED WITH TCR $V_\beta$8(39-59) PEPTIDES BEFORE OR AFTER ONSET OF DISEASE

The ability of the TCR $V_\beta$8(39-59) given either SC (in adjuvant) or ID (in saline) to disrupt the disease process when given at various times after induction of EAE with GPBP was tested (Table 12). In Experiment 1, the TCR peptide was administered either on day 10 (line 2) or when the first rat in a group showed clinical signs of EAE (lines 3 and 4) at the time of onset. In both cases, with both routes of injection of the peptide, there was a significant reduction in the duration of the disease, though not in the severity or time of onset. The efficacy of the peptide given in saline via the ID route is of particular importance to human therapy, as it is preferred to SC injection in adjuvant.

Table 12

| CLINICAL EAE IN RATS TREATED WITH TCR $V_\beta 8$(39-59) PEPTIDES BEFORE OR AFTER ONSET OF DISEASE | | | | | |
|---|---|---|---|---|---|
| Treatment | Day | Day of Onset | Disease Severity | Disease Duration | Disease Incidence |
| Experiment 1 | | | | | |
| Control | -- | 13.0±0 | 3.0 ± 0 | 6.8 ± 0.8 | 5/5 |
| TCR(100μg)(SC in CFA) | 10 | 14.0±0.4 | 2.8 ± 0.4 | 3.2 ± 0.4 | 5/5 |
| TCR (100 μg) (SC in CFA) | 13 | 13.0±0 | 3.0 ± 0 | 4.0 ± 0 | 5/5 |
| TCR (50 μg) (ID) | 13 | 13.4±0.5 | 2.6 ± 0.4 | 3.2 ± 0.5 | 5/5 |
| Experiment 2 | | | | | |
| Control | -- | 13.0±0.5 | 3.0 ± 0 | 5.8 ± 0.4 | 6/6 |
| TCR (200 μg) (ID) | 13 | 14.0±1.4 | 2.3 ± 0.8 | 3.0 ± 1.2 | 6/6 |
| TCR (50 μg) (ID) | 14 | 16.0±1.0 | 1.7 ± 1.5 | 2.3 ± 2.0 | 4/6 |
| TCR (50 μg) (ID) | 7 | 15.0±0.8 | 1.7 ± 1.4 | 2.0 ± 2.0 | 4/6 |
| TCR (50 μg) | 0 | 15.6±2.6 | 1.8 ± 1.0 | 2.3 ± 1.6 | 5/6 |
| Control TCR ($V_\beta$14 (39-59) | 13 | 13.7±0.5 | 2.5 ± 0.8 | 3.5 ± 0.5 | 6/6 |
| TCR refers to the peptide TCR $V_\beta 8$(39-59). | | | | | |

In Experiment 2, the time of ID administration of the TCR peptide was varied, as was the dose. As shown in Table 12, 50 μg of the peptide administered on either days 0 (day of EAE induction), 7, or 14 resulted in a significant reduction in the duration of disease. A delay in onset of the disease was also observed. Furthermore, the percentage of animals showing signs of the disease decreased. A larger dose of the peptide (200 μg) appeared less efficacious than the lower dose, possibly due to a short-term overload of the peptide which could have comprised the immune response generated against the TCR. Treatment with a similarly sized peptide corresponding to an irrelevant TCR had no effect on onset, severity or incidence of EAE, but may have reduced the duration somewhat (last line of Table 12).

**EXAMPLE IV**

T CELL RESPONSES OF LN CELLS AND TCR PEPTIDE-SELECTED T CELL LINES FROM RATS RECEIVING TCR PEPTIDE THERAPY

The proliferative responses and specificity of T cells in the draining LN (popliteal) of rats treated with TCR $V_\beta 8$ (39-59) peptide for EAE on day 13 after disease induction were examined (Table 13). On day 0, Lewis rats received an EAE-inducing regimen of GP-BP + CFA SC into their hind footpads. On day 13, they were divided into three groups and received either saline (column 1), 100 μg TCR $V_\beta 8$(39-59) peptide (+ CFA) SC in the hind footpads (column 2) or 50 μg of TCR $V_\beta 8$(39-59) in saline ID in the ear pinna. On day 20, about 7 days after onset of EAE, popliteal LNs were removed and T cell proliferative activity in response to the indicated antigens or mitogens were tested directly

LN cells from control rats responded best to Con A, PPD, GPBP and GPBP (72-89), and GPBP (45-89) (which includes the 72-89 sequence and another immunogenic but non-encephalitogenic peptide). In contrast, LN cells from rats given the TCR peptide SC in CFA, did not show significant proliferative responses to GPBP or to any of the BP fragments. LN cells from rats treated ID with the TCR peptide

Table 13

| PROLIFERATIVE RESPONSES OF LN CELLS FROM LEWIS RATS AFTER EAE INDUCTION AND TREATMENT WITH TCR PEPTIDES | | | |
|---|---|---|---|
| | | ---- T Cell Proliferation (cpm x $10^{-3}$) ---- | |
| In Vitro Stimulus | Control | TCR $V_\beta 8$(39-59) | |
| | | 100 µg SC (in CFA) | 50 µg ID |
| Medium | 28 | 47 | 31 |
| Con A | 85 | 78 | 146 |
| PPD | 91 | 72 | 104 |
| TCR $V_\beta 8$(39-59) | 31 | 39 | 30 |
| GPBP | 49 | 50 | 44 |
| GPBP (72-89) | 44 | 38 | 37 |
| GPBP (87-99) | 37 | 36 | 34 |
| GPBP (45-89) | 48 | 50 | 49 |
| GPBP (1-38) | 30 | 47 | 34 |
| GPBP (90-170) | 36 | 54 | 44 |
| TCR $V_\beta 8$(39-59) + GPBP | 50 | 59 | 48 |
| Results are shown as net $^3$H-thymidine incorporation of 5 x $10^5$ cells stimulated in microwells. | | | |

responded similarly to the control cells, with the exception of a reduced response to GPBP(72-89). In addition, the latter group showed an increased response to GPBP(90-170), which is not known to be encephalitogenic. This indicates that epitope switching had occurred. An aliquot of each of the above 3 groups of LN cells was stimulated in culture with either GPBP or with the TCR peptide for 3 days, and the cells expanded in IL-2 for 5 more days. The proliferative responses to the various antigens and mitogens of these selected T cells were examined, as above. The results are shown in Table 14.

Control T cells (Table 14, column 1) responded well to GPBP, GPBP(72-89), P1 and rat BP (indicating homologous recognition in the CNS). THe last line of the Table indicates that when T cells of this group were injected into naive rats, they were encephalitogenic.

T cells from the group of animals treated with the TCR peptide in CFA, SC, and selected in culture with GPBP (column 2) responded poorly to GPBP, GPBP(72-89) and rat BP. The response to GPBP P1 was apparently due to the second (non-encephalitogenic) epitope, since the response to the GPBP(72-89) epitope was weak. The potent response to the TCR peptide indicated that a 7 day exposure (with no further selection with this peptide) was sufficient for anti-TCR peptide immunity. Of great significance is the observation that these cells were unable to transfer EAE, indicating that the encephalitogenic clones could not be selected during culture in the presence of the GPBP antigen.

The cells from the above TCR-immunized animals, when selected in vitro with the TCR peptide rather than with GPBP (column 3), appeared to respond only to the TCR peptide (and, of course, the T cell mitogen, Con A).

Finally, cells from rats treated with the TCR peptide ID and selected in culture with GPBP (column 4) behaved essentially like the control cells. That is, they recognized the GPBP(72-89) encephalitogenic epitope and were able to transfer EAE. This indicates that encephalitogenic T cell precursors were still present in the rats treated in this manner and could be selected in culture. This suggests the possibility that the reduced duration of EAE seen in rats treated with TCR peptide ID (see Example III and Table 12) does not involve regulation of the draining LN cells. However, it is important to note that the LNs draining the site of ID injection (i.e., the cervical LN when ID injection is in the ear pinna) may show different regulatory properties.

Table 14

| PROLIFERATIVE RESPONSES OF PEPTIDE-SELECTED T CELLS FROM LEWIS RATS AFTER EAE INDUCTION AND IN VIVO TREATMENT WITH TCR PEPTIDES | | | | |
|---|---|---|---|---|
| Day 13 Inj: Control | | TCR $V_\beta$8(39-59) + CFA | | TCR $V_\beta$ 8(39-59) ID |
| Selection with: GPBP | | GPBP | $V_\beta$8(39-59) | GPBP |
| In Vitro Stimulation: | | | | |
| Control | 15 | 17 | 8 | 10 |
| Con A | 76 | 52 | 68 | 95 |
| GPBP | 99 | 29 | 6 | 122 |
| TCR $V_\beta$8(39-59) | 24 | 66 | 85 | 10 |
| GPBP + TCR | 110 | 82 | 105 | 127 |
| GPBP (72-89) | 51 | 20 | 12 | 52 |
| GPBP (45-89) | 73 | 38 | 9 | 78 |
| GPBP (1-38) | 13 | 19 | 11 | 12 |
| GPBP (90-170) | 23 | 25 | 10 | 11 |
| GPBP(87-99) | 15 | 18 | 15 | 9 |
| Rat BP | 46 | 14 | 4 | 36 |
| Ability to Transfer EAE | YES | NO | | YES |

## EXAMPLE V

TREATMENT OF AUTOIMMUNE DISEASE WITH A TOXIN-CONJUGATED ANTIBODY TO A TCR PEPTIDE

In the present example, a mAb is produced by immunizing mice with the TCR $V_\beta$8(39-59) peptide and carrying out the methods for making a hybridoma as described above. The mAb is then conjugated to the ricin A chain, to yield an immunotoxin. The toxin conjugated antibody is injected into rats along at the same time as an encephalitogenic dose of GPBP (prophylaxis), and into other rats after onset of EAE (therapy).

Prophylactic treatment with the ricin A chain-conjugated anti-TCR peptide antibody (1-4 injections at doses of 0.05 to 0.2 mg/kg) results in a significantly reduced incidence and severity of EAE. Therapeutic treatment with similar doses of the conjugate results in a significant shortening of the duration and a lessening in the severity of the disease.

## EXAMPLE VI

TREATMENT OF ARTHRITIS WITH TCR PEPTIDES

The present example describes how human rheumatoid arthritis is treated by the composition and methods of the invention. It is modeled by two animal models: (1 ) Arthritis induced in susceptible mice by injection of Type II collagen (Stuart, J.M., et al., Ann. Rev. Immunol. 2:199-218 (1984) and (2) arthritis induced in susceptible rats by injection of Mycobacterial heat shock protein (HSP) (Van Eden, W., et al., Nature 331:171-173 (1988)). Arthritogenic T cells responsive to collagen or HSP and capable of transferring the disease are selected in vitro in the presence of collagen or HSP using methods described above. The TCR associated with disease-mediating T cells is identified, and the presumptive amino acid sequence is determined by nucleic acid sequencing, as above. Using the algorithms of Margalit et al. and Rothbard et al. (supra), an immunogenic portion of the TCR that comprises at least part of the second complementarity determining region is synthesized and used to immunize mice (for collagen arthritis) or rats (for adjuvant arthritis).

Animals treated with the TCR peptide in conjunction with disease induction are significantly protected from development of arthritis, as measured by joint swelling and by T cell reactivity to the arthritogen. Animals treated with the TCR peptide after onset of the disease show a significant shortening of the duration and a lessening in the severity of the symptoms of arthritis.

Passive immunization with antibodies induced against the TCR peptide associated with arthritis also show similar prophylactic and therapeutic effects on arthritis induction. Successful treatment is achieved by polyclonal antibodies, mAbs, chimeric antibodies, and immunotoxin-conjugated antibodies.

## EXAMPLE VII

TREATMENT OF THYROIDITIS WITH TCR PEPTIDES

Human thyroiditis, including Hashimoto's thyroiditis and Graves' disease, is treated by the composition and methods of the invention as described in the present example. Although the precise nature of the target autoantigen is uncertain, immune reactivity to thyroglobulin and to thyrotrophin receptor, respectively, is associated with these diseases. Thyroiditis is modeled in mice by administration of thyroglobulin (Maron, R., et al., J. Exp. Med. 152:1115-1120 (1980)). T cells responsive to thyroglobulin and to thyroid follicular cell antigens, and capable of transferring the disease, are selected in vitro in the presence of either thyroglobulin, thyroid cells, or thyroid cell membrane preparations using methods described above. The TCR associated with disease-mediating T cells is identified, and the presumptive amino acid sequence is determined by nucleic acid sequencing, as above. Using the algorithms of Margalit et al. and Rothbard et al. (supra), an immunogenic portion of the TCR that comprises at least part of the second complementarity determining region is synthesized and used to immunize mice.

Animals treated with the TCR peptide in conjunction with disease induction are significantly protected from development of thyroiditis and of T cell reactivity to the thyroid antigens. Animals treated with the TCR peptide after onset of the disease show a significant shortening of the duration and a lessening in the severity of the symptoms of thyroiditis.

Passive immunization with antibodies induced against the TCR peptide associated with thyroiditis also shows similar prophylactic and therapeutic effects on disease induction. Successful treatment is achieved by polyclonal antibodies, mAbs, chimeric antibodies, and immunotoxin-conjugated antibodies.

## EXAMPLE VIII

TREATMENT OF DIABETES WITH TCR PEPTIDES

Insulin-dependent diabetes mellitus (IDDM), or type I diabetes, is an autoimmune disease characterized by immune reactivity directed to pancreatic islet (or beta) cells, resulting in the cells' destruction and shutdown of insulin production. The target antigens for this immune attack have not been characterized with certainty. The present example describes how IDDM is treated by the compositions and methods of this invention.

The disease is modeled in animals in which it occurs naturally, or can be induced in certain strains of mice (Kanasawa et al., Diabetologia 27:113 (1984). Other mouse strains can be caused to exhibit this disease by transferring lymphocytes from the susceptible strains.

T cells responsive to pancreatic islet cell antigens and capable of transferring the disease are selected in vitro in the presence of either islet cells, or islet cell membrane preparations using methods described above. The TCR associated with disease-mediating T cells is identified, and the presumptive amino acid sequence is determined by nucleic acid sequencing, as above. Using the algorithms of Margalit et al. and Rothbard et al. (supra), an immunogenic portion of the TCR that comprises at least part of the second complementarity determining region is synthesized and used to immunize mice.

Animals treated with the TCR peptide in conjunction with disease induction are significantly protected from development of diabetes and of T cell reactivity to the islet cell antigens. Animals treated with the TCR peptide after onset of the disease show a significant shortening of the duration and a lessening in the severity of the symptoms of diabetes.

Passive immunization with antibodies induced against the TCR peptide associated with diabetes also show similar prophylactic and therapeutic effects on disease induction. Successful treatment is achieved by polyclonal antibodies, mAbs, chimeric antibodies, and immunotoxin-conjugated antibodies.

## EXAMPLE IX

TREATMENT OF EXPERIMENTAL AUTOIMMUNE ENCEPHALOMYELITIS WITH T CELL RECEPTOR V REGION PEPTIDE

Immunization of rats and mice with myelin basic protein (MBP) induces encephalitogenic T cells that express a limited repertoire of T cell receptor V region genes. Preceding examples demonstrate that a synthetic peptide from the Vβ8 sequence shared by most encephalitogenic rat T cell clones induces protection against EAE by stimulating specific regulatory T cells and antibodies. In the present example, the same TCR peptide, which corresponds to the 39-59 residues of the Vβ8 sequence and includes the second complementarity determining region, is demonstrated to be highly effective as therapy for EAE.

The TCR Vβ8-39-59 peptide, when given s.q. in complete Freund's adjuvant to rats with moderate EAE, halted disease progression and significantly shortened disease course. When the TCR peptide was given i.d. in the ear, the effects were delayed for 1 day, but again led to a faster resolution of clinical signs. MBP-selected T cell lines from the treated rats responded poorly to MBP, but retained reactivity to the TCR peptide and failed to transfer to normal recipients. The rapid clinical effect of the TCR peptide suggested triggering of a pre-existing regulatory network evoked in response to EAE development. In support of this concept, direct evidence is presented in the present example of T cell recognition of the TCR peptide in untreated rats undergoing EAE.

A. MATERIALS AND METHODS

Animals: Female Lewis rats, 6-8 weeks old, were obtained from Harlan Sprague Dawley (Indianapolis, IN). Rats were housed and maintained at the Portland VAMC Animal Resource Facility in accordance with Federal and Institutional guidelines.

Antigens: GP- or Rt-MBP was extracted and purified according to the method of Eylar (Eylar, E.H., et al., J. Biol. Chem. 246:5770 (1971 )). Enzymatic cleavage fragments of GP-MBP encompassing residues 1-37, 43-89, and 90-169, a synthetic peptide of GP-MBP corresponding to residues 72-89, and the synthetic peptides corresponding to the 39-59 residues of TCR $V_\beta 8$ and TCR $V_\beta 14$ were synthesized and purified as described previously (Vandenbark, A. A., et al., Nature 341:541 (1989); Eylar, E. H., et al., J. Biol. Chem. 246:5770 (1971)). These peptides were >90% pure by high pressure liquid chromatography analysis.

Clinical Protocols: EAE was induced in all experiments by a single subcutaneous (s.q. ) injection in one hind footpad of 50 μg GP-MBP in complete Freund's adjuvant containing 100 μg heat killed M. tuberculosis H37RA (DIFCO, Detroit, MI). In the prevention protocol, rats were injected s.q. on one hind footpad 40 days prior to EAE induction with 100 μg TCR $V_\beta 8$-39-59 or TCR $V_\beta 14$-39-59 in CFA containing 100 μg M. tuberculosis. In suppression protocols, the TCR peptides were injected at the same time (100 μg s.q. in CFA, or 50 μg i.d. in 0.1 ml saline in the ear), 7 days (i. d.), or 11 days (i.d.) after challenge with GP-MBP. In the treatment protocols, the TCR peptides were given either s.q. in CFA or i.d. in the ear on the first day that clinical signs of EAE were noted (usually day 12 after challenge with GP-MBP). Animals were scored daily for clinical signs of EAE, using a rating scale of 0-4, in which 0 = no signs; 1 = limp tail; 2 = hind leg weakness, ataxia; 3 = hind quarter paralysis; 4 = front and hind quarter paralysis, moribund condition. Treatment groups were compared with control groups for differences in maximum disease severity and duration of clinical signs by Student's unpaired t test. Delayed type hypersensitivity reactions were measured by the ear swelling assay (Offner, H., et al., J. Exper. Med. 170:355 (1989)) 24 and 48 hours after injection i.d. of 50 μg antigen. GP-MBP-specific T cell lines were selected from TCR peptide treated and untreated rats as described previously (Vandenbark, A. A., et al., J. Immunol. 135:223 (1985)). Ten million GP-MBP activated line cells were transferred i.p. into naive rats to test for encephalitogenic activity, scored as described above for actively induced EAE.

Lymphocyte Proliferation: Activation of T cells was measured by $^3$H-Tdy uptake. 500,000 lymph node cells or 20,000 line cells in the presence of 1 million irradiated thymic accessory cells were incubated with culture medium and antigens in microtiter wells for 18 hours prior to the addition of 0.5 μBq labeled thymidine. The cell cultures were harvested onto glass fiber filters and counted by liquid scintillation techniques. Mean CPM were calculated from triplicate cultures. Standard deviations (SD) from replicate cultures varied <10% from the mean value.

B. RESULTS

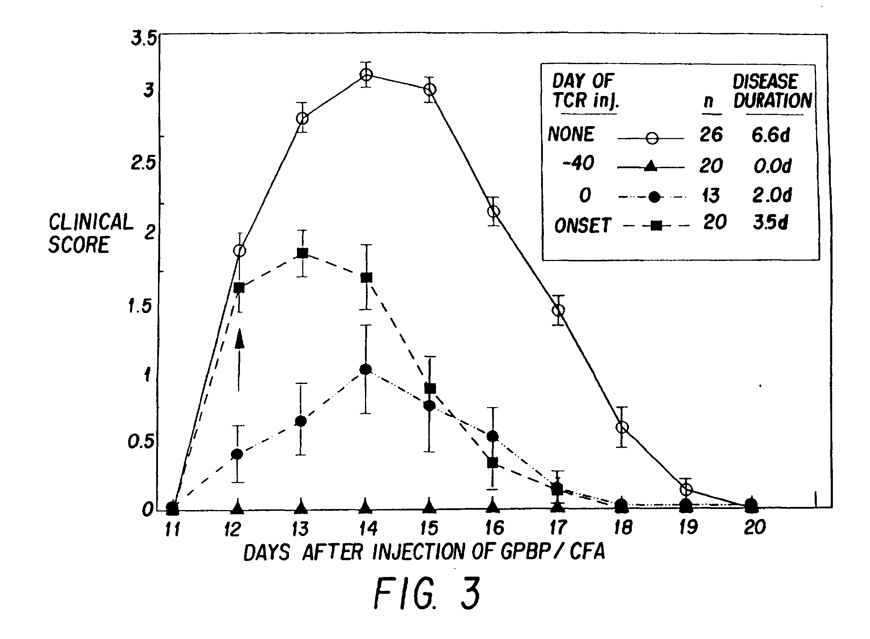

To evaluate the regulatory effect of the TCR peptides on EAE, the $V_\beta 8$-39-59 peptide, a control peptide $V_\beta 14$-39-59, or saline were injected prior to, simultaneously with, or after the injection of the encephalitogenic emulsion, GP-MBP/CFA. The average daily clinical scores of the most effective prevention, suppression, and treatment protocols are presented in Figures 3-5, and all groups tested are summarized in Table 15.

Clinical effects of TCR/CFA. Injection of the Vs8-39-59 peptide in CFA 40 days prior to challenge with GP-MBP induced complete protection against clinical EAE (Figure 3). Furthermore, simultaneous injection of the peptide in CFA with the encephalitogenic emulsion suppressed EAE, reducing the incidence (8/13 in the treated group versus 26/26 in controls), severity (score of 1.3 versus 3.4), and duration (2.0 versus 6.6 days) of clinical disease (Figure 3, Table 15). Rats injected 40 days prior to or at the same time as EAE induction with the $V_\beta 14$-39-59 peptide in CFA, or with CFA alone, developed EAE that was indistinguishable from the controls (Table 15).

TABLE 15

| Prevention, Suppression and Treatment of EAE with TCR Vβ8-39-59 Peptide. | | | | | | |
|---|---|---|---|---|---|---|
| | | | | | Clinical EAE | |
| Expt. Group | | Expts | EAE/TOTAL | Onset | Max Clin. Signs | Duration |
| GP-BP/CFA only | | 5 | 26/26 | 12 | $3.4 \pm 0.2$ | $6.6 \pm 0.9$ |
| TCR/CFA | Day -40 | 4 | 0/20 | -- | $0 \pm 0$ | $0 \pm 0$ |
| | Day 0 | 3 | 8/13 | 13 | $1.3 \pm 1.1**$ | $2.0 \pm 1.8**$ |
| | Day 10 | 1 | 5/5 | 13 | $2.8 \pm 0.4$ | $3.6 \pm 0.9**$ |
| | Day onset | 4 | 20/20 | 12 | $2.3 \pm 0.9**$ | $3.5 \pm 1.4**$ |
| Saline/CFA | Day -40 | 2 | 8/8 | 12 | $3.3 \pm 0.4$ | $6.5 \pm 0.9$ |
| | Day 0 | 1 | 6/6 | 12 | $3.4 \pm 0.3$ | $6.6 \pm 0.5$ |
| | Day onset | 1 | 6/6 | 12 | $3.3 \pm 0.2$ | $6.7 \pm 0.8$ |
| Vβ14/CFA | Day -40 | 2 | 8/8 | 12 | $3.2 \pm 0.3$ | $6.1 \pm 0.6$ |
| | Day 0 | 1 | 6/6 | 12 | $3.3 \pm 0.5$ | $6.4 \pm 0.3$ |
| | Day onset | 1 | 4/4 | 12 | $3.0 \pm 0.4$ | $6.0 \pm 1.5$ |
| TCR i.d. | Day 0 | 1 | 5/6 | 15 | $1.8 \pm 1.0**$ | $3.0 \pm 1.8**$ |
| | Day 7 | 1 | 4/6 | 14 | $1.7 \pm 1.4*$ | $2.0 \pm 1.9**$ |
| | Day before onset | 1 | 6/8 | 12 | $1.8 \pm 1.3*$ | $2.5 \pm 2.1**$ |
| | 10µg, day of onset | 1 | 6/6 | 12 | $2.8 \pm 0.4$ | $4.0 \pm 0.9**$ |
| | 50µg, day of onset | 1 | 9/9 | 12 | $2.8 \pm 0.3$ | $3.1 \pm 0.3**$ |

\* $p < 0.05$;

\*\* $p < 0.01$

To evaluate its therapeutic effects, the TCR $V_\beta$8-39-59 peptide in CFA was injected s.q. into rats on the first day or onset of clinical signs. Rats at the time of this treatment exhibited hind leg weakness, ataxia, and incontinence (an average grade of 1.8). As is shown in Figure 3, treatment with the TCR peptide/CFA prevented further progression of clinical signs and shortened the duration of EAE from 6.6 days (controls) to 3.5 days. Treatment with TCR $V_\beta$14-39-59/CFA or CFA alone had no effect on clinical EAE (Table 15).

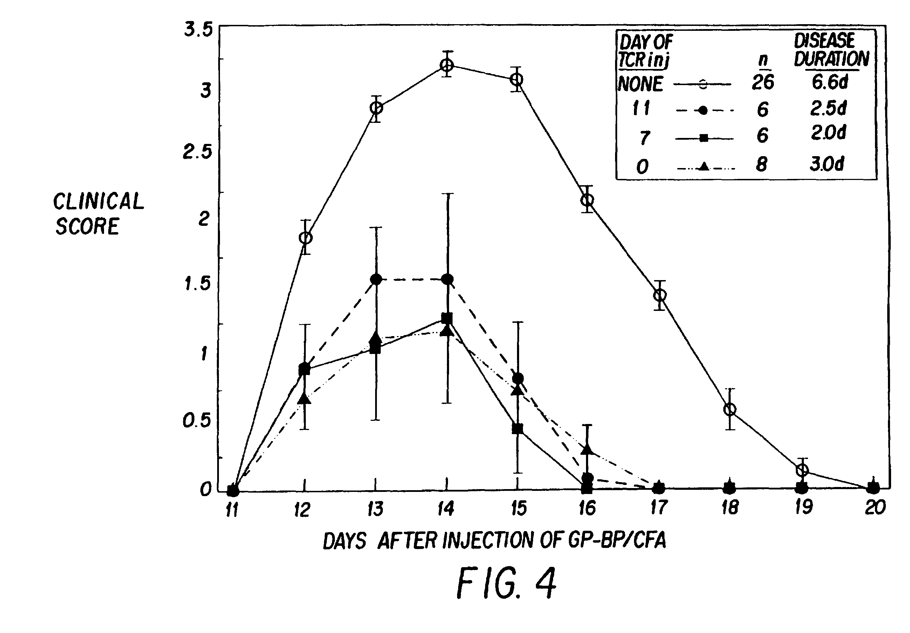

Clinical Effects of TCR Peptide Given i.d. To avoid the use of CFA, an evaluation was made of the effects on EAE of administering a saline solution of the TCR peptide intradermally in the ear. As is shown in Figure 4, i.d. administration of 50 µg of the TCR peptide at the same time as the encephalitogenic challenge (day 0), or on days 7 or 11 after challenge, all had similar suppressive effects on EAE, reducing the maximum clinical severity from 3.4 to 1.7-1.8, and shortening the duration of EAE from 6.6 days to 3-4 days (Table 17).

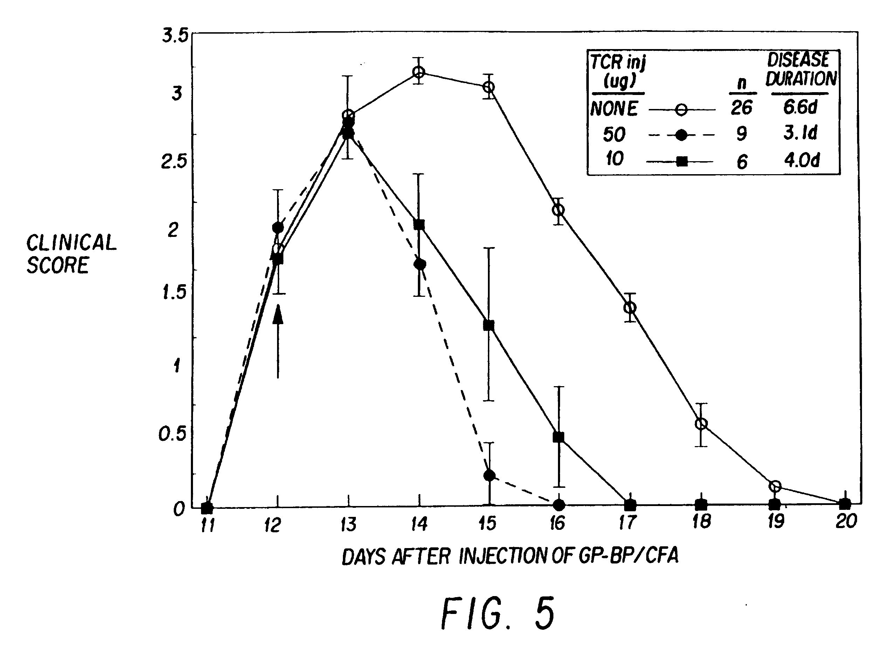

When the TCR peptide was injected on the day clinical signs were first noted (average clinical score of 1.9), no clinical effect on EAE was observed during the first day; however, during subsequent days, the severity of EAE was reduced markedly versus controls (Fig. 5). The 50 µg/rat dose of TCR peptide caused a faster resolution of EAE (3.1 days) than the lower 10 µg/rat dose of peptide (4.0 days), compared to 6.6 days for the controls.

The rapid effect of the TCR $V_\beta$8-39-59 peptide in resolving clinical EAE suggested that treatment with the peptide may have triggered a recall response to the TCR, induced initially in response to EAE. To document this possibility in vivo, rats undergoing or recovered from EAE induced with GP-MBP/CFA (without prior exposure to the TCR peptide) had significant DTH response to the Vβ8 peptide ($p < 0.01$ compared to naive or CFA immunized rats), but not to the Vβ14 peptide (Table 16). The magnitude of the response to $V_\beta$8 peptide in rats undergoing EAE was understandably less than the response in rats immunized previously with a protective regime of TCR peptide in CFA (Table 16).

T cell responses in protected rats. To evaluate the effects of TCR Vβ8 peptide therapy on T cell responses, lymph node cells (LNC) draining the site of GP-MBP/CFA injection were tested for antigen-induced proliferation, and then expanded into T cell lines. As is shown in Table 17, LNC from rats treated with TCR $V_\beta$8-39-59 had a high level of background proliferation (47,000 CPM), and similar responses to GP-MBP and other test antigens. T cell lines selected with GP-MBP (MBP/lst) responded weakly to the selecting antigen and not at all to Rt-MBP and GP-S72-89. The highest response of this line was to the TCR-$V_\beta$8-39-59 peptide. With weak GP-MBP recognition and strong TCR response, it was not surprising that the T cell line failed to transfer clinical signs of EAE to naive rats (Table 17). A similar pattern of high TCR response and low reactivity to GP-MBP and other antigens was also observed in the T cell line ($V_\beta$8/lst) selected with TCR $V_\beta$8-39-59 (Table 17).

In contrast, LNC from untreated EAE-recovered rats responded predictably to GP-MBP, Rt-MBP, and GP-S72-89 (Table 17). Unexpectedly, however, these LNC also responded to the TCR $V_\beta$8-39-59 peptide and to a lesser degree to the TCR $V_\beta$14-39-59 peptide. When selected with GP-MBP, the resulting T cell line responded strongly to MBP

## TABLE 16

### Delayed Hypersensitivity Responses to TCR Peptides.

48 hour DTH Response

| Immunization Status | Ear Test | Vβ8-39-59 | Vβ14-39-59 | GP-BP |
|---|---|---|---|---|
| Normal or saline/CFA | Day 12 | 6 ± 3 (9) | 10 ± 4 (18) | 5 ± 3 (11) |
| GP-BP/CFA | Day 12[*] | 11 ± 2 (16)[**] | 11 ± 4 (8) | 35 ± 5 (14)[**] |
| TCR Vβ8 peptide/CFA | Day 30 | 36 ± 8 (30)[**] | 9 ± 3 (18) | ND |
| TCR Vβ14 peptide/CFA | Day 30 | 6 ± 3 (12) | 40 ± 4 (12)[**] | ND |

[*]EAE onset.

[**] $p < 0.01$ versus naive or saline/CFA immunized controls.

The DTH response was evaluated in naive Lewis rats or rats immunized with either GP-BP/CFA or saline/CFA without prior exposure to TCR peptides. Similarly, rats immunized with either TCR Vβ8-39-59/CFA or TCR Vβ14-39-59/CFA were tested with either peptide. The ear swelling response (DTH) was measured 48 hours after the i.d. injection. In parentheses are shown the number of animals tested.

EP 0 552 142 B2

epitopes, and transferred severe clinical EAE to naive recipients in spite of low-level residual activity to the TCR $V_\beta 8$ peptide (Table 17). Upon further selection with the TCR $V_\beta 8$ peptide, the specific response to this TCR peptide was

## TABLE 17

### T Cell Responses After Treatment of EAE with TCR Vβ8 39-59 Peptide/CFA

| Status[a] | Stim.[b] | Proliferation (CPM/1000) | | | | | | Passive EAE | |
|---|---|---|---|---|---|---|---|---|---|
| | | Control | GP-BP | Rt-BP | 72-89 | Vβ8 | Vβ14 | N | Severity |
| Treated | LNC | 47 ± 1 | 50 ± 7 | 49 ± 2 | 38 ± 4 | 39 ± 4 | ND | -- | -- |
| | BP/1st | 17 ± 4 | 29 ± 6 | 14 ± 2 | 20 ± 2 | 66 ± 6 | ND | 0/3 | 0 |
| | Vβ8/1st | 8 ± 1 | 6 ± 1 | 4 ± 1 | 12 ± 5 | 85 ± 2 | 8 ± 2 | -- | -- |
| | | | | | | | | | |
| Untreated | LNC | 24 ± 4 | 49 ± 5 | 40 ± 2 | 44 ± 5 | 34 ± 4 | 29 ± 4 | -- | -- |
| | BP/1st | 15 ± 3 | 99 ± 9 | 46 ± 3 | 51 ± 6 | 24 ± 3 | 17 ± 2 | 3/3 | 3.0 |
| | Vβ8/1st | 11 ± 4 | 23 ± 2 | 11 ± 4 | 18 ± 2 | 34 ± 2 | 11 ± 2 | -- | -- |
| | Vβ14/1st | 10 ± 1 | 11 ± 1 | ND | 8 ± 1 | 11 ± 1 | 25 ± 6 | -- | -- |

[a]Treated rats were injected s.q. with 50 μg TCR vβ8-39-59 peptide/CFA on the first day of clinical EAE induced 12 days ealier with GP-BP/CFA. Untreated rats received only GP-BP/CFA (see Fig. 1).

[b]LN cells collected after the untreated group recovered from EAE (day 21) were stimulated with GP-BP, TCR Vβ8-39-59 peptide, or TCR Vβ14-39-59 peptide, and were expanded in IL-2 prior to restimulation with the indicated antigens (indicated as BP/1st, Vβ8/1st, or Vβ14/1st). For EAE transfer studies, 10⁷ GP-BP activated T cells from treated (protected) and untreated groups were injected i.p. into naive rats. Underlined values indicate significant difference versus control cultures.

40

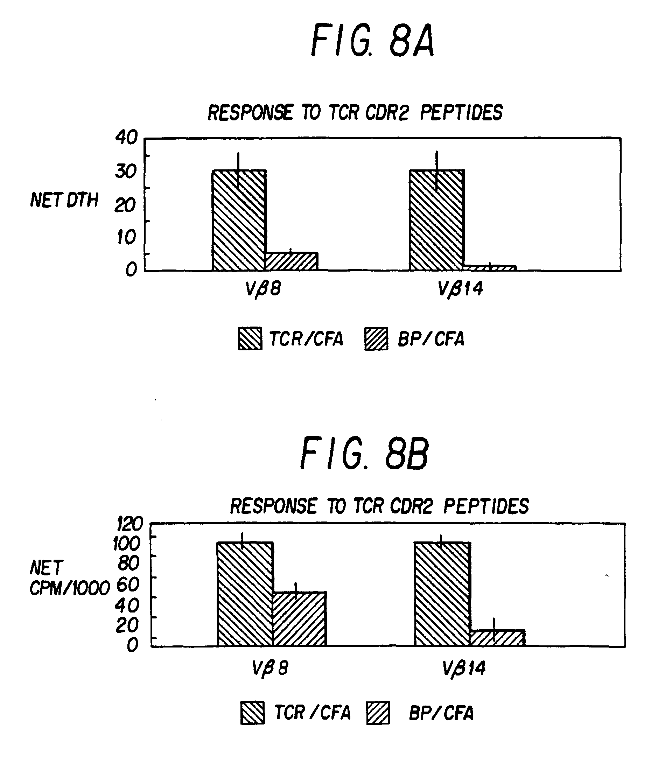

amplified, although a low level response to GP-MBP and S72-89 persisted. Further selection with the TCR $V_\beta 14$ peptide amplified only $V_\beta 14$ reactive T cells. Thus, in both TCR-selected lines, the response pattern verified the presence of TCR reactive T cells from the LN of EAE-recovered rats. As shown in Figure 8, strong DTH and proliferative responses were observed in animals preimmunized with the respective TCR peptides/CFA. Rats preimmunized with TCR $V_\beta 8$-but not with $V_\beta 14$ peptides were protected from subsequent challenge with GP-MBP/CFA. Significant DTH and strong proliferative responses to the TCR $V_\beta 8$-peptide were also observed in rats recovering from EAE that had never been immunized with the synthetic peptide, indicating that a natural regulatory response to the TCR $V_\beta 8$-peptide was induced as a consequence of the EAE disease process.

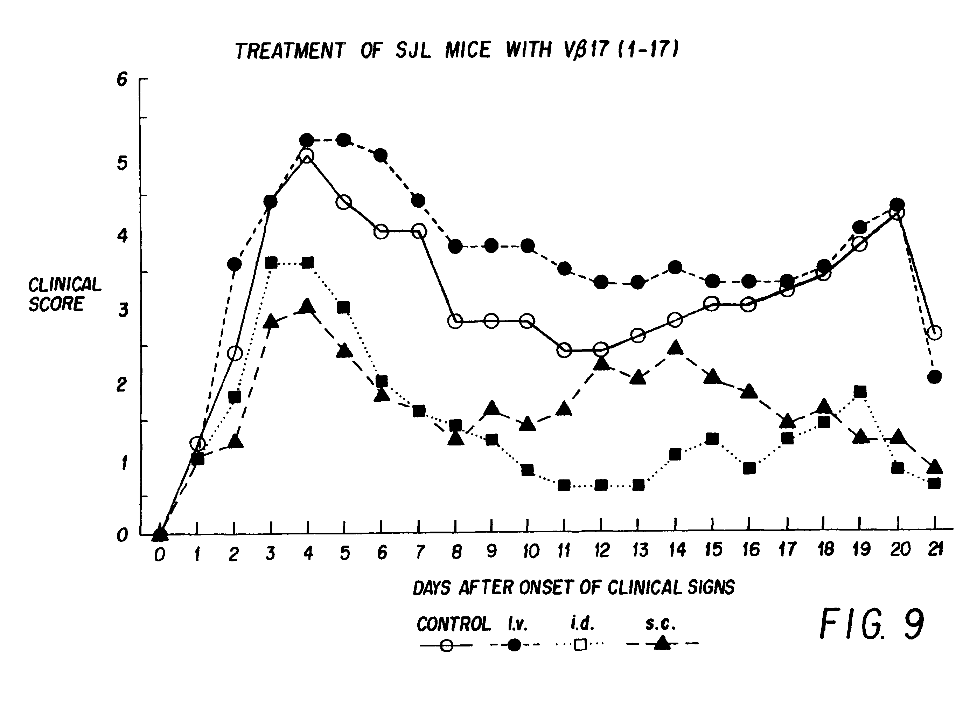

An additional well known MS model is EAE in mice, which, in contrast to rats, is characterized by a relapsing clinical progression. Groups of 6 SJL/J mice were injected with the 139-151 peptide of proteolipid apoprotein (PLP) in CFA. On the first day of the onset of clinical signs (day 14), the mice received 50 µg of a synthetic peptide corresponding to residues 1-17 of the TCR $V_\beta 17$ sequence by i.v., i.d., or s.q. administration. As shown in Figure 9, both s.q. and i. d. injection of the TCR peptide reduced the severity and shortened the duration of disease in the initial episode and in relapsing EAE.

## C. DISCUSSION

This example demonstrates clearly the therapeutic administration of the TCR $V_\beta 8$-39-59 peptide in EAE, which is consistent with previous examples demonstrating the effectiveness of the TCR peptide in preventing and suppressing EAE. The rapid clinical effect, as well as DTH and lymphocyte proliferation responses to the TCR peptide, indicate that T cell responses to the TCR $V_\beta 8$ peptide were already present in rats undergoing EAE that had never been immunized purposefully with the synthetic peptide.

Preimmunization with the $V_\beta 8$ peptide in CFA for 40 days prior to GP-MBP challenge was the most effective protocol tested (Figure 3 and Table 15). It is apparent that this period of immunization is optimal for the induction of both protective T cells and antibodies to the TCR $V_\beta 8$ peptide. Injection of the TCR $V_\beta 8$ peptide at the same time as GP-MBP challenge was less protective than preimmunization, but this protocol still suppressed completely all clinical signs of EAE in more than 30% (6/19) of the rats (Table 15). In the remainder, the clinical course of EAE was shorter and milder. Injection of the TCR $V_\beta 8$ peptide after GP-MBP challenge but before onset of clinical EAE was also effective, completely preventing onset of EAE in 4/19 rats, and generally reducing disease severity and duration in the rest (Table 15).

A surprising aspect of the present example is the almost immediate clinical effect of the TCR Vβ8 peptide injected into sick animals. All rats receiving TCR peptide therapy recovered from EAE faster than controls. Those injected with TCR peptide in CFA did not progress clinically before recovery. Those injected with TCR peptide intradermally did progress the first day, but then recovered as fast as the TCR/CFA injected rats. Both the 10 µg and 50 µg doses of peptide speeded recovery, but the higher dose resolved EAE one day sooner than the lower dose.

The TCR peptide therapy appeared to be effective by down-regulating T cell responses to encephalitogenic determinants of GP-MBP. Lymph node cells from the GP-MBP-challenged, TCR peptide-treated rats had high levels of proliferating cells, but no specific BP responses (Table 17). However, T cell lines selected with GP-MBP proliferated in the presence of GP-MBP and TCR $V_\beta 8$ peptide, but not TCR $V_\beta 14$ peptide, indicating the presence of both effector and regulatory T cell specificities. That this cell mixture could not transfer EAE may be attributable to the apparent dominance of TCR $V_\beta 8$ reactive cells (net 49,000 CPM) over GP-MBP reactive cells (net 12,000 CPM) or S72-89 reactive cells (net 3,000 CPM). In contrast, LNC from EAE-recovered rats that were challenged initially with GP-MBP but not treated with TCR $V_\beta 8$ peptide recognized GP-MBP, Rt-MBP, and S72-89, and to a lesser degree the TCR $V_\beta 8$ and $V_\beta 14$ peptides (Table 17). T cell lines selected with GP-MBP were highly encephalitogenic, due most likely to the dominance of GP-MBP-reactive T cells (net 84,000 CPM) over TCR $V_\beta 8$-reactive T cells (net 9,000 CPM). The persistence of TCR $V_\beta 8$-peptide-reactive T cells in lines selected with GP-MBP is somewhat unusual in that T cell lines selected with one antigen typically lose responses to all other antigens. No cross-reactivity has been detected between GP-MBP and the TCR-$V_\beta 8$ peptide.

Lewis rats do not relapse spontaneously when EAE is induced with MBP/CFA. Although this monophasic course of EAE does not allow testing of TCR $V_\beta 8$-39-59 peptide therapy on relapsing disease, it does provide the opportunity to determine if the strong recovery mechanisms in this strain include immune responses to the TCR $V_\beta 8$ peptide. Lewis rat T cells that respond to either encephalitogenic determinant (72-84 or 87-99 sequence) of MBP utilize preferentially the $V_\alpha 2/V_\beta 8$ gene combination in their TCR. Injection of live or attenuated encephalitogenic T cells can induce protection against EAE, as well as idiotypic and "ergotypic" responses. The increased frequency of encephalitogenic T cells induced during EAE may have the same effect of perturbing the regulatory network, and it is conceivable that at least a part of this network is directed naturally at the TCR $V_\beta 8$-39-59 sequence.

The present data support this contention. Sick or recovered rats given the TCR $V_\beta 8$ peptide i.d. had small but significant DTH responses to this peptide, but no DTH to the corresponding $V_\beta 14$ peptide (Table 16). Furthermore, lymph node cells from recovered rats responded better to the $V_\beta 8$ TCR peptide, and T cells specific for either peptide could be enriched by in vitro selection techniques (Table 17). Together, those findings provide direct evidence for the natural induction of immunity to the TCR $V_\beta$8-39-59 sequence expressed by encephalitogenic T cells. However, this may not be the only important determinant on the TCR, since other sequences within the $TCR_\alpha$ or $_\beta$ chains also induce regulatory T cells and antibodies. The protective, suppressive and therapeutic effects of the TCR $V_\beta$8-39-59 region clearly demonstrate its importance as a determinant for the idiotypic regulation of EAE.

Further, data presented for SJUJ mice, which experience a biphasic clinical course of EAE, demonstrate that adminsistration of a TCR $V_\beta$17 peptide at the onset of clinical signs reduces the severity and duration of symptoms during both the initial episode and relapse. This provides additional support for the importance of TCR V region peptides as tools in the treatment of autoimmune diseases modeled by EAE.


## EXAMPLE X


### SPECIFICITY OF HUMAN T CELL CLONES REACTIVE TO IMMUNODOMINANT EPITOPES OF MYELIN BASIC PROTEIN


Introduction


Myelin basic protein (MBP) is highly antigenic, and causes experimental autoimmune encephalomyelitis (EAE) in a variety of animal species when injected with adjuvants (Alvord, E.C., Jr., In Experimental Allergic Encephalomyelitis: A useful model for multiple sclerosis, Alvord, E.C., Jr., et al. (eds.), Alan R. Liss, Inc., New York, pp. 523-537 (1984)).

The encephalitogenic property of MBP is encompassed within a discrete number of immunodominant epitopes (about 10). Within each strain, one or more of these epitopes, in association with the available Class II MHC molecules, induces CD4+ T effector lymphocytes that home to the central nervous system (CNS), causing perivascular inflammatory lesions and nerve dysfunction (Zamvil, S.S., et al., J. Immunol. 139:1075 (1987); Offner, H., et al., J. Exp. Med. 170:355 (1989)). The genetic background, including both MHC and non-MHC genes, influences which MBP epitopes are encephalitogenic (Beraud, E., et al., J. Immunol. 136:511 (1986); Zamvil , S.S., et al., J. Exp. Med. 162:2107 (1985)), the clinical course and severity of the disease (Fritz, R.B., et al., J. Immunol. 134:2328 (1985); Hinrich, D.J., et al., J. Exp. Med. 166:1906 (1987); Linthicum, D.S., et al., J. Exp. Med. 155:31 (1982); Dietsch, G.E., etal., J. Immunol. 142:1476 (1989); Mokhtarian, F., et al., Nature (London) 309:356 (1984)), demyelination (Mokhtarian, F., et al., Nature (London) 309: 356 (1984)), and resistance mechanisms (Bernard, C.C., Clin. Exp. Immunol. 29:100 (1977); Welch, A. M., et al., J. Immunol. 125:186 (1980); Varriale, S., et al., J. Immunol. 125:186 (1989); Whitham, R.H., et al., Cell. Immunol. 126: 290 (1990)).

The spectrum of clinical and histologic signs induced by MBP-specific T cells resembles in many ways the human diseases multiple sclerosis (MS) and acute disseminated encephalomyelitis (ADE) (Paterson, P.Y., Adv. Immunol. 5: 131 (1966); Maksman, B.H., et al., Proc. Soc. Exp. Biol. Med 175:282 (1984)). Consequently, human T cell recognition of MBP has been of considerable interest.

There are several lines of evidence that suggest the involvement of T cells, including those specific for human MBP, in the pathogenesis of MS. Genetic studies indicate linkage disequilibrium between T cell receptor V and C region genes within families with MS (Hauser, S.L., et al., Neurology 39:275 (1989); Beall, S.S., et al., J. Cell. Biochem. 11D: 223 (1987)) or among patients generally (Oksenberg, J.R., et al., Proc. Natl . Acad. Sci. USA 86:988 (1989)). However, the actual involvement of MBP-reactive T cells in the pathogenesis of MS can only be demonstrated if selective regulation or removal of MBP-reactive T cells can affect the disease process. Such selective regulation is now possible in EAE.

In the present example, a synthetic TCR peptide was used to induce regulatory T cells and antibodies directed against the TCR on the pernicious T cells. This approach prevented the induction of EAE. Moreover, as demonstrated in the previous example, administration of the TCR peptide to clinically sick rats arrested disease progression and speeded recovery. The application of this approach for regulating potentially encephalitogenic T cells in MS patients depends on whether or not MBP-reactive T cells preferentially utilize a limited number of TCR V region genes in response to immunodominant epitopes of human MBP.

To this end, T cell lines from MS patients and controls were selected in a manner that allowed emergence of T cells that recognize immunodominant MBP epitopes. From these lines, 109 MBP-specific T cell clones were isolated and characterized for phenotype, epitope specificity, MHC restriction, and TCR V gene expression. The data demonstrate at the clonal level that T cells from MS patients recognize more and different MBP epitopes than do T cells from normal donors. Furthermore, in one MS donor with the disease-associated HLA-DR2/DQwl haplotype, 4 of 8 T cell clones tested expressed the $V_\beta$5.2 phenotype, indicating preferential TCR V gene use in response to MBP.

A. <u>MATERIALS AND METHODS</u>

<u>Human Subjects:</u> The MS patients evaluated in this study included 11 patients with clinically or laboratory-supported definite MS who were attending the Oregon Health Sciences University MS clinic. There were 7 females and 4 males (mean age of 46, range 34-67 years), who had MS for 6-35 years. The patients had an average ambulation index (AI) of 3.4±1.6 (range 1-6) and an average Kurtzke disability status score (KDSS) of 4.3±2.0 (range 2-4) (Kurtzke, J.F., <u>Neurology 15</u>:654 (1965)).

The normal individuals included 6 female and 3 male employees (mean age of 36, range 25-55 years) from the Veterans Affairs Medical Center and Oregon Health Sciences University. These normal individuals were selected on the basis of positive PBL proliferation responses to human MBP as described previously (Vandenbark, A.A., <u>et al.,</u> <u>J. Neurosci. Res. 23</u>:21 (1989)).

All subjects were HLA-typed retrospective to T cell line selection by a standard serological method utilized by the Oregon Health Sciences University Transplantation Laboratory. The frequency of HLA Class II alleles (DR,DQ) showed an uneven distribution for DR2 (7 of 11 patients-63%-were DR2 positive; 3 of 9 normals-33%-were DR2 positive), that in general represented the expected occurrence for these two groups (Theofilopoulos, A.N., in <u>Basic and Clinical Immunology,</u> Stites, D.P., <u>et al.</u> (eds.), Appleton and Lange Publishers, Los Altos, CA, pp. 151-154 (1987)).

<u>Antigens:</u> Human (Hu-) MBP was extracted and purified from snap frozen human brain (Eylar, E.H., <u>et al.,</u> <u>Arch. Biochem. Biophys. 132</u>:34 (1969)). Peptides of Hu-MBP, including P1 (residues 45-89), P2 (residues 1-38) and P4 (residues 90-170), were obtained by peptic cleavage and purified by Sephadex, ion exchange chromatography, and high pressure liquid chromatography (Chou, C.H.-J., <u>et al., J. Neurochem. 28</u>:115 (1977)). A series of synthetic peptides corresponding to the Hu-MBP sequences 13-28, 39-54, 55-74, 72-84, 87-99, 90-109, 110-129, 130-149, and 149-170 was synthesized by the Merrifield solid-phase method and purified by HPLC according to methods described previously (Hashim, G.A., <u>et al., J. Neurosci. Res. 16</u>:467 (1986)).

<u>T Cell Lines:</u> Human MBP-reactive T cell lines were selected from the blood of 11 MS patients and 9 normal individuals who were responsive to Hu-MBP in previous screening tests (Vandenbark, A.A., <u>et al., J. Neurosci. Res.</u> <u>23:21 (1989)</u>). Blood mononuclear cells (MNC) were separated by Ficoll density gradient centrifugation and cultured with 50 ug/ml Hu-MBP in complete medium (RPMI 1640 with 10% human AB serum, L-glutamine, sodium pyruvate and antibiotics) at a cell density $5 \times 10^5$ per well of a flat-bottomed 96-well plate for 5 days at 37°C in a 5% $CO_2$ atmosphere. The stimulated cells were transferred into IL-2 rich medium (containing 50 u/ml recombinant IL-2, AMGEN Biologicals, Thousand Oaks, CA) for expanding activated T cells. When growth in IL-2 slowed, the T cells were re-stimulated with 25 ug/ml Hu-MBP presented by autologous monocytes contained in irradiated peripheral blood MNC (4,500 rad) at the ratio of 1:10 (T:MNC). The T cell lines were re-stimulated 4-5 times until the cell number was sufficient for assessing MBP epitope specificity, MHC restriction, and phenotype.

<u>T Cell Cloning</u>: T cell clones were obtained by limiting dilution of MBP-specific T cell lines that had been re-stimulated twice with Hu-MBP. After 4 days in IL-2-enriched medium, T lymphoblasts were diluted to 1, 10 and 30 cells/20 ul of culture medium containing Hu-MBP (50 ug/ml), IL-2 (50 u/ml) and irradiated MNC ($1.5 \times 10^6$), and the cell mixture was placed into each well of a 60-well Tarasaki microtest tray (NUNC Inc., Naperville, IL) (Lamb, J.R., <u>et al.,</u> <u>J. Immunol 128</u>:233 (1982)). Recovery of human MBP-specific clones was most efficient when at least 10 Hu-MBP reactive line cells were seeded per well, producing a 20% rate of recovery. When only one line cell was seeded per well, the rate of recovery was 2%. Hu-MBP reactive T cells were recloned by seeding at 1 cell/well. The Tarasaki trays were incubated at 37°C and 5% $CO_2$ for 7 days. For re-stimulation, the cells from each positive well were transferred to a single well of a round-bottomed 96-well plate, into which were added 200 ul of complete medium with 25 ug/ml Hu-MBP and $2 \times 10^5$ irradiated MNC. 50 μ/ml of IL-2 were added to the cells on the third day after stimulation and the cells maintained in IL-2 for another four days. When the cell number reached $2-4 \times 10^5$, the cultures were transferred into a 24 well plate in 1 ml, and re-stimulated with Hu-MBP in the presence of $3 \times 10^6$ autologous irradiated MNC, and later expanded in 2 ml of IL-2 rich medium.

<u>Proliferation Assay:</u> The specificity of the cell response was evaluated by incubating $2 \times 10^4$ T cells with $1 \times 10^5$ irradiated (4,500 rad) autologous blood MNC in 0.2 ml triplicate cultures in a 96 well, round bottomed microtiter plate in the absence of antigens and in the presence of 2 ug/ml Con A, 50 ug/ml Hu-MBP, 50 ug/ml Hu-MBP fragments (P1, P2 and P4), 50 ug/ml of synthetic peptides of Hu-MBP, and 1/200 diluted <u>Herpes simplex</u> virus (HSV) antigen (Whittaker M.A. Bioproducts, Walkersville, MD). Microtiter cultures were incubated for 3 days at 37°C at 5% $CO_2$, and were pulsed with 0.5 uBq $^3$H-TdR for the last 18 hr. The cells were harvested on glass fiber filters and incorporated $^3$H-TdR was counted using a β-scintillation counter. Proliferation was expressed as CPM of stimulated culture ± SD (background subtracted). Backgrounds ranged from 200 to 2,000 CPM. MHC restriction was evaluated by incubating the T cells plus Hu-MBP, Hu-MBP fragments or synthetic peptides of Hu-MBP in the presence of antibodies specific for framework determinants of molecules from the HLA-DP, -DO or -DR locus (antibodies were purchased from Becton Dickinson Pharmaceuticals, Mountain View, CA).

<u>Phenotyping T Cells:</u> T cell lines and clones were phenotyped using Leu 3a (anti-CD4+ T helper) and Leu 2a (anti-CD8+ T cytotoxic/suppressor) monoclonal antibodies (Becton Dickinson), as described previously (Vandenbark, A.A., et al., J. Neuroimmunol. 8:103 (1985)). T cell clones were phenotyped for the expression of TCR Vβ chain gene products using mouse monoclonal antibodies specific for human TCR Vβ5.2/5.3 (5A), Vβ5.3 (5B), Vβ6.7, Vβ8.1, and Vβ12 (DIVERSI-T$^m$ a$_\beta$ TcR Screening Panel 1A, T Cell Sciences Inc., Cambridge, MA). Two x 10$^5$ T cells were incubated with 5 ul of each antibody for 1 hr at 4°C, followed by 3 washes with medium containing 5% human AB serum and further incubation with FITC-conjugated goat anti-mouse IgG for 30 min. After 2 washes and fixation in 2% formaldehyde, the stained cells were evaluated for immunofluorescence using a FACScan flow cytometer.

B. RESULTS

Characterization of Hu-MBP SDecific T Cell Lines from MS Patients and Normals. Hu-MBP specific T cell lines were selected from the blood of 11 patients with MS and 9 normal donors with previously demonstrated proliferation responses to Hu-MBP. Each line was selected from 30-50 million blood cells by repeated stimulation with whole Hu-MBP and expansion with IL-2. From previous experience in selecting rodent T cell lines, these conditions should allow expansion and focusing of representative T cell responses towards immunodominant Hu-MBP epitopes.

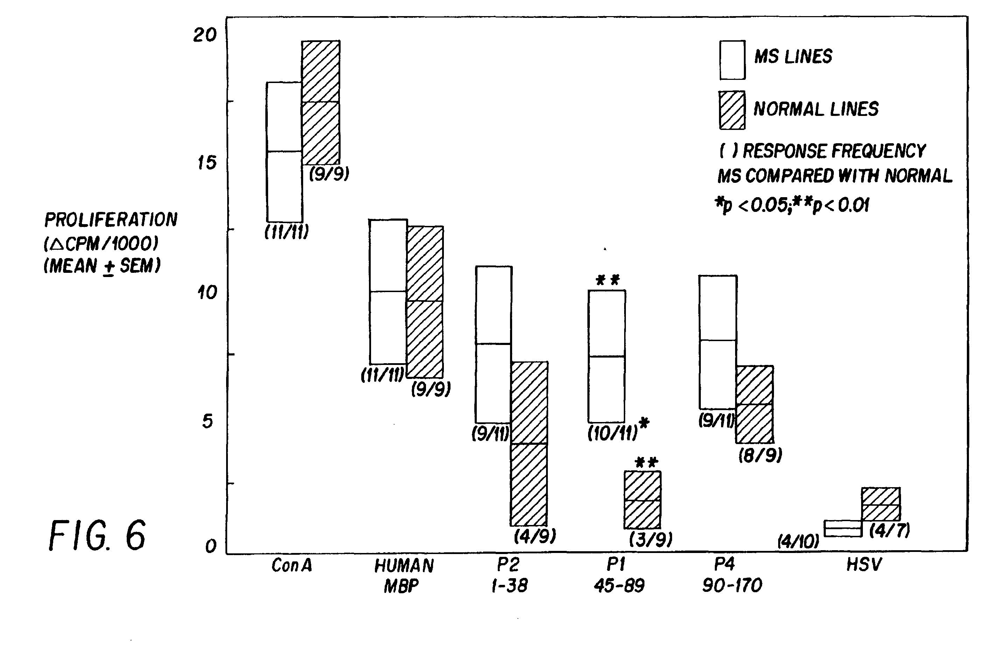

The T cell lines from MS patients and normal donors responded equally well to both Hu-MBP and Con A, with negligible responses to HSV antigens (Figure 6). T cell lines were evaluated for response to highly purified enzymatic cleavage fragments spanning the entire sequence of Hu-MBP, including P1 (residues 45-89), P2 (residues 1-38) and P4 (residues 90-170). Eight of 11 MS lines responded to all 3 Hu-MBP fragments, two responded to 2 of 3 fragments, and only 1 line responded to a single fragment. In contrast, 5 of 9 normal lines responded to a single fragment, 3 lines responded to all fragments and 1 line did not respond to any fragment. The rate of responders to the 45-89 fragment was significantly greater in the MS group versus controls, and on average, the MS T cell lines were significantly more reactive to both the 45-89 (P1 ) and 1-38 (P2) fragments of Hu-MBP (Figure 6). However, there was no difference in frequency or magnitude of response to the 90-170 (P4) fragment. All of the human T cell lines had a mixed phenotype of CD4+ and CD8+ T cells. However, the T cell lines from MS patients had a relatively higher rate of CD4+ and lower rate of CD8+ subpopulations compared to normal donors (78 ± 13% versus 58 ± 8% for CD4+ cells; 15 ± 6% versus 30 ± 12% for CD8+cells, Table 18).

Selection and Characterization of Hu-MBP Specific T Cell Clones. Although the general range of immunodominant T cell epitopes on Hu-MBP can be inferred from the pattern of reactivity of T cell lines to Hu-MBP peptides, proof of T cell recognition must be derived from analysis of clones. To this end, a total of 109 human MBP-specific T cell clones from 7 MS T cell lines (50 clones) and 6 normal T cell lines (59 clones) were evaluated for response to Hu-MBP and Hu-MBP fragments and synthetic peptides. The T cell line donors were comparable except for HLA-DR2 distribution (86% of MS patients versus 33% of normals were DR2 positive; see Table 19).

All of the T cell clones responded to Hu-MBP, but not to Herpes simplex virus (HSV) antigen, with a similar response level in both groups. In total, 48 T cell clones (distributed equally between the two groups) were phenotyped for CD4 and CDB markers. Of these, 45 clones were CD4+ and 3 (from one normal donor) were CD8+ (Table 18). This predominance of CD4+ clones was expected from previous experience in rats and mice, but did not reflect the relative proportion of these subsets in the T cell lines from which the clones were derived (Table 18).

Clonal Specificities Reflect the Pattern of T Cell Line Responses. In order to establish the validity of the clonal analysis, it is important to evaluate how well the T cell clonal specificities represent the T cell line responses. In the lines which yielded sufficient clones for comparison, the number of clones responding specifically to a distinct fragment of MBP showed a significant correlation with the Hu-MBP fragment-directed response of the parent T cell line (paired Chi Square test, $p < 0.05$).

Table 18

| Phenotype Distribution of M8P-Specific 7 Cell Lines and Clones from MS Patients and Normal Individuals | | | | | |
|---|---|---|---|---|---|
| | T Cell Line | | T Cell Clone | | |
| Donor | CD4+ % | CD8+ % | # CD4+ | # CD8+ | Total # Tested |
| MS1(HL) | 86.0 | 7.5 | 5 | 0 | 5 |
| MS2(JH) | 52.4 | 25.8 | 5 | 0 | 5 |
| MS3(MD) | 93.5 | 15.5 | 6 | 0 | 6 |
| MS4(SO) | 76.8 | 17.0 | 1 | 0 | 1 |
| NS5(BS) | Not tested | | Not tested | | |
| MS6(MR) | 84.0 | 10.0 | 7 | 0 | 7 |
| MS7(RB) | 86.0 | 14.0 | Not tested | | |

Table 18   (continued)

| Phenotype Distribution of M8P-Specific 7 Cell Lines and Clones from MS Patients and Normal Individuals | | | | | |
| --- | --- | --- | --- | --- | --- |
| | T Cell Line | | T Cell Clone | | |
| Donor | CD4+ % | CD8+ % | # CD4+ | # CD8+ | Total # Tested |
| Total | 78.1±13 | 14.9±6 | 24 | 0 | 24 |
| N 1 (BP) | 56.0 | 37.0 | 2 | 0 | 2 |
| N 2(MA) | Not tested | | 6 | 3 | 9 |
| N 3(LT) | 66.0 | 10.0 | 4 | 0 | 4 |
| N 4(DB) | 58.5 | 36.0 | 2 | 0 | 2 |
| N 5(HY) | 45.0 | 42.0 | 1 | 0 | 1 |
| N 6(JT) | 66.0 | 25.0 | 6 | 0 | 6 |
| Total | 59.3+8 | 30.0+12 | 21 | 3 | 24 |

## Table 19

### Peptide Specificities of T Cell Clones from
### MS Patients and Normal Individuals

| Donor | | | Clone number responding to | | | | |
|---|---|---|---|---|---|---|---|
| Name | Sex | HLAtype | P2(1-38) | P1(45-89) | P4(90-170) | None | Total |
| MS1(NL) | F | DR1,2/DQw1 | 2 | 7 | 0 | 10 | 19 |
| MS2(JH) | F | DR7,?*/DQw2,3 | 3 | 0 | 0 | 1 | 4 |
| MS3(MD) | F | DR2,4/DQw1,3 | 2 | 0 | 8 | 0 | 10 |
| MS4(SO) | F | DR2,w6/DQw1 | 0 | 0 | 1 | 0 | 1 |
| MS5(BS) | M | DR2/DQw1 | 0 | 1 | 3 | 0 | 4 |
| MS6(MR) | M | DR2/DQw1 | 3 | 0 | 4 | 3 | 10 |
| MS7(RB) | M | DR2,7/DQw1 | 0 | 1 | 1 | 0 | 2 |
| | | | 10(20)** | 9(18)** | 17(34) | 14(28)** | 50(100) |
| MS8(LB) | F | DR4/DQw3 | | | | | |
| MS9(SS) | F | DR5,w6/DQw1,3 | | | | | |
| MS10(MB) | F | DR5,w6/DQw1,3 | | | | | |
| MS11(JS) | M | DR2/DQw1 | | | | | |

EP 0 552 142 B2

Table 19 (continued)

| Donor | | | Clone number responding to | | | | |
|-------|-----|--------|-----------|-----------|------------|---------|---------|
| Name | Sex | HLAtype | P2(1-38) | P1(45-89) | P4(90-170) | None | Total |
| N1(BP) | F | DR1,3/DQw1,2 | 0 | 0 | 4 | 1 | 5 |
| N2(MA) | F | DR3,w6/DQw1,2 | 2 | 0 | 16 | 2 | 20 |
| N3(LT) | F | DR2,7/DQw1,2 | 0 | 1 | 2 | 4 | 7 |
| N4(DB) | M | DR2/DQw1 | 0 | 0 | 1 | 2 | 3 |
| N5(HY) | M | DR4,7/DQw2,3 | 0 | 0 | 1 | 0 | 1 |
| N6(JT) | M | DR7/DQw2 | 0 | 0 | 0 | 23 | 23 |
| | | | 2(3) | 1(2) | 24(41) | 32(54) | 59(100) |
| N7(SO) | F | DR1,2/DQw1 | | | | | |
| N8(PP) | F | DRw8/DQw3 | | | | | |
| N9(LS) | F | Not tested | | | | | |

* Antigens in linkage disequilibrium with DR antigens are present, but expected antigens did not type clearly.
** ( )=% of total clones; compared to normals, p<0.01.

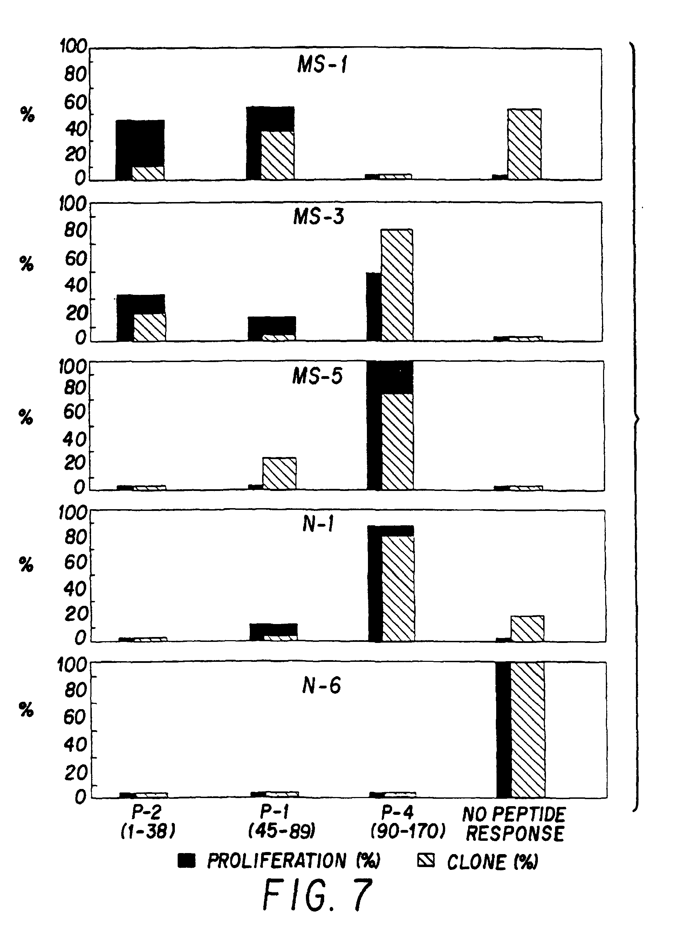

Representative comparisons from 3 MS and 2 normal donors are shown in Figure 7. Based on the pattern of T cell line responses, it was expected that more clones reactive to P1 and to P2 would be selected from the MS T cell lines than from control lines, but that the frequency of P4 reactive clones would be relatively equal. This indeed was the case, as is shown in Table 19. Unexpectedly, 46 of the 109 MBP-reactive T cell clones did not respond to any single fragment of Hu-MBP, even though the response to Hu-MBP was vigorous, ranging from 10,000 to 27,000 cpm with only 1,000-2,000 cpm background. This finding was more frequent in the normal group than in the MS group, largely on the basis of a single normal donor (Table 19).

Biased Clonal Specificities within P4. Although the P4 region of Hu-MBP represents the C terminal half of the molecule, approximately 2/3 (41 of 63) of the clones reactive to Hu-MBP peptides responded to this fragment. To identify epitope specificities, 23 of these clones from 4 normal and 4 MS donors were tested in a proliferation assay against a series of synthetic peptides corresponding to different portions of the P4 region. As is shown in Table 20, the clonal distribution was biased in normal donors towards the 110-129 sequence, and in MS donors towards the 130-149 sequence. Responses to the 149-171 sequence were similar in both groups, and only one MS clone responded to the 87-99 sequence (Table 20).

HLA-DR2 Is Capable of Restricting Multiple Hu-MBP Epitopes. The association of the HLA-DR2/DQwl haplotype with MS suggests that these Class II molecules, especially DR2, could play a critical role in restricting CD4+ T cell responses to CNS autoantigens. To this end, the Hu-MBP epitopes recognized by 17 T cell clones from 3 HLA-DR2/DQw1 donors are summarized in Table 21. In total, this set of T cell clones recognized P2 (3 clones), P1 (1 clone), P4 (8 clones), or no peptide (5 clones). Within P4, 1 MS clone recognized the 87-99 epitope, 1 normal clone recognized the 110-129 epitope, 1 MS clone recognized the 130-149 epitope, and 4 clones (3 MS and 1 normal) recognized the 149-171 epitope. Responses in 7 of 7 P4-reactive clones tested were inhibited with anti-HLA-DR antibody, clearly implicating DR2 as the restriction element (Table 21). Since the DR locus is used predominantly in restricting human T cell responses to MBP, it is likely that the majority of the 10 untested clones were also DR2 restricted. In any case, it is clear that DR2 can restrict a wide variety of Hu-MBP epitopes in humans.

TCR Vβ Gene Usage by Hu-MBP Reactive T Cell Clones. The preferential use of TCR $V_\alpha$ and $V_\beta$ gene families by encephalitogenic MBP-specific T cells from rats and mice has led to successful vaccination and therapeutic strategies directed against common TCR sequences on the pernicious T cells. It is unknown, however, if a similar mechanism in humans leads to the restricted use of TCR V genes in response to MBP or other antigens. To begin the analysis of TCR V gene use, 38 T cell clones (19 from each group) were phenotyped for expression of Vβ gene products, using a panel of 5 monoclonal antibodies specific for Vβ5.2, Vβ5.3, Vβ6.7, Vβ8.1, and Vβ12 (Table 22). TCR V gene expression could be positively identified in six of the clones, all from MS donors: Two of these clones expressed Vβ6.7, whereas the other 4 clones expressed Vβ5.2. Of the Vβ5.2+ clones, 1 was from a DR2,4 donor, and 3 were from a DR2 homozygous donor. One additional clone from the DR2 homozygous donor expressed rearranged mRNA for Vβ5.2 , indicating that in this

# Table 20

## Epitope Specificities of P4-Reactive T Cell Clones from MS Patients and Normal Individuals

| Donor Number Total(DR2+) | Number of clones responding to | | | | | Total |
|---|---|---|---|---|---|---|
| | 87-99 | 91-109 | 110-129 | 130-149 | 149-171 | |
| MS | 4(4) | 1 | 0 | 1** | 3**(2)* | 4(3)* | 9 |
| Normal | 4(2) | 0 | 0 | 9(3)* | 0 | 5(2) | 14 |

*( ) Number of donors responded.
** Compared to normals, p < 0.01.

EP 0 552 142 B2

## Table 21

### Human MBP Epitope Specificities in DR2/DQw1 Homozygous Donors

| Donor | Clone | MBP | Proliferation (cpm/1000 minus background) | | | Epitope/allele |
|-------|-------|-----|------|------|------|----------------|
| | | | P2(1-38) | P1(45-89) | P4(90-170) | |
| MS5(BS) | #24(14B7) | 3±0 | 0 | 3±0 | 0 | P1 |
| | #14(8B3) | 19±1 | 0 | 0 | 27±0 | P4 |
| | # 5(5B9) | 49±0 | 0 | 0 | 22±2 | 149-171/DR2 |
| | #23(11D3) | 60±4 | 0 | 0 | 31±2 | 149-171/DR2 |
| MS6(MR) | # 9(5D7) | 1±0 | 0 | 0 | 0 | P2 |
| | #43(5D2) | 1±0 | 2±0 | 0 | 0 | P2 |
| | #52(8C4) | 2±0 | 1±0 | 0 | 0 | P2 |
| | #41(4F3) | 3±0 | 0 | 0 | 1±0 | 87-99/DR2 |
| | #48(10B7) | 2±0 | 0 | 0 | 6±0 | 130-149/DR2 |
| | #22(3C6) | 1±0 | 0 | 0 | 1±0 | 149-171/DR2 |
| | #4S(3C3) | 4±0 | 0 | 0 | 2±1 | 149-171/DR2 |
| | #40(D310) | 13±0 | 0 | 0 | 0 | No Peptide |
| | #51(7B7) | 1±0 | 0 | 0 | 0 | No Peptide |
| | #2(3A2) | 3±0 | 0 | 0 | 0 | No Peptide |
| Normal | 4(DB) | | | | | |
| | #36(9B4) | 4±0 | 0 | 0 | 4±0 | 110-129/DR2 |
| | # 9(7E3) | 1±0 | 0 | 0 | 0 | No Peptide |
| | #19(8C5) | 2±0 | 0 | 0 | 0 | No Peptide |

EP 0 552 142 B2

Table 22

T Cell Receptor Vʙ Chain Expression of MBP-Reactive T Cell Clones

| Donor | # of Clones Tested | # of Clones Identified | Name | CD4/CD8 | TcR* | Epitope/MHC |
|---|---|---|---|---|---|---|
| MS1(NL) | 2 | 1 | #47 | CD4+ | Vʙ6.7 | HMBP/DR1,2** |
| MS2(JH) | 3 | 1 | #49 | CD4+ | Vʙ6.7 | HMBP/DR7,?** |
| MS3(MD) | 6 | 1 | #26 | CD4+ | Vʙ5.2 | 90-170/DR2,4 |
| MS4(SO) | 1 | 0 | - | - | - | - |
| MS6(MR) | 7 | 3 | #22 | CD4+ | Vʙ5.2 | 149-171/DR2 |
| | | | #41 | CD4+ | Vʙ5.2 | 87-99/DR2 |
| | | | #43 | CD4+ | Vʙ5.2 | 1-38/DR2 |
| Total 5 | 19 | 6 | | | | |
| N 1(BP) | 1 | 0 | - | - | - | - |
| N 2(MA) | 7*** | 0 | - | - | - | - |
| N 3(LT) | 3 | 0 | - | - | - | - |
| N 4(DB) | 3 | 0 | - | - | - | - |
| N 6(JT) | 5 | 0 | - | - | - | - |
| Total 5 | 19 | 0 | | | | |

*DIVERST-Tm aʙ TcR screening panel 1A (T Cell Sciences, Inc., Cambridge, MA) included Vʙ5a (Vʙ5.2 and 5.3), Vʙ5b (Vʙ5.3), Vʙ6 (Vʙ6.7), Vʙ8 (Vʙ8.1) and Vʙ12 (Vʙ12) mAbs against TcR Vʙ chain.
**Responded to whole Human MBP, but not to any peptides.
***Three CD8+ clones were included.

EP 0 552 142 B2

individual, 4/8 clones analyzed use the same TCR Vβ gene in response to Hu-MBP, even though each has a distinct epitope specificity (Table 22).

## C. DISCUSSION

The present example clearly demonstrates at the clonal level that MS patients have a more complex and altered pattern of T cell recognition of immunodominant Hu-MBP epitopes than do normal Hu-MBP responders. These data suggest an increased exposure to immunogenic forms of Hu-MBP in MS patients, thereby increasing the likelihood of inducing or perpetuating encephalitogenic T cells. The potential relevance of T cell recognition of Hu-MBP in human paralytic conditions such as MS must be viewed in light of the potent encephalitogenic function of MBP-reactive T cells in animals, and the increased frequency of activated MBP-reactive T cells in the blood and CSF of MS patients (Allegretta, M., et al., Science 247:718 (1990); Link, H., et al., Neurology 40(Suppl. 1):283 (1990)).

The T cell clones evaluated in this study were isolated from Hu-MBP specific T cell lines selected in vitro to allow the emergence of specificities directed at immunodominant Hu-MBP epitopes. Encephalitogenic determinants display imunodominance during the selection of rat and mouse T cell lines with whole MBP (Bourdette, D., et al., Cell. Immunol. 112:351 (1988); Vandenbark, A.A., et al., J. Immunol. 135:229 (1985)), and it is likely that T cells to immunodominant Hu-MBP determinants could also be encephalitogenic under permissive conditions. In this study, the immunodominant epitopes inferred from T cell line responses showed a significant correlation with the specificities identified through clonal analysis (Figure 7).

These results validate conclusions drawn from the line data regarding specificity, and document that the clones which survived the selection procedure were representative of the Hu-MBP responsive T cell population within the lines.

However, the phenotype of the clones was uniformly CD4+ (with the exception of 3 clones from a single normal donor), even though the T cell lines all contained substantial levels of CD8+ T cells. It is not clear if the CD8+ T cells within the lines were Hu-MBP specific, or if they were simply carried in the lines by the relatively high levels of IL-2 added to the cultures. It was apparent, however, that the normal T cell lines consistently contained a higher percentage of CD8+ cells than the MS lines. It is interesting and potentially relevant to the regulation of T cell function that one of the CD8+clones could inhibit MBP-induced proliferation of a CD4+ clone from the same normal donor. Such regulatory CD8+ T cells, if present in increased numbers in vivo, could account for the lack of clinical disease in normal individuals with Hu-MBP reactive T cells. A critical level of these CD8+ T cells in conjunction with increased levels of adherent suppressor cells (similar to those observed in mice (Whitham, R.H., et al., Cell. Immunol. 126:290 (1990))) could account for the inability to select Hu-MBP specific T cell lines from more than 60% of normal donors (Vandenbark, A.A., et al., J. Neurosci. Res. 23:21 (1989)).

In contrast, T cell lines can be selected from more than 80% of MS patients. These regulatory cell types would not be expected to influence the efficiency of recovering MBP-specific T cell clones by limiting dilution directly from blood, without prior line selection, as reported by others (Hafler, D.A., et al., J. Immunol 139:68 (1987); Richert, J.R., et al., J. Neuroimmunol. 23:55 (1989); Richert, J.R., et al., Ann. Neurol. 26:342 (1989)).

T cell responses to Hu-MBP in MS patients included a broader range of specificities than the responses in normal individuals (Figure 6). In addition to common epitopes, T cells from MS patients showed a biased response to the N terminal half of MBP and towards at least one epitope in the C terminal half of the molecule. The most consistent difference in response between MS and normal donors was to P1 (residues 45-89). Previous studies have shown that in MS, immunoreactive fragments of Hu-MBP-like material are present in the cerebrospinal fluid (CSF), with the dominant antigenic form spanning residues 45-89 (Whitaker, J.N., J. Immunol. 129:2729 (1982)). The detectable concentration of this fragment increases in CSF after central nervous system injury, providing a feasible explanation for the increased occurrence of P1-reactive T cells in MS patients. The bias of MS responses to P2 and to the 130-149 epitope within P4 could also be explained by the release of immunoreactive fragments of Hu-MBP during demyelination, although MHC restriction effects cannot be ruled out as yet. From animal studies, it is clear that long-term immunization with MBP induces an expanding repertoire of T cells to less and less dominant combinations of MHC and MBP epitopes (Offner, H., et al., J. Exp. Med. 170:355 (1989)). These additional T cell specificities may or may not be encephalitogenic, depending on their ability to recognize homologous MBP. The increased complexity of Hu-MBP responsive T cell specificities in MS patients is consistent with increased exposure to immunogenic fragments of MBP released during demyelination, and it is conceivable that the long-term, chronic nature of MS involves the continuous induction or restimulation of encephalitogenic T cell specificities.

Approximately 40% of the Hu-MBP reactive T cell clones, especially the clones from normal T cell lines, did not respond to any Hu-MBP fragment. Such a response could involve junctional epitopes spanning the cleavage sites of Hu-MBP (eg. residues 30-55 or 80-100), conformational epitopes destroyed by cleavage, or isoforms or post-translational variants of Hu-MBP lost during purification of cleavage fragments. Although the function of these cells in humans is unclear, similar cells occur at high frequency (50%) in EAE-recovered Lewis rats. Such T cells transfer delayed hypersensitivity responses to MBP, but do not transfer EAE or protection against EAE.

Responses in both MS and normal T cell clones were predominantly restricted by HLA-DR, although no strong association was found between any specific epitope and a given DR allele. HLA-DR2, which is over-represented in MS patients, was capable of restricting multiple epitopes of Hu-MBP, and some epitopes (e.g., 149-171) could be restricted by several HLA-DR alleles. In a previous T cell line study (Chou, Y.K., et al., J. Neurosci. Res. 23:207 (1989)), HLA-DR alleles were reported to be restricted to 26/33 Hu-MBP epitope-specific T cell responses, whereas HLA-DQ restricted 6/33 responses only in patients, and HLA-DP restricted a single normal donor response to P2.

The present data from T cell clones confirm the overwhelming restriction function of HLA-DR molecules on Hu-MBP recognition, as well as the ability of an undefined HLA-DP allele (from a different normal donor, DR7, ?/ DQw2,3) to restrict epitopes within the Hu-MBP 1-38 region recognized by three individual clones. However, no HLA-DQ restricted clones were found.

A major question regarding the use of TCR VB peptides to regulate T cell responses in humans is whether or not MBP-specific T cells utilize a limited set of V genes. The present data, using antibodies specific for only about 1/10th of the TCR VB repertoire, positively identified 6/38 clones, all from MS donors. In one HLA-DR2/DQwl homozygous donor with chronic progressive MS, 4 of 8 T cell clones specific for different Hu-MBP epitopes expressed the same V$\beta$5.2 gene in the T cell receptor (all phenotypically V$\beta$5.2 positive clones were confirmed by PcR), suggesting for the first time a bias in TCR V gene use by humans in response to Hu-MBP. The use of the same TCR V region gene in response to different Hu-MBP epitopes is similar to MBP responses in rats and mice, and indicates that the choice of the TCR is not epitope driven.

One important practical implication of this observation is that biases in TCR repertoire can be ascertained without defining the epitope specificities of MBP-reactive clones. Table 23 presents PcR data which demonstrate that MBP-specific T cell clones from human MS patients are skewed in their TCR $V_{\beta}$ gene use. Total mRNA was extracted from individual T cell clones specific for human MBP from MS patients and normal donors, and rearranged TCR $V_{\beta}$ message was amplified by PcR and identified by specific probes. Preferential use of $V_{\beta}$5.2 in MS donors MS-1 and MS-2, and preferential use of $V_{\beta}$ 14 by normal donor N-1 by MBP-specific T cell clones was demonstrated. This provides additional support for the conclusion that humans also respond preferentially with V region genes to autoantigens such as MBP.

TABLE 23

| BP-Specific TCR V$\beta$ Gene Use | | | |
|---|---|---|---|
| **Donor** | **HLA Type** | **# Clones** | **V$\beta$ Gene** |
| MS-1 | DR 1,2 | 13 | 5.2 |
|  |  | 1 | 5.1 |
| MS-2 | DR 2 | 4 | 5.2 |
|  |  | 4 | 3,4,6,9 |
| MS-3 | DR 2,4 | 1 | 5.2 |
| MS-4 | DR 2, W6 | 1 | 6 |
| N1 | DR 3,W6 | 12 | 14 |
| N2 | DR 4,7 | 1 | 2 |
| Conclusion: BP-specific T cell clones are skewed In their ICR V$\beta$ gene use, with v$\beta$5.2 expressed by 18/24 MS clones. | | | |

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

**Claims**

1. A peptide having about 15-30 amino acids comprising an amino acid sequence of a marker T cell receptor characteristic of an immune-related disease, said peptide being capable of inducing protection from said disease and comprising at least part of the second complementary determining region of said T cell receptor, or a functional derivative of said peptide, provided that the peptide does not have the sequence

   ```
   Asp-Met-Gly-His-Gly-Leu-Arg-Leu-Ile-His-Tyr-Ser-Tyr-Asp-
   Val-Asn-Ser-Thr-Glu-Lys.
   ```

2. A peptide as claimed in claim 1 having a peptide sequence corresponding to a human marker T cell receptor.

3. A peptide as claimed in claim 1 or claim 2 conjugated to a carrier or antibody which may be a monoclonal antibody

4. A pharmaceutical composition comprising a peptide as claimed in any of claims 1 to 3 in admixture with a pharmaceutically acceptable excipient.

5. A peptide as claimed in any of claims 1 to 3 wherein said disease is an autoimmune disease, such as rheumatoid arthritis, adjuvant arthritis, myasthenia gravis, encephalomyelitis, multiple sclerosis, thyroiditis, diabetes, inflammatory bowel disease or systemic lupus erythematosus.

6. A peptide as claimed in any of claims 1 to 3 wherein said disease is a malignant disease, such as T cell lymphoma.

7. The use of a peptide as claimed in any of claims 1 to 3 in the manufacture of a medicament for preventing, suppressing or treating an immune-related disease such as an autoimmune disease.

8. A method for selecting a peptide having an amino acid sequence of a T cell receptor, wherein said T cell receptor is a marker T cell receptor characteristic of an immune-related disease, said peptide being capable of inducing protection from said disease, comprising the steps of:

   (a) removing T cells from a subject susceptible to said disease;
   (b) expanding said T cells of step (a) in culture in the presence of an autoantigen preparation;
   (c) identifying the T cell receptor expressed by said expanded T cells of step (b); and
   (d) selecting said peptide from the amino acid sequence comprising at least part of the second complementarity determining region of said T cell receptor provided that the peptide does not have the sequence Asp-Met-Gly-His-Gly-Leu-Arg-Leu-Ile-His-Tyr-Ser-Tyr-Asp-val-Asn-Ser-Thr-Glu-Lys.

9. A method as claimed in claim 8 wherein said identifying step (c) comprises determining the nucleotide sequence of at least part of the gene encoding the second complementarity determining region.

10. A method as claimed in claim 8 wherein said identifying step (c) comprises determining the amino acid sequence of at least part of the second complementarity determining region of said T cell receptor

11. A method for preparing a peptide having an amino acid sequence of a T cell receptor, wherein said T cell receptor is a marker T cell receptor characteristic of an immune-related disease, said peptide being capable of inducing protection from said disease, comprising the steps of:

    (a) selecting a peptide according to a method as claimed in any of claims 8 to 10; and
    (b) synthesising said peptide or a functional derivative thereof.

12. A method as claimed in claim 11 wherein said synthesis is by chemical synthesis or by expression of a nucleotide sequence encoding said peptide.

13. A method for preparing a polyclonal antibody capable of protecting a subject from an immune-related disease comprising:

(a) administering to an animal a peptide as claimed in any of claims 1 to 3; and

(b) preparing said antibody from a body fluid of said animal.

14. A method for preparing a monoclonal antibody capable of protecting a subject from an immune-related disease comprising:

(a) administering to an animal a peptide as claimed in any of claims 1 to 3;

(b) removing spleen cells or B cells from said animal;

(c) fusing said spleen or B cells with a fusion partner cell line resulting in production of a hybridoma which secretes said monoclonal antibody; and

(d) preparing said secreted monoclonal antibody

15. A method as claimed in claim 13 or 14 wherein said animal is a subject susceptible to said immune-related disease.

16. An antibody specific for a peptide as claimed in any one of claims 1 to 3, wherein the antibody is preferably polyclonal, monoclonal or chimeric.

17. An antibody as claimed in claim 16 conjugated to a cytotoxic agent such as a ribosomal inhibitory protein, for example a ricin A chain.

18. The use of an antibody as claimed in claim 16 or 17 in the manufacture of an agent for preventing, suppressing or treating an autoimmune disease.

**Patentansprüche**

1. Peptid aus etwa 15-30 Aminisäuren, das eine Aminosäurensequenz eines T-Zell-Rezeptor-Markers enthält, der für eine immunobezogene Krankheit charakteristisch ist, wobei das Peptid in der Lage ist, einen Schutz gegen die Krankheit zu induzieren und wenigstens einen Teil der zweiten komplementären determinierenden Region des T-Zell-Rezeptors oder ein funktionales Derivat des Peptids enthält, vorausgesetzt, dass das Peptid nicht die Sequenz

```
Asp-Met-Gly-His-Gly-Leu-Arg-Ile-His-Tyr-Ser-Tyr-Asp-Val-Asn-
Ser-Thr-Glu-Lys
```

aufweist.

2. Peptid nach Anspruch 1 mit einer Peptidsequenz, die einem menschlichen T-Zell-Rezeptor-Marker entspricht.

3. Peptid nach Anspruch 1 oder 2, das mit einem Träger oder einem Antikörper konjugiert ist, der ein monoklonaler Antikörper sein kann.

4. Pharmazeutische Zusammensetzung, die ein Peptid nach einem der Ansprüche 1 bis 3 in einer Mischung mit einem pharmazeutisch verträglichen Exzipienten aufweist.

5. Peptid nach einem der Ansprüche 1 bis 3, wobei die Krankheit eine Autoimmunkrankheit ist, wie rheumatoide Arthritis, adjuvante Arthritis, Myasthenia Gravis, Enzephalomyelitis, multiple Sklerose, Thyroiditis, Diabetes, entzündliche Darmerkrankung oder systemischer erythematoser Lupus.

6. Peptid nach einem der Ansprüche 1 bis 3, wobei die Krankheit eine maligne Krankheit, beispielsweise ein T-Zell-Lymphom ist.

7. Verwendung eines Peptids nach einem der Ansprüche 1 bis 3 zur Herstellung eines Medikaments, um immunobezogene Krankheiten zu verhindern, zu unterdrücken oder zu behandeln, beispielsweise eine Autoimmunkrankheit.

8. Verfahren zum Auswählen eines Peptids, das eine Aminosäurensequenz eines T-Zell-Rezeptors aufweist, der ein T-zell-Rezeptor-Marker ist, der für eine immunobezogene Krankheit kennzeichnend ist, wobei das Peptid in der Lage ist, einen Schutz gegen die Krankheit zu induzieren, und zu dem Verfahren die Schritte gehören:

(a) dass die T-Zellen aus einem Lebewesen entnommen werden, die für die Krankheit empfindlich sind;
(b) dass die T-Zellen aus Schritt (a) in einer Kultur in Anwesenheit einer Autoantigenzubereitung expandiert werden;
(c) dass der T-Zell-Rezeptor, der von den expandierten T-Zellen nach Schritt (b) exprimiert wird, identifiziert wird; und
(d) dass aus der Aminosäuresequenz dasjenige Peptid ausgewählt wird, das wenigstens ein Teil der zweiten komplementären determinierenden Region des T-Zell-Rezeptors aufweist, vorausgesetzt, dass das Peptid nicht die Sequenz Asp-Met-Gly-His-Gly-Leu-Arg-Leu-Ile-His-Tyr-Ser-Tyr-Asp-Val-Asn-Ser-Thr-Glu-Lys

aufweist.

9.  Verfahren nach Anspruch 8, bei dem es zu dem Identifizierungsschritt (c) gehört, dass die Nukleotidsequenz wenigstens eines Teils des Gens bestimmt wird, das für die zweite komplementäre determinierende Region codiert.

10.  Verfahren nach Anspruch 8, bei dem es zu dem Identifizierungsschritt (c) gehört, dass die Aminosäuresequenz wenigstens eines Teils der zweiten komplementären determinierenden Region des T-Zell-Rezeptors bestimmt wird.

11.  Verfahren zum Zubereiten eines Peptids mit einer Aminosäuresequenz eines T-Zell-Rezeptors, der ein T-Zell-Rezeptor-Marker ist, der für eine immunobezogene Krankheit charakteristisch ist, wobei das Peptid in der Lage ist, einen Schutz gegen diese Krankheit zu induzieren, und zu dem Verfahren die Schritte gehören:

(a) dass ein Peptid nach einem Verfahren nach einem der Ansprüche 8 bis 10 ausgewählt wird; und
(b) dass das Peptid oder ein funktionales Derivat hiervon synthetisiert wird.

12.  Verfahren nach Anspruch 11, bei dem die Synthese eine chemische Synthese oder die Expremierung einer Nukleotidsequenz ist, die für das Peptid codiert.

13.  Verfahren zum Zubereiten eines polyklonalen Antikörpers, der in der Lage ist, ein Lebewesen gegen eine immunobezogen Krankheit zu schützen, wobei es zu dem Verfahren gehört:

(a) dass einem tierischen Lebewesen ein Peptid nach einem der Ansprüche 1 bis 3 verabreicht wird; und
(b) dass der Antikörper aus einer Körperflüssigkeit des tierischen Lebewesens zubereitet wird.

14.  Verfahren zum Zubereiten eines monoklonalen Antikörpers, der in der Lage ist, ein Lebewesen gegen eine immunobezogene Krankheit zu schützen, wobei es zu dem Verfahren gehört:

(a) dass einem tierischen Lebewesen ein Peptid nach einem der Ansprüche 1 bis 3 verabreicht wird;
(b) dass Milzzellen oder B-Zellen aus dem tierischen Lebewesen entnommen werden;
(c) dass die Milzzellen oder die B-Zellen mit einem Fusionspartner der Zell-Linie fusioniert werden, was zur Herstellung einer Hybridomzelle führt, die den monoklonalen Antikörper sezerniert; und
(d) dass der sezernierte monoklonale Antikörper aufbereitet wird.

15.  Verfahren nach den Ansprüchen 13 oder 14, bei dem das tierische Lebewesen ein Lebewesen ist, das empfindlich für eine immunobezogene Krankheit ist.

16.  Antikörper, der für ein Peptid nach einem der Ansprüche 1 bis 3 spezifisch ist, wobei der Antikörper vorzugsweise ein polyklonaler, ein monoklonaler oder eine Chimere ist.

17.  Antikörper nach Anspruch 16, der mit einem zytotoxischen Wirkstoff konjugiert ist, beispielsweise einem ribosomalen inhibierenden Protein, beispielsweise einer Ricin-A-Kette.

18.  Verwendung eines Antikörpers nach den Ansprüchen 16 oder 17 zur Herstellung eines Wirkstoffs zur Verhinderung, Unterdrückung oder Behandlung einer Autoimmunkrankheit.

**Revendications**

1. Peptide ayant environ 15-30 acides aminés comprenant une séquence d'acides aminés d'un récepteur marqueur de lymphocyte T caractéristique d'une maladie immuno-associée, ledit peptide étant capable d'induire une protection à l'égard de ladite maladie et comprenant au moins une partie de la seconde région de détermination de complémentarité dudit récepteur de lymphocyte T ou un dérivé fonctionnel dudit peptide, pourvu que le peptide n'ait pas la séquence

        `Asp-Met-Gly-His-Gly-Leu-Arg-Leu-Ile-His-Tyr-Ser-Tyr-Asp-Val-`
        `Asn-Ser-Thr-Glu-Lys.`

2. Peptide selon la revendication 1 ayant une séquence peptidique correspondant à un récepteur marqueur humain de lymphocyte T.

3. Peptide selon la revendication 1 ou la revendication 2 conjugué à un véhicule ou un anticorps, qui peut être un anticorps monoclonal.

4. Composition pharmaceutique comprenant un peptide selon l'une quelconque des revendications 1 à 3 en mélange avec un excipient pharmaceutiquement acceptable.

5. Peptide selon l'une quelconque des revendications 1 à 3, ladite maladie étant une maladie auto-immune, telle que l'arthrite rhumatoïde, l'arthrite auxiliaire, la myasthénie, l'encéphalomyélite, la sclérose en plaques disséminées, la thyroldite, le diabète, les maladies inflammatoires de l'intestin ou le lupus érythémateux disséminé.

6. Peptide selon l'une quelconque des revendications 1 à 3, ladite maladie étant une infection maligne, telle qu'un lymphome à cellules T

7. Utilisation d'un peptide selon l'une quelconque des revendications 1 à 3 pour lafabrication d'un médicament destiné à prévenir, supprimer ou traiter une maladie immuno-associée telle qu'une maladie auto-immune.

8. Procédé pour sélectionner un peptide ayant une séquence d'acide aminé d'un récepteur de lymphocyte T, ledit récepteur de lymphocyte T étant un récepteur marqueur de lymphocyte T caractéristique d'une maladie immunoassociée, ledit peptide étant capable d'induire une protection à l'égard de ladite maladie, ledit procédé comprenant les étapes consistant à

    (a) prélever les lymphocytes T d'un sujet prédisposé envers ladite maladie;
    (b) multiplier lesdits lymphocytes T de l'étape (a) en culture en présence d'une préparation d'autoantigènes;
    (c) identifier le récepteur de lymphocyte T exprimé par lesdits lymphocytes T multipliés de l'étape (b); et
    (d) sélectionner ledit peptide à partir de la séquence d'acides aminés comprenant au moins une partie de la seconde région de détermination de complémentarité dudit récepteur de lymphocyte T pourvu que le peptide n'ait pas la séquence Asp-Met-Gly-HisGly-Leu-Arg-Leu-Ile-His-Tyr-Ser-Tyr-Asp-Val-Asn-Ser-Thr-Glu-Lys

9. Procédé selon la revendication 8 dans lequel ladite étape d'identification (c) comprend la détermination de la séquence nucléotidique d'au moins une partie du gène codant pour la seconde région de détermination de complémentarité.

10. Procédé selon la revendication 8 dans lequel ladite étape d'identification (c) comprend la détermination de la séquence d'acides aminés d'au moins une partie de la seconde région de détermination de complémentarité dudit récepteur de lymphocyte T.

11. Procédé de préparation d'un peptide ayant une séquence d'acides aminés du récepteur de lymphocyte T, dans lequel ledit récepteur de lymphocyte T est un récepteur marqueur de lymphocyte T caractéristique d'une maladie immuno-associée, ledit peptide étant capable d'induire une protection à l'égard de ladite maladie, comprenant les étapes consistant à:

    (a) sélectionner un peptide conformément au procédé selon l'une quelconque des revendications 8 à 10; et
    (b) synthétiser ledit peptide ou un dérivé fonctionnel de celui-ci.

**12.** Procédé selon la revendication 11, dans lequel ladite synthèse est une synthèse chimique ou une synthèse par expression de la séquence nucléotidique codant pour ledit peptide.

**13.** Procédé de préparation d'un anticorps polyclonal capable de protéger un sujet à l'égard d'une maladie immunoassociée, comprenant

(a) l'administration à un animal d'un peptide selon l'une des revendications 1 à 3; et
(b) (b) la préparation dudit anticorps à partir d'un liquide corporel dudit animal.

**14.** Procédé de préparation d'un anticorps monoclonal capable de protéger un sujet à l'égard d'une maladie immunoassociée, comprenant

(a) l'administration à un animal d'un peptide selon l'une des revendications 1 à 3;
(b) le prélèvement de cellules de la rate ou de lymphocytes B dudit animal;
(c) la fusion desdites cellules de la rate ou des lymphocytes B avec une lignée cellulaire partenaire de fusion résultant dans la production d'un hybridome qui sécrète ledit anticorps monoclonal; et
(d) la préparation dudit anticorps monoclonal sécrété.

**15.** Procédé selon la revendication 13 ou 14 dans lequel ledit animal est un sujet prédisposé envers ladite maladie immuno-associée.

**16.** Anticorps spécifique d'un peptide selon l'une quelconque des revendications 1 à 3, dans lequel l'anticorps est de préférence polyclonal, monoclonal ou chimérique.

**17.** Anticorps selon la revendication 16 conjugué à un agent cytotoxique tel qu'une protéine inhibitrice ribosomique, par exemple la chaîne A de la ricine.

**18.** Utilisation d'un anticorps selon la revendication 16 ou 17 pour la fabrication d'un agent de prévention, suppression ou traitement d'une maladie auto-immune.

| ANTIBODY | INHIBITOR |
|----------|-----------|
| ANTI TcR | TcR |
| ANTI TcR | S49 |
| ANTI S49 | S49 |
| ANTI S49 | TcR |

FIG. 1

Vβ8−

## FIG.2A

RIGHT
ANGLE
SCATTER

1    10   100  1000
LOG  FLUORESCENCE INTENSITY

Vβ8+

## FIG.2B

RIGHT
ANGLE
SCATTER

1    10   100  1000
LOG FLUORESCENCE  INTENSITY

Vβ8−

## FIG.2C

250

#CELLS/
CHANNEL

0

1    10   100  1000
LOG FLUORESCENCE INTENSITY

Vβ8+

## FIG.2D

250

#CELLS/
CHANNEL

0

1    10   100  1000
LOG FLUORESCENCE INTENSITY

FIG. 3

EP 0 552 142 B2

FIG. 4

FIG. 5

FIG. 6

FIG. 7

# FIG. 8A

## RESPONSE TO TCR CDR2 PEPTIDES

## FIG. 8B

## RESPONSE TO TCR CDR2 PEPTIDES

TREATMENT OF SJL MICE WITH Vβ17 (1-17)

CLINICAL SCORE

DAYS AFTER ONSET OF CLINICAL SIGNS

CONTROL    l.v.    i.d.    s.c.

FIG. 9

EP 0 552 142 B2